(19) Europäisches Patentamt
European Patent Office
Office européen des brevets

(11) EP 2 303 908 B1

(12) **FASCICULE DE BREVET EUROPEEN**

(45) Date de publication et mention de la délivrance du brevet:
**26.07.2017 Bulletin 2017/30**

(21) Numéro de dépôt: **09772291.2**

(22) Date de dépôt: **10.06.2009**

(51) Int Cl.:
***C07K 1/10*** *(2006.01)* ***C07K 7/64*** *(2006.01)*
***C12Q 1/37*** *(2006.01)* ***G01N 33/52*** *(2006.01)*
***G01N 33/58*** *(2006.01)*

(86) Numéro de dépôt international:
**PCT/EP2009/057194**

(87) Numéro de publication internationale:
**WO 2010/000591 (07.01.2010 Gazette 2010/01)**

(54) **PEPTIDES CYCLIQUES FLUORESCENTS, PROCEDES DE PREPARATION DE CEUX-CI ET UTILISATION DE CES PEPTIDES POUR MESURER L'ACTIVITE ENZYMATIQUE D'UNE ENZYME PROTEASE**

FLUORESZENTE CYCLISCHE PEPTIDE, VERFAHREN ZU DEREN HERSTELLUNG UND VERWENDUNG DER PEPTIDE ZUM MESSEN DER ENZYMATISCHEN AKTIVITÄT EINES PROTEASEENZYMS

FLUORESCENT CYCLIC PEPTIDES, METHODS FOR THE PREPARATION THEREOF, AND USE OF SAID PEPTIDES FOR MEASURING THE ENZYMATIC ACTIVITY OF A PROTEASE ENZYME

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **10.06.2008 FR 0853850**

(43) Date de publication de la demande:
**06.04.2011 Bulletin 2011/14**

(73) Titulaires:
- **Commissariat à l'Énergie Atomique et aux Énergies Alternatives 75015 Paris (FR)**
- **Université Victor Segalen Bordeaux 2 33076 Bordeaux (FR)**

(72) Inventeurs:
- **BERTHELOT, Thomas F-91140 Villebon Sur Yvette (FR)**
- **DELERIS, Gérard F-33000 Bordeaux (FR)**

(74) Mandataire: **Ilgart, Jean-Christophe BREVALEX 95, rue d'Amsterdam 75378 Paris Cedex 8 (FR)**

(56) Documents cités:
**WO-A1-01/04623 WO-A2-02/055098**

- **GRUENEWALD JAN ET AL.: "Fluorescence resonance energy transfer as a probe of peptide cyclization catalyzed by nonribosomal thioesterase domains" CHEMISTRY & BIOLOGY, vol. 12, no. 8, août 2005 (2005-08), pages 873-881, XP005042258 CAMBRIDGE ISSN: 1074-5521 DOI: 10.1016/j.chembiol.2005.05.019**
- **NAGASE H ET AL.: "Human matrix metalloproteinase specificity studies using collagen sequence-based synthetic peptides" BIOPOLYMERS, vol. 40, no. 4, 1 janvier 1996 (1996-01-01), pages 399-416, XP009112150 NEW YORK, NY, US ISSN: 0006-3525 DOI: 10.1002/(SICI)1097-0282(1996)40:4<399::AID-BIP5>3.0.CO;2-R cité dans la demande**
- **BERTHELOT THOMAS ET AL.: "Synthesis of N-epsilon-(7-diethylaminocoumarin-3-carboxyl)- and N-epsilon-(7-methoxycoumarin-3-carboxyl)-L-Fmoc lysine as tools for protease cleavage detection by fluorescence" JOURNAL OF PEPTIDE SCIENCE, vol. 11, no. 3, 1 mars 2005 (2005-03-01), pages 153-160, XP009111804 JOHN WILEY AND SONS LTD ISSN: 1075-2617 DOI: 10.1002/psc.608 [extrait le 2004-10-11]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- KELLY KIMBERLY ET AL.: "Detection of invasive colon cancer using a novel, targeted, library-derived fluorescent peptide" CANCER RESEARCH, vol. 64, no. 17, 1 septembre 2004 (2004-09-01), pages 6247-6251, XP9113912 United States ISSN: 0008-5472 DOI: 10.1158/0008-5472.CAN-04-0817
- BENITO J M ET AL.: "Bicyclic organo-peptides as selective carbohydrate receptors: Design, solid-phase synthesis, and on-bead binding capability" QSAR AND COMBINATORIAL SCIENCE, vol. 23, no. 2-3, avril 2004 (2004-04), pages 117-129, XP8041410 De ISSN: 1611-020X DOI: 10.1002/qsar.200320011
- VAN DEN ELSEN JEAN M H ET AL.: "On the interaction between a bactericidal antibody and a PorA epitope of Neisseria meningitidis in outer membrane vesicles: A competitive fluorescence polarization immunoassay" ANALYTICAL BIOCHEMISTRY, vol. 247, no. 2, 1 mai 1997 (1997-05-01), pages 382-388, XP9113907 ISSN: 0003-2697 DOI: 10.1006/abio.1997.2064
- LOMBARD C ET AL: "Assays of matrix metalloproteinases (MMPs) activities: a review", BIOCHIMIE, MASSON, PARIS, FR, vol. 87, no. 3-4, 1 March 2005 (2005-03-01), pages 265-272, XP027603266, ISSN: 0300-9084 [retrieved on 2005-03-01]

SEQUENCE LISTING

<110> Commissariat à l'Energie Atomique

<120> Peptides cycliques fluorescents, procédés de préparation de ceux-ci et utilisation de ces peptides pour mesurer l'activité enzymatique d'une enzyme protéase

<130> SP32080 (PCT) / FG

<140> PCT/EP2009/XXXXX
<141> 2009-06-10

<150> FR 08/53850
<151> 2008-06-10

<160> 60

<170> PatentIn version 3.5

<210> 1
<211> 8
<212> PRT
<213> unidentified

<400> 1

```
Gly Pro Gln Gly Leu Leu Gly Ala
1               5
```

<210> 2
<211> 8
<212> PRT
<213> unidentified

<400> 2

```
Ala Pro Gln Gly Ile Ala Gly Gln
1               5
```

<210> 3
<211> 8

<212> PRT
<213> unidentified

<400> 3

```
Gly Pro Gln Gly Leu Ala Gly Gln
1               5
```

<210> 4
<211> 8
<212> PRT
<213> unidentified

<400> 4

```
Gly Pro Leu Gly Ile Ala Gly Ile
1               5
```

<210> 5
<211> 8
<212> PRT
<213> unidentified

<400> 5

```
Gly Pro Glu Gly Leu Arg Val Gly
1               5
```

<210> 6
<211> 8
<212> PRT
<213> unidentified

<400> 6

```
Tyr Glu Ala Gly Leu Gly Val Val
1               5
```

<210> 7
<211> 8
<212> PRT
<213> unidentified

<400> 7

```
Ala Gly Leu Gly Val Val Glu Arg
1               5
```

<210> 8
<211> 8
<212> PRT
<213> unidentified

<400> 8

Ala Gly Leu Gly Ile Ser Ser Thr
1               5

<210> 9
<211> 8
<212> PRT
<213> unidentified

<400> 9

Gly Ala Met Phe Leu Glu Ala Ile
1               5

<210> 10
<211> 8
<212> PRT
<213> unidentified

<400> 10

Ile Pro Glu Asn Phe Phe Gly Val
1               5

<210> 11
<211> 8
<212> PRT
<213> unidentified

<400> 11

Thr Glu Gly Glu Ala Arg Gly Ser
1               5

<210> 12
<211> 8
<212> PRT
<213> unidentified

<400> 12

Arg Ala Ile His Ile Gln Ala Glu
1               5

<210> 13
<211> 8
<212> PRT
<213> unidentified

<400> 13

Leu Arg Ala Tyr Leu Leu Pro Ala
1               5

<210> 14
<211> 8
<212> PRT
<213> unidentified

<400> 14

```
Gly Pro Leu Gly Met Arg Gly Leu
1               5
```

<210> 15
<211> 7
<212> PRT
<213> unidentified

<400> 15

```
Pro Gln Gly Leu Glu Ala Lys
1               5
```

<210> 16
<211> 7
<212> PRT
<213> unidentified

<220>
<221> MISC_FEATURE
<222> (7) .. (7)
<223> X représente D-Arg

<400> 16

```
Pro Leu Gly Leu Trp Ala Xaa
1               5
```

<210> 17
<211> 7
<212> PRT
<213> unidentified

<400> 17

```
Pro Leu Ala Leu Trp Ala Arg
1               5
```

<210> 18
<211> 6
<212> PRT
<213> unidentified

<220>
<221> MISC_FEATURE
<222> (2)..(2)
<223> X représente une 3-cyclohexyl alanine (Cha)

<220>
<221> MISC_FEATURE
<222> (3)..(3)
<223> X représente un acide alpha amino-butyrique (Abu)

<220>
<221> MISC_FEATURE
<222> (4)..(4)

<223> X représente Cys(Me)

<400> 18

```
Pro Xaa Xaa Xaa His Ala
1               5
```

<210> 19
<211> 6
<212> PRT
<213> unidentified

<220>
<221> MISC_FEATURE
<222> (2)..(2)
<223> X représente une 3 cyclohexyl alanine (Cha)

<220>
<221> MISC_FEATURE
<222> (4)..(4)
<223> X représente Cys(Me)

<400> 19

```
Pro Xaa Gly Xaa His Ala
1               5
```

<210> 20
<211> 6
<212> PRT
<213> unidentified

<220>
<221> MISC_FEATURE
<222> (2)..(2)
<223> X représente une 3 cyclohexyl alanine (Cha)

<220>
<221> MISC_FEATURE
<222> (4)..(4)
<223> X représente Cys(Me)

<400> 20

```
Pro Xaa Gly Xaa His Ala
1               5
```

<210> 21
<211> 7
<212> PRT
<213> unidentified

<220>
<221> MISC_FEATURE
<222> (5) .. (5)
<223> X représente un (N-3(2,4-dinitrophényl)-L-2,3-diaminopropionyle (Dpa)

<400> 21

Pro Leu Gly Leu Xaa Ala Arg
1 5

<210> 22
<211> 5
<212> PRT
<213> unidentified

<220>
<221> BINDING
<222> (3)..(4)
<223> Les acides aminés 3 et 4 sont séparés par un groupement -[2-mercapto-4-méthyl-pentanoyl]-

<400> 22

Pro Leu Gly Leu Gly
1 5

<210> 23
<211> 7
<212> PRT
<213> unidentified

<220>
<221> MISC_FEATURE
<222> (4)..(4)
<223> X représente Cys(Me)

<220>
<221> MISC_FEATURE
<222> (7) .. (7)
<223> X représente D-Arg

<400> 23

Pro Leu Gly Xaa His Ala Xaa
1 5

<210> 24
<211> 7
<212> PRT
<213> unidentified

<400> 24

Arg Pro Leu Ala Leu Trp Arg
1 5

<210> 25
<211> 6
<212> PRT
<213> unidentified

<220>
<221> MISC_FEATURE
<222> (2)..(2)
<223> X représente une 3 cyclohexyl alanine (Cha)

<220>
<221> MISC_FEATURE
<222> (4)..(4)
<223> X représente une norvaline (Nva)

<400> 25

```
Pro Xaa Gly Xaa His Ala
1               5
```

<210> 26
<211> 9
<212> PRT
<213> unidentified

<220>
<221> MISC_FEATURE
<222> (7) .. (7)
<223> X représente une norvaline (Nva)

<400> 26

```
Arg Pro Lys Pro Tyr Ala Xaa Trp Met
1               5
```

<210> 27
<211> 8
<212> PRT
<213> unidentified

<400> 27

```
Pro Pro Gly Ala Tyr His Gly Ala
1               5
```

<210> 28
<211> 8
<212> PRT
<213> unidentified

<400> 28

```
Gly Pro His Leu Leu Val Glu Ala
1               5
```

<210> 29
<211> 7
<212> PRT
<213> unidentified

<400> 29

```
Pro Gln Gly Leu Glu Ala Lys
1               5
```

<210> 30
<211> 7
<212> PRT

<213> unidentified

<220>
<221> MISC_FEATURE
<222> (4)..(4)
<223> X représente une norvaline (Nva)

<220>
<221> MISC_FEATURE
<222> (5) .. (5)
<223> X représente un (N-3(2,4-dinitrophényl)-L-2,3-diaminopropionyle (Dpa)

<400> 30

```
Pro Leu Ala Xaa Xaa Ala Arg
1               5
```

<210> 31
<211> 7
<212> PRT
<213> unidentified

<400> 31

```
Pro Leu Gly Met Trp Ser Arg
1               5
```

<210> 32
<211> 5
<212> PRT
<213> unidentified

<220>
<221> BINDING
<222> (3)..(4)
<223> Les acides aminés 3 et 4 sont séparés par un groupement -SCH[CH2CH(CH3)2]-CO-

<400> 32

```
Pro Leu Gly Leu Gly
1               5
```

<210> 33
<211> 8
<212> PRT
<213> unidentified

<400> 33

```
Arg Pro Pro Gly Phe Ser Ala Phe
1               5
```

<210> 34
<211> 8
<212> PRT
<213> unidentified

<400> 34

```
Arg Val Gly Phe Tyr Glu Ser Asp
1               5
```

<210> 35
<211> 8
<212> PRT
<213> unidentified

<400> 35

```
Leu Leu Ser Ala Leu Val Glu Thr
1               5
```

<210> 36
<211> 8
<212> PRT
<213> unidentified

<400> 36

```
Glu Ala Ile Pro Met Ser Ile Pro
1               5
```

<210> 37
<211> 8
<212> PRT
<213> unidentified

<400> 37

```
Ile Ala Gly Arg Ser Leu Asn Pro
1               5
```

<210> 38
<211> 8
<212> PRT
<213> unidentified

<400> 38

```
Lys Pro Gln Gln Phe Phe Gly Leu
1               5
```

<210> 39
<211> 8
<212> PRT
<213> unidentified

<400> 39

```
Asp Val Ala Gln Phe Val Leu Thr
1               5
```

<210> 40
<211> 8
<212> PRT
<213> unidentified

<400> 40

Asp Thr Leu Glu Val Met Arg Lys
1 5

<210> 41
<211> 8
<212> PRT
<213> unidentified

<400> 41

Asp Val Gly His Phe Arg Thr Phe
1 5

<210> 42
<211> 8
<212> PRT
<213> unidentified

<400> 42

Asp Ser Gly Gly Phe Met Leu Thr
1 5

<210> 43
<211> 8
<212> PRT
<213> unidentified

<400> 43

Arg Val Ala Glu Met Arg Gly Glu
1 5

<210> 44
<211> 8
<212> PRT
<213> unidentified

<400> 44

Asp Leu Gly Arg Phe Gln Thr Phe
1 5

<210> 45
<211> 8
<212> PRT
<213> unidentified

<400> 45

Pro Phe Ser Pro Leu Val Ala Thr
1 5

<210> 46
<211> 8
<212> PRT

<213> unidentified

<400> 46

Ala Pro Gly Asn Ala Ser Glu Ser
1 5

<210> 47
<211> 8
<212> PRT
<213> unidentified

<400> 47

Phe Ser Ser Glu Ser Lys Arg Glu
1 5

<210> 48
<211> 8
<212> PRT
<213> unidentified

<400> 48

Pro Pro Glu Glu Leu Lys Phe Gln
1 5

<210> 49
<211> 8
<212> PRT
<213> unidentified

<400> 49

Gly Pro Leu Gly Met Arg Gly Leu
1 5

<210> 50
<211> 12
<212> PRT
<213> unidentified

<220>
<221> MISC_FEATURE
<222> (7) .. (7)
<223> X représente une norvaline (Nva)

<400> 50

Arg Pro Lys Pro Val Glu Xaa Trp Arg Glu Ala Lys
1 5 10

<210> 51
<211> 7
<212> PRT
<213> unidentified

<400> 51

Pro Tyr Ala Tyr Trp Met Arg
1 5

<210> 52
<211> 8
<212> PRT
<213> unidentified

<220>
<221> MISC_FEATURE
<222> (7) .. (7)
<223> X représente une norvaline (Nva)

<400> 52

Arg Pro Lys Pro Leu Ala Xaa Trp
1 5

<210> 53
<211> 7
<212> PRT
<213> unidentified

<400> 53

Pro Leu Ala Tyr Trp Ala Arg
1 5

<210> 54
<211> 8
<212> PRT
<213> unidentified

<400> 54

Gly Pro Pro Gly Val Val Gly Pro
1 5

<210> 55
<211> 8
<212> PRT
<213> unidentified

<400> 55

Gly Pro Pro Gly Leu Arg Gly Glu
1 5

<210> 56
<211> 8
<212> PRT
<213> unidentified

<400> 56

Gly Pro Gly Gly Val Val Gly Pro
1 5

<210> 57
<211> 8
<212> PRT
<213> unidentified

<400> 57

```
Ile Pro Gln Asn Phe Phe Gly Val
1               5
```

<210> 58
<211> 8
<212> PRT
<213> unidentified

<400> 58

```
Pro Pro Gly Ala Tyr His Gly Ala
1               5
```

<210> 59
<211> 8
<212> PRT
<213> unidentified

<400> 59

```
Arg Pro Lys Pro Gln Gln Phe Trp
1               5
```

<210> 60
<211> 7
<212> PRT
<213> unidentified

<220>
<221> MISC_FEATURE
<222> (4)..(4)
<223> X représente Cys(p-OMeBz)

<400> 60

```
Pro Leu Ala Xaa Trp Ala Arg
1               5
```

## Description

### DOMAINE TECHNIQUE

La présente invention a trait à de nouveaux peptides cycliques fluorescents destinés à permettre la mesure de l'activité enzymatique d'une enzyme protéase, notamment par la technique dite de FRET (acronyme correspondant à la terminologie anglaise « Fluorescence Resonance Energy Transfer » ou encore « Förster's Resonance Energy Transfer »).

L'invention trouve, notamment, son application dans le domaine du diagnostic de maladies découlant d'un dérèglement enzymatique.

### ÉTAT DE LA TECHNIQUE ANTÉRIEURE

Les enzymes présentent des mécanismes d'action complexes faisant intervenir notamment des phénomènes de régulations, qui, lorsqu'ils sont déréglés, peuvent être à l'origine de l'apparition de troubles pathologiques.

C'est le cas notamment des métalloprotéinases (connues sous l'abréviation MMP), qui sont capables de cliver tous les éléments de la matrice extracellulaire, certains facteurs de croissance ainsi que des molécules d'adhésion. Ces enzymes sont naturellement régulées par des inhibiteurs comme les TIMP (acronyme correspondant à la terminologie anglaise « Tissue inhibitor of metalloproteinases »). Lorsque la balance MMP/TIMP est déréglée, des processus pathologiques peuvent apparaître tels que la croissance tumorale, les métastases, les arthrites rhumatoïdes, les maladies cardiaques.

Eu égard aux implications des enzymes dans de nombreuses pathologies, il s'est révélé très vite primordial de mettre en place des outils permettant de mesurer l'activité de ces enzymes, en vue d'établir éventuellement une corrélation avec la pathologie détectée. A l'heure actuelle, les outils disponibles consistent en des substrats de l'enzyme, dont on veut mesurer l'activité, ces substrats présentant des groupes fluorescents, l'activité pouvant ainsi être quantifiée par des mesures de fluorescence. En particulier, la technique de fluorescence la plus utilisée est le FRET (acronyme correspondant à la terminologie anglaise « Fluorescence Resonance Energy Transfer »). Toutefois, cette technique n'est pas basée en tant que telle sur la mesure du transfert de fluorescence mais sur la mesure de transfert d'une énergie électronique. Il faut ainsi faire la distinction entre le transfert radiatif et le transfert non radiatif (ou résonant). Le transfert radiatif ne donne pas accès à des informations comme la distance entre groupes, leurs orientations, qui peuvent être des données essentielles pour comprendre ou caractériser les systèmes étudiés.

Le transfert radiatif est un processus se déroulant en deux étapes. Dans un premier temps, un photon est émis par un groupe donneur et dans un deuxième temps, ce photon est absorbé par un groupe accepteur de photons identique ou différent du groupe donneur. Ce transfert ne requiert aucune interaction entre les groupes mais dépend du recouvrement spectral des espèces et de la concentration.

Le transfert non radiatif de l'énergie d'excitation nécessite une interaction entre un groupe donneur et un groupe accepteur. Ce transfert ne peut se produire, que si le spectre d'émission du groupe donneur recouvre partiellement le spectre d'absorption du groupe accepteur, afin qu'il existe une correspondance en énergie entre des transitions vibroniques du groupe donneur et des transitions vibroniques du groupe accepteur. De telles transitions sont dites couplées, ou « en résonance ».

Le phénomène de transfert non radiatif d'énergie a été étudié par Förster. Il a pu mettre en évidence, que ce phénomène physique se déroule sur de très courtes distances, de l'ordre de 10 à 100 Å. Lorsque la distance entre le groupe donneur et le groupe accepteur augmente, ceci se traduit par une variation du transfert de l'énergie. Ce phénomène peut ainsi être utilisé pour évaluer l'activité enzymatique d'une enzyme protéase. En effet, en mettant en contact un substrat comprenant à la fois un groupe donneur et un groupe accepteur disposés respectivement à chacun de ses extrémités avec une enzyme protéase spécifique, il s'ensuit un clivage du substrat et ainsi, une augmentation de la distance entre le groupe donneur et le groupe accepteur et, par voie de conséquence, une variation du transfert de l'énergie d'excitation (ou énergie de résonance). A partir de cette variation, on peut ainsi remonter à une estimation de l'activité de l'enzyme.

Les substrats actuellement utilisés sont pour la plupart des substrats de structure linéaire, qui se révèlent être peu stables en milieu biologique, car ils sont notamment susceptibles d'être dégradés par des exoprotéases et des protéases non spécifiques.

Des substrats de structure linéaire sont notamment décrits dans Lombard et al. (Biochimie 87 (2005) 265-272).

Il est décrit également des substrats de structure cyclique mais pour des applications totalement différentes de celles de la détermination de l'activité enzymatique d'enzympes protéases, tel que cela est le cas dans :

- WO 01/04623, qui décrit des substrats cycliques pour la détermination de la concentrations d'ions potassium dans un échantillon ; et

- Gruenewald et al. (Chemistry Biology, vol.12, n°8, août 2005, p.873-881), qui décrit des substrats cycliques pour suivre la cyclisation de peptides induite par des thioestérases.

Il existe donc un véritable besoin pour des peptides utilisables comme outils pour la quantification de l'activité enzymatique d'une enzyme protéase prédéterminée, qui soient stables et qui permettent notamment de suivre l'activité enzymatique de ladite enzyme par la technique FRET.

## EXPOSÉ DE L'INVENTION

Les Inventeurs ont ainsi mis au point de nouveaux peptides répondant au besoin susmentionné.

Ainsi, l'invention a trait, selon un premier objet, à un peptide cyclique spécifique (désigné également par cyclopeptide) comprenant la séquence suivante :

$$-S_1-X_1-S_2-X_2-$$

dans laquelle $S_1$ est une première séquence peptidique cible d'une enzyme protéase $E_1$ du type MMP, $S_2$ est une seconde séquence peptidique cible d'une enzyme protéase $E_2$ du type MMP, $S_1$ et $S_2$ pouvant être identiques ou différents, ladite première séquence peptidique $S_1$ et ladite seconde

séquence peptidique $S_2$ comprenant de 4 à 14 acides aminés, $E_1$ et $E_2$ pouvant correspondre à la même enzyme protéase ou à deux enzymes protéases distinctes, $X_1$ est une sonde porteuse d'un groupe donneur fluorescent et $X_2$ est une sonde porteuse d'un groupe accepteur fluorescent ou non fluorescent.

De préférence, le groupe donneur et le groupe accepteur sont choisis de sorte à ce que le spectre d'émission de fluorescence du groupe donneur recouvre, au moins en partie, le spectre d'absorption du groupe accepteur.

Ainsi, en présence d'une enzyme protéase E (si $E_1$ et $E_2$ ne constituent qu'une seule et même enzyme et $S_1$ et $S_2$ sont des séquences peptidiques cibles de la même enzyme protéase) ou à la fois d'une enzyme protéase $E_1$ et d'une enzyme protéase $E_2$ (lorsqu'elles sont distinctes et que $S_1$ est une séquence peptidique cible de l'enzyme protéase $E_1$ et $S_2$ est une séquence peptidique cible de l'enzyme protéase $E_2$), les peptides cycliques de l'invention vont se cliver à la fois au niveau de la première séquence peptidique $S_1$ et au niveau de la seconde séquence peptidique $S_2$, ce qui va générer un éloignement dans l'espace des sondes $X_1$ et $X_2$, du fait de la disposition des sondes $X_1$ et $X_2$. S'ensuit un éloignement des groupes donneur et accepteur et, par voie de conséquence, une variation du transfert de l'énergie de résonance entre ces deux groupes. A partir de la variation d'énergie de résonance, il est ainsi possible de remonter à l'activité enzymatique de l'enzyme protéase E ou des enzymes protéases $E_1$ et $E_2$.

Qui plus est, le fait que les peptides de l'invention soient cycliques permet de surmonter les inconvénients des peptides linéaires classiquement utilisés dans l'art antérieur, notamment les problèmes de stabilité rencontrés avec les peptides linéaires de l'art antérieur.

Selon un mode de réalisation particulier de l'invention, $E_1$ et $E_2$ correspondent à la même enzyme protéase.

Pour étudier l'activité enzymatique d'une enzyme protéase E, dans le cas où $E_1$=$E_2$, les inventeurs auraient pu imaginer tout simplement de cycliser une seule séquence peptidique linéaire cible de l'enzyme protéase E à étudier, comprenant à ses extrémités une sonde $X_1$ et une sonde $X_2$ selon le schéma suivant :

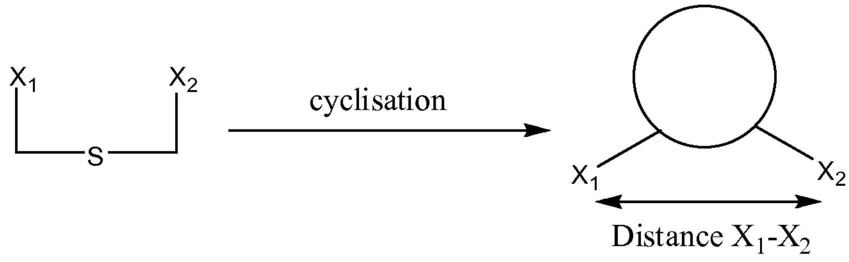


En présence de l'enzyme protéase E à étudier, le peptide cyclique se cliverait selon le schéma suivant :

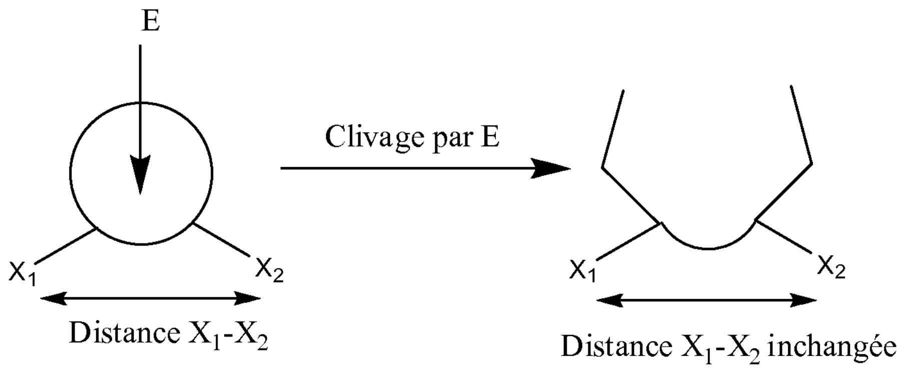


Après clivage, la distance entre les groupes $X_1$ et $X_2$ est inchangée. Il s'ensuit aucune variation du transfert de résonance, et donc une impossibilité de quantifier l'activité de l'enzyme protéase E.

Avant d'entrer plus en détail dans la description, nous proposons les définitions suivantes.

Par groupe donneur fluorescent, on entend classiquement un groupe apte à absorber de l'énergie lumineuse (dite lumière d'excitation, dont la longueur d'onde appartient généralement au domaine de l'ultraviolet) et de la restituer sous deux formes possibles en fonction de son environnement :

a) sous forme d'un transfert d'énergie d'excitation par résonance avec un groupe accepteur, à condition que ce dernier se trouve à une distance compatible avec le phénomène de FRET décrit précédemment et préférentiellement, à une distance allant de 10 à 100 Å ; ou
b) sous forme d'une lumière fluorescente (dite lumière d'émission du donneur), dont la longueur d'onde appartient, généralement, au domaine du visible.

Par groupe accepteur, on entend classiquement, au sens de l'invention, un groupe apte à absorber l'énergie d'excitation du groupe donneur par énergie de résonance. Cette énergie d'excitation pourra être restituée selon deux grands modes :

a) par émission radiative (émission de photons), auquel cas on parlera de groupe accepteur fluorescent ; ou
b) par émission non radiative (à savoir tout autre mode de désactivation autre que l'émission de photons), auquel cas on parlera de groupe accepteur non fluorescent.

Par sonde, on entend classiquement, au sens de l'invention, un reste d'acide aminé porteur d'un groupe donneur (dans le cas de la sonde $X_1$) ou porteur d'un groupe accepteur (dans le cas de la sonde $X_2$).

Par reste d'acide aminé, on entend, classiquement, le reste d'acide aminé résultant de la réaction d'une fonction $-NH_2$ d'un acide aminé ou $-CO_2H$ et analogues avec une fonction d'un autre composé, de sorte à former une liaison covalente.

Par séquence peptidique cible, on entend classiquement, au sens de l'invention, une séquence peptidique apte à être reconnue et clivée par une enzyme protéase.

L'utilisation d'un peptide cyclique comprenant deux séquences peptidiques cibles de l'enzyme protéase $E_1$ et de l'enzyme protéase $E_2$ ($E_1$ et $E_2$ pouvant être identiques ou différents) entre lesquelles sont disposées respectivement une sonde $X_1$ et une sonde $X_2$ conforme à l'invention permet, comme explicité ci-dessus, de fournir de bons outils pour la quantification de l'activité desdites enzymes protéases.

Les peptides cycliques de l'invention répondent à la formule suivante :

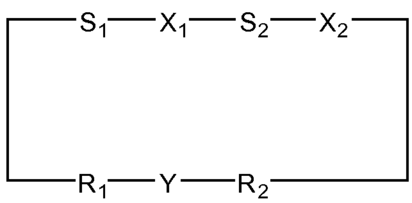

dans laquelle :

- $S_1$, $X_1$, $S_2$ et $X_2$ répondent à la même définition que celle donnée ci-dessus ;

- $R_1$ et $R_2$ correspondent indépendamment à une liaison simple, un reste d'acide aminé ou une séquence peptidique ;
- Y correspond à un groupe -CONH- ou -NHCO-.

$S_1$ et $S_2$ peuvent être identiques, lorsque le peptide est destiné à quantifier l'activité d'une seule enzyme protéase ($E_1$ et $E_2$ étant alors identiques) ou différents, notamment lorsque le peptide est destiné à différencier la présence d'isoformes de la même enzyme protéase ou la présence de deux enzymes protéases distinctes $E_1$ et $E_2$.

Lorsque $S_1$ et $S_2$ sont identiques, elles peuvent être disposées dans le même sens ou en sens inverse. Par exemple, lorsque $S_1$ correspond à la séquence -Gly-Pro-Gln-Gly-Leu-Leu-Gly-Ala-, la séquence $S_2$ peut correspondre à la même séquence disposée en sens inverse, à savoir la séquence suivante -Ala-Gly-Leu-Leu-Gly-Gln-Pro-Gly-.

$R_1$ et $R_2$ peuvent correspondre, selon un mode de réalisation particulier de l'invention, à un reste d'acide aminé ou à une séquence peptidique. Ceci est particulièrement avantageux lorsque les peptides cycliques sont destinés au domaine galénique, et notamment pour des raisons de stabilité des peptides en milieu biologique.

Les peptides cycliques de l'invention sont adaptés pour la mesure de l'activité enzymatique d'enzymes protéases du type MMP (connues également sous le nom de « métalloprotéases de matrice extracellulaire »).

L'enzyme protéase $E_1$ et l'enzyme protéase $E_2$ sont des enzymes du type MMP, auquel cas la première séquence peptidique $S_1$ est cible d'une enzyme protéase MMP et la seconde séquence peptidique $S_2$ est cible d'une enzyme protéase MMP.

Les MMP sont une famille d'endopeptidases, au nombre de 28, contenant un atome de zinc au niveau de leur site actif. Elles peuvent être classées en deux sous-familles, selon qu'elles sont sécrétées ou associées à une membrane. Les MMP sécrétées comprennent les collagénases (telles que MMP-1, 8, 13 et 18), les gélatinases (telles que MMP-2 et 9), les matrilysines (telles que MMP-7 et 26), les stromélysines (telles que MMP-3, 10 et 11), l'épilysine (MMP-28), l'énamélysine (MMP-20). Les MMP associées à une membrane comprennent les MMP transmembranaires telles que MMP-14, MMP-15, MMP-16, MMP-17, MMP-23, MMP-24 et MMP-25.

Les MMP sont capables de cliver tous les éléments de la matrice extracellulaire ainsi que certains facteurs de croissance, ou encore des molécules d'adhésion. Les MMP sont régulées par des inhibiteurs naturels tels que les TIMP et jouent un rôle important dans les processus physiologiques (tels que la croissance embryonnaire, l'angiogénèse, le remodelage du tissu osseux). Lorsque la balance MMP/TIMP est déréglée, des processus pathologiques apparaissent tels que la croissance tumorale, les métastases, les arthrites rhumatoïdes, les maladies cardiaques.

Le rôle des MMP dans la progression tumorale est multiple, notamment du fait de la capacité des MMP à dégrader la matrice extracellulaire, favorisant ainsi l'invasion tumorale. Il a été démontré que les MMP ont également une activité protéolytique contre des protéines non matricielles, ce qui leur confère un rôle complexe dans plusieurs autres étapes de la progression tumorale, la perte d'adhérence, l'invasion, la prolifération, l'angiogénèse, l'intravasion, l'extravasion et la croissance métastatique.

Les peptides cycliques se révèlent donc être d'excellents outils pour le suivi de l'activité enzymatique d'enzymes protéases du type MMP, afin, notamment, d'établir des liens entre l'activité enzymatique et les pathologies détectées.

Les peptides cycliques étant destinés au suivi de l'activité des MMP, les séquences peptidiques cibles $S_1$ et $S_2$ sont cibles d'une enzyme MMP (si $S_1$=$S_2$ ou si $S_1$ et $S_2$ sont des séquences cibles différentes destinées à distinguer deux isomorphes d'une même enzyme) ou de deux enzymes MMP (si $S_1$ et $S_2$ sont distinctes et sont cibles respectivement de deux enzymes protéases distinctes), ces séquences correspondant aux séquences des substrats de la (des) MMP reconnues par la(les) MMP dont on veut suivre l'activité.

Plus précisément, les séquences peptidiques cibles $S_1$ et $S_2$ sont cibles d'une enzyme MMP choisie parmi la MMP-1, MMP-2, MMP-3, MMP-5, MMP-7, MMP-8, MMP-9, MMP-10, MMP-11, MMP-12, MMP-13, MMP-14, MMP-15, MMP-16, MMP-17, MMP-19, MMP-23, MMP-25 et MMP-26 et les mélanges de celles-ci.

Les séquences peptidiques $S_1$ et $S_2$ sont des séquences linéaires présentant une longueur allant de 4 à 14 acides aminés, ce qui permet aux peptides cycliques les comprenant de présenter une conformation nécessaire pour présenter une activité. $S_1$ et $S_2$ seront avantageusement choisies de sorte à ce que la distance entre leurs extrémités N et C terminales soit comprise entre 10 et 100 Å, une telle distance étant compatible avec la distance effective pour réaliser du FRET entre un groupe donneur et un groupe accepteur tel que défini précédemment. L'utilisation d'un logiciel de modélisation moléculaire permet de prédire avec fiabilité une telle distance.

Les séquences peptidiques $S_1$ et $S_2$ peuvent être issues de séquences peptidiques naturelles rencontrées dans les substrats naturels des MMP ou bien être issues de séquences synthétiques reconnues comme étant des substrats synthétiques des MMP.

Avantageusement, les peptides cycliques de l'invention ne contiennent que des acides aminés naturels, ce qui permet de limiter leur éventuelle cytotoxicité et d'éviter l'introduction d'acides aminés non naturels dans les organismes concernés.

Dans certains cas, il est possible qu'un substrat naturel ou synthétique soit reconnu et clivé par plusieurs MMP. Afin de déterminer quelle MMP est responsable du clivage, il faudra alors utiliser et comparer les données de cinétique enzymatique des différentes MMP pour ce substrat.

Ces données de cinétique enzymatique qui permettent la discrimination entre plusieurs MMP, sont généralement la constante de Mickaelis (Km) qui est l'indicateur d'affinité d'un enzyme pour son substrat et le rapport de la constante catalytique (Kcat, nombre de moles de produits formées par seconde et par mole d'enzyme) sur la constante de Mickaelis qui est l'indicateur de l'efficacité catalytique de l'enzyme. Généralement, il existe donc un couple de valeurs (Km et kcat/Km) spécifique pour un couple MMP/substrat. Il sera donc possible, à titre d'exemple, pour 2 MMP reconnaissant le même substrat de déterminer laquelle est responsable de l'activité visualisée.

Les séquences synthétiques peuvent comprendre, par exemple les acides aminés ou groupes suivants :

Nva (Nor-valine) ; Cha (3-cyclohexylalanine) ; Dpa (N-3(2,4-dinitrophényl)-L-2,3-diaminopropionyle) ; Abu : acide $\alpha$-aminobutyrique ; Cys(Me) correspondant à la S-méthylcystéine, ce qui signifie que le groupe -SH est remplacé par le groupe - $S-CH_3$, D-Arg correspondant à la D-Arginine.

Dans la suite de cet exposé, on précise que les abréviations listées ci-dessous ont les significations suivantes :

Gly : glycine ; Pro : proline ; Leu : leucine ; Alla : alanine ; Gln : glutamine ; Ile : isoleucine ; Arg : arginine ; Val : valine ; Tyr : tyrosine ; Glu : glutamate ; Met : méthionine ; Phe : phénylalanine ; Asn : Asparagine ; Thr : thréonine ; Trp : tryptophane ; Ser : sérine ; His : histidine, ces abréviations correspondant à la nomenclature officielle à 3 lettres des acides aminés.

Lorsque la première séquence $S_1$ et/ou la seconde séquence $S_2$ sont destinées à être cibles de l'enzyme MMP-1, elles correspondent à l'une au moins des séquences suivantes :

-Gly-Pro-Gln-Gly-Leu-Leu-Gly-Ala-SEQ. 1
-Ala-Pro-Gln-Gly-Ile-Ala-Gly-Gln-SEQ. 2
-Gly-Pro-Gln-Gly-Leu-Ala-Gly-Gln-SEQ.3
-Gly-Pro-Leu-Gly-Ile-Ala-Gly-Ile-SEQ.4
-Gly-Pro-Glu-Gly-Leu-Arg-Val-Gly-SEQ.5
-Tyr-Glu-Ala-Gly-Leu-Gly-Val-Val-SEQ.6
-Ala-Gly-Leu-Gly-Val-Val-Glu-Arg-SEQ.7
-Ala-Gly-Leu-Gly-Ile-Ser-Ser-Thr-SEQ.8
-Gly-Ala-Met-Phe-Leu-Glu-Ala-Ile-SEQ.9
-Ile-Pro-Glu-Asn-Phe-Phe-Gly-Val-SEQ.10
-Thr-Glu-Gly-Glu-Ala-Arg-Gly-Ser-SEQ.11
*-Arg-Ala-Ile-His-Ile-Gln-Ala-Glu-SEQ.12*
*-Leu-Arg-Ala-Tyr-Leu-Leu-Pro-Ala-SEQ.13*
*-Gly-Pro-Leu-Gly-Met-Arg-Gly-Leu-SEQ.14*
Deng, S.J., et al. 2000. J. Biol. Chem. 275, 31422
*-Pro-Gln-Gly-Leu-Glu-Ala-Lys-SEQ.15*
Beekman, B., et al. 1996. FEBS Lett 390, 221
*-Pro-Leu-Gly-Leu-Trp-Ala-D-Arg-SEQ. 16*
Bickett, D.M., et al. 1993. Anal. Biochem. 212, 58
Knight, C.G., et al. 1992. FEBS Lett. 296, 263
Darlak, K., et al. 1990. J. Biol. Chem. 265, 5199
Stack, M.S., and Gray, R.D. 1989. J. Biol. Chem. 264, 4277
*-Pro-Leu-Ala-Leu-Trp-Ala-Arg-SEQ. 17*
Netzel-Arnett, S., et al. 1991. Anal. Biochem. 195, 86
-Pro-Cha-Abu-Cys(Me)-His-Ala-SEQ.18
McGeehan, G.M., et al. 1994. J. Biol. Chem. 269, 32814
-Pro-Cha-Gly-Cys(Me)-His-Ala-SEQ. 19
Bickett, D.M., et al. 1993. Anal. Biochem. 212, 58
*-Pro-Cha-Gly-Cys(Me)-His-Ala-SEQ. 20*
Mohan, M.J. et al. 2002. Biochemistry 41, 9462
Hanessian, S. et al. 2001. J. Med. Chem. 44, 3066
Ambrose, W.P. et al. 1998. Anal. Biochem. 263, 150
*-Pro-Leu-Gly-Leu-Dpa-Ala-Arg-SEQ.21*
Knäuper, V. et al. 1996. J. Biol. Chem. 271, 1544
*-Pro-Leu-Gly-[2-mercapto-4-methyl-pentanoyl]-Leu-Gly-SEQ. 22*
Weingarten, H.; Feder, J. 1985. Anal. Biochem. 147, 437

Weingarten, H. et al. 1985. Biochemistry. 24, 6730
*-Pro-Leu-Ala-Leu-Trp-Ala-Arg-SEQ. 17*
A. Santala, A. et al. 1999. FEBS Lett. 461, 153-156
Nagase, H. et al. 1994 J.Biol.Chem. 269, 20952-20957
*-Pro-Leu-Gly-Cys(Me)-His-Ala-D-Arg-SEQ. 23*
J. Berman et al. 1992. J.Biol.Chem. 267, 1434-1437
*-Arg-Pro-Leu-Ala-Leu-Trp-Arg-SEQ. 24*
Kraft, P.J. et al. 2001. Connect.Tissue Res. 42, 149-163
Itoh, M. et al. 1997. J.Pharm.Biomed.Anal. 15, 1417-1426
Welch, A.R. et al. 1995. Arch.Biochem.Biophys. 324, 59-64
*-Pro-Cha-Gly-Nva-His-Ala-SEQ. 25*
Lauer-Fields, J.L. and Fields, G.B. 2002 Biol.Chem. 383, 1095-1105
Lauer-Fields, J.L. et al. 2001. Biochemistry 40, 5795-5803
*-Arg-Pro-Lys-Pro-Tyr-Ala-Nva-Trp-Met-SEQ. 26*
Bremer, C. et al.2002. Acad.Radiol. 9 Suppl 2, S314-S315
Nagase, H. et al. 1994. J.Biol.Chem. 269, 20952-20957

Lorsque la première séquence $S_1$ et/ou la seconde séquence $S_2$ sont destinées à être cibles de l'enzyme MMP-2, elles correspondent à l'une au moins des séquences suivantes :

*-Ile-Pro-Glu-Asn-Phe-Phe-Gly-Val-SEQ.10*
-Pro-Pro-Gly-Ala-Tyr-His-Gly-Ala-SEQ.27
*-Arg-Ala-Ile-His-Ile-Gln-Ala-Glu-SEQ.12*
*-Gly-Pro-His-Leu-Leu-Val-Glu-Ala-SEQ.28*
*-Leu-Arg-Ala-Tyr-Leu-Leu-Pro-Ala-SEQ.13*
-Pro-Gln-Gly-Leu-Glu-Ala-Lys-SEQ.29
Beekman, B., et al. 1996. FEBS Lett 390, 221
*-Pro-Leu-Gly-Leu-Trp-Ala-D-Arg-SEQ. 16*
Bickett, D.M., et al. 1993. Anal. Biochem. 212, 58
Knight, C.G., et al. 1992. FEBS Lett. 296, 263
Darlak, K., et al. 1990. J. Biol. Chem. 265, 5199
Stack, M.S., and Gray, R.D. 1989. J. Biol. Chem. 264, 4277
-Pro-Leu-Ala-Nva-Dpa-Ala-Arg-SEQ.30
Murphy, G., et al. 1994. J. Biol. Chem. 269, 6632
*-Pro-Leu-Gly-Leu-Dpa-Ala-Arg-SEQ.21*
Knight, C.G., et al. 1992. FEBS Lett. 296, 263
*-Pro-Leu-Gly-Met-Trp-Ser-Arg-SEQ. 31*
Netzel-Arnett, S., et al. 1991. Anal. Biochem. 195, 86
*-Pro-Leu-Gly-SCH[$CH_2CH(CH_3)_2$]-CO-Leu-Gly-SEQ. 32*
Weingarten, H., et al. 1985. Anal. Biochem. 147, 437
Weingarten, H., et al. 1985. Biochemistry 24, 6730
*-Arg-Pro-Pro-Gly-Phe-Ser-Ala-Phe-SEQ. 33*
G.D. Johnson and K. Ahn 2000. Anal. Biochem. 286, 112
*-Pro-Leu-Gly-Leu-Dpa-Ala-Arg-SEQ.21*
Knight, C.G. et al. 1992. FEBS Lett. 296, 263
*-Pro-Leu-Gly-[2-mercapto-4-methyl-pentanoyl]-Leu-Gly-SEQ.22*
Weingarten, H.; Feder, J. 1985. Anal. Biochem. 147, 437
Weingarten, H. et al. 1985. Biochemistry. 24, 6730
Xia, T. et al. 1996. Biochim. Biophys. Acta 1293, 259
*-Pro-Leu-Gly-Cys(Me)-His-Ala-D-Arg-SEQ.23*
J. Berman et al., 1992. J.Biol.Chem. 267, 1434-1437
*-Arg-Pro-Leu-Ala-Leu-Trp-Arg-SEQ.24*
Kraft, P.J. et al. 2001. Connect.Tissue Res. 42, 149-163
Itoh, M. et al. 1997. J.Pharm.Biomed.Anal. 15, 1417-1426
Welch, A.R. et al. 1995. Arch.Biochem.Biophys. 324, 59-64
*-Pro-Cha-Gly-Nva-His-Ala-SEQ. 25*
Lauer-Fields, J.L. and Fields, G.B. 2002 Biol.Chem. 383, 1095-1105
Lauer-Fields, J.L. et al. 2001. Biochemistry 40, 5795-5803

*-Arg-Pro-Lys-Pro-Tyr-Ala-Nva-Trp-Met-SEQ. 26*
Bremer, C. et al.2002. Acad.Radiol. 9 Suppl 2, S314-S315
Nagase, H. et al. 1994. J.Biol.Chem. 269, 20952-20957

Lorsque la première séquence $S_1$ et/ou la seconde séquence $S_2$ sont destinées à être cibles de l'enzyme MMP-3, elles correspondent à l'une au moins des séquences suivantes :

- *Gly-Pro-Glu-Gly-Leu-Arg-Val-Gly-SEQ.5*
- Arg-Val-Gly-Phe-Tyr-Glu-Ser-Asp-SEQ.34
- Leu-Leu-Ser-Ala-Leu-Val-Glu-Thr-SEQ.35
- Glu-Ala-Ile-Pro-Met-Ser-Ile-Pro-SEQ.36
- Ile-Ala-Gly-Arg-Ser-Leu-Asn-Pro-SEQ.37
- *Ile-Pro-Glu-Asn-Phe-Phe-Gly-Val-SEQ.10*
- Lys-Pro-Gln-Gln-Phe-Phe-Gly-Leu-SEQ.38
- Asp-Val-Ala-Gln-Phe-Val-Leu-Thr-SEQ.39
- Asp-Thr-Leu-Glu-Val-Met-Arg-Lys-SEQ.40
- Asp-Val-Gly-His-Phe-Arg-Thr-Phe-SEQ.41
- Asp-Ser-Gly-Gly-Phe-Met-Leu-Thr-SEQ.42
- Arg-Val-Ala-Glu-Met-Arg-Gly-Glu-SEQ.43
- Asp-Leu-Gly-Arg-Phe-Gln-Thr-Phe-SEQ.44
- Pro-Phe-Ser-Pro-Leu-Val-Ala-Thr-SEQ.45
- *Leu-Arg-Ala-Tyr-Leu-Leu-Pro-Ala-SEQ.13*
- *Ala-Pro-Gly-Asn-Ala-Ser-Glu-Ser-SEQ.46*
- *Phe-Ser-Ser-Glu-Ser-Lys-Arg-Glu-SEQ.47*
- *Arg-Ala-Ile-His-Ile-Gln-Ala-Glu-SEQ.12*
- *Gly-Pro-His-Leu-Leu-Val-Glu-Ala-SEQ.28*
- Pro-Pro-Glu-Glu-Leu-Lys-Phe-Gln-SEQ.48
- *Gly-Pro-Leu-Gly-Met-Arg-Gly-Leu-SEQ. 49*
  Deng, S.J., et al. 2000. J. Biol. Chem. 275, 31422
- *Pro-Gln-Gly-Leu-Glu-Ala-Lys-SEQ. 15*
  Beekman, B., et al. 1996. FEBS Lett 390, 221
  -Arg-Pro-Lys-Pro-Val-Glu-Nva-Trp-Arg-Glu-Ala-Lys-SEQ. 50
  Beekman, B., et al. 1997. FEBS Lett 418, 305
- *Pro-Leu-Gly-Leu-Trp-Ala-D-Arg-SEQ. 16*
  Bickett, D.M., et al. 1993. Anal. Biochem. 212, 58
  Knight, C.G., et al. 1992. FEBS Lett. 296, 263
  Darlak, K., et al. 1990. J. Biol. Chem. 265, 5199
  Stack, M.S., and Gray, R.D. 1989. J. Biol. Chem. 264, 4277
- *Pro-Tyr-Ala-Tyr-Trp-Met-Arg-SEQ. 51*
  Netzel-Arnett, S., et al. 1991. Anal. Biochem. 195, 86
- *Arg-Pro-Lys-Pro-Leu-Ala-Nva-Trp-SEQ. 52*
  Bickett, D.M., et al. 1994. Ann. N.Y. Acad. Sci. 732, 351
- *Pro-Cha-Gly-Cys(Me)-His-Ala-SEQ. 20*
  Mohan, M.J. et al. 2002. Biochemistry 41, 9462
  Hanessian, S. et al. 2001. J. Med. Chem. 44, 3066
  Ambrose, W.P. et al. 1998. Anal. Biochem. 263, 150
- *Pro-Leu-Gly-Leu-Dpa-Ala-Arg-SEQ. 21*
  Knight, C.G. et al. 1992. FEBS Lett. 296, 263
  *-Pro-Leu-Gly-[2-mercapto-4-methyl-pentanoyl]-Leu-Gly-SEQ. 22*
  Weingarten, H.; Feder, J. 1985. Anal. Biochem. 147, 437
  Weingarten, H. et al. 1985. Biochemistry. 24, 6730
- *Arg-Pro-Lys-Pro-Tyr-Ala-Nva-Trp-Met-SEQ. 26*
  Bremer, C. et al.2002. Acad.Radiol. 9 Suppl 2, S314-S315
  Nagase, H. et al. 1994. J.Biol.Chem. 269, 20952-20957
- *Arg-Pro-Lys-Pro-Val-Glu-Nva-Trp-Arg-SEQ. 50*
  Bremer, C. et al.2002. Acad.Radiol. 9 Suppl 2, S314-S315
  Nagase, H. et al. 1994. J.Biol.Chem. 269, 20952-20957

Lorsque la première séquence $S_1$ et/ou la seconde séquence $S_2$ sont destinées à être cibles de l'enzyme MMP-5, elles correspondent à l'une au moins des séquences suivantes :

*-Arg-Pro-Lys-Pro-Val-Glu-Nva-Trp-Arg-Glu-Ala-Lys-SEQ. 50*
Beekman, B., et al. 1997. FEBS Lett 418, 305

Lorsque la première séquence $S_1$ et/ou la seconde séquence $S_2$ sont destinées à être cibles de l'enzyme MMP-7, elles correspondent à l'une au moins des séquences suivantes :

*-Pro-Leu-Gly-Leu-Trp-Ala-D-Arg-SEQ. 16*
Bickett, D.M., et al. 1993. Anal. Biochem. 212, 58
Knight, C.G., et al. 1992. FEBS Lett. 296, 263
Darlak, K., et al. 1990. J. Biol. Chem. 265, 5199
Stack, M.S., and Gray, R.D. 1989. J. Biol. Chem. 264, 4277
*-Pro-Leu-Gly-Leu-Dpa-Ala-Arg-SEQ. 21*
Knight, C.G., et al. 1992. FEBS Lett. 296, 263
*-Arg-Pro-Leu-Ala-Leu-Trp-Arg-Ser-SEQ. 24*
Welch, A.R., et al. 1996. Biochemistry 35, 10103
Welch, A.R., et al. 1995. Arch. Biochem. Biophys. 324, 59
*-Pro-Cha-Gly-Cys(Me)-His-Ala-SEQ. 20*
Mohan, M.J. et al. 2002. Biochemistry 41, 9462
Hanessian, S. et al. 2001. J. Med. Chem. 44, 3066
Ambrose, W.P. et al. 1998. Anal. Biochem. 263, 150
*-Pro-Leu-Gly-[2-mercapto-4-methyl-pentanoyl]-Leu-Gly-SEQ. 22*
Weingarten, H.; Feder, J. 1985. Anal. Biochem. 147, 437
Weingarten, H. et al. 1985. Biochemistry. 24, 6730
*-Pro-Leu-Ala-Leu-Trp-Ala-Arg-SEQ. 17*
A. Santala, A. et al. 1999. FEBS Lett. 461, 153-156
Shabani, F. et al.1998. Free Radic.Res. 28, 115-123
Nagase, H. et al. 1994 J.Biol.Chem. 269, 20952-20957
*-Pro-Tyr-Ala-Tyr-Trp-Met-Arg-SEQ. 51*
Finch-Arietta, M. et al., 1993. Agents Actions 39 SpecNo,C189-C191
Netzel-Arnett, S. et al. 1991 Anal.Biochem.195, 86-92
*-Arg-Pro-Lys-Pro-Leu-Ala-Nva-Trp-SEQ. 52*
Finch-Arietta, M. et al., 1993. Agents Actions 39 SpecNo,C189-C191
Bickett, D.M. et al., 1994. Ann.N.Y.Acad.Sci. 732, 351-355

Lorsque la première séquence $S_1$ et/ou la seconde séquence $S_2$ sont destinées à être cibles de l'enzyme MMP-8, elles correspondent à l'une au moins des séquences suivantes :

*-Pro-Leu-Ala-Tyr-Trp-Ala-Arg-SEQ. 53*
Netzel-Arnett, S., et al. 1991. Anal. Biochem. 195, 86
*-Pro-Leu-Gly-Leu-Trp-Ala-D-Arg-SEQ. 16*
Grams, F. et al. 2001. Biol. Chem. 382, 1277
*-Pro-Cha-Gly-Cys(Me)-His-Ala-SEQ. 20*
Mohan, M.J. et al. 2002. Biochemistry 41, 9462
Hanessian, S. et al. 2001. J. Med. Chem. 44, 3066
Ambrose, W.P. et al. 1998. Anal. Biochem. 263, 150
*-Pro-Leu-Gly-Leu-Dpa-Ala-Arg-SEQ. 21*
Knäuper, V. et al. 1996. J. Biol. Chem. 271,1544
*-Pro-Leu-Gly-[2-mercapto-4-methyl-pentanoyl]-Leu-Gly-SEQ. 22*
Weingarten, H.; Feder, J. 1985. Anal. Biochem. 147, 437
Weingarten, H. et al. 1985. Biochemistry. 24, 6730
*-Pro-Leu-Ala-Leu-Trp-Ala-Arg-SEQ. 17*
A. Santala, A. et al. 1999. FEBS Lett. 461, 153-156
Shabani, F. et al.1998. Free Radic.Res. 28, 115-123
Nagase, H. et al. 1994 J.Biol.Chem. 269, 20952-20957
*-Pro-Leu-Gly-Cys(Me)-His-Ala-D-Arg-SEQ. 23*

J. Berman et al., 1992. J.Biol.Chem. 267, 1434-1437
*-Arg-Pro-Leu-Ala-Leu-Trp-Arg-SEQ. 24*
Kraft, P.J. et al. 2001. Connect.Tissue Res. 42, 149-163
Itoh, M. et al. 1997. J.Pharm.Biomed.Anal. 15, 1417-1426
Welch, A.R. et al. 1995. Arch.Biochem.Biophys. 324, 59-64
*-Pro-Cha-Gly-Nva-His-Ala-SEQ. 25*
Lauer-Fields, J.L. and Fields, G.B. 2002 Biol.Chem. 383, 1095-1105
Lauer-Fields, J.L. et al. 2001. Biochemistry 40, 5795-5803

Lorsque la première séquence $S_1$ et/ou la seconde séquence $S_2$ sont destinées à être cibles de l'enzyme MMP-9, elles correspondent à l'une au moins des séquences suivantes :

-Gly-Pro-Pro-Gly-Val-Val-Gly-Pro-SEQ.54

-Gly-Pro-Pro-Gly-Leu-Arg-Gly-Glu-SEQ.55

-Gly-Pro-Gly-Gly-Val-Val-Gly-Pro-SEQ.56

-Ile-Pro-Gln-Asn-Phe-Phe-Gly-Val-SEQ.57

-Pro-Pro-Gly-Ala-Tyr-His-Gly-Ala-SEQ.58

*-Arg-Ala-Ile-His-Ile-Gln-Ala-Glu-SEQ.12*

*-Gly-Pro-Leu-Gly-Met-Arg-Gly-Leu-SEQ. 14*
Deng, S.J., et al. 2000. J. Biol. Chem. 275, 31422

*-Pro-Gln-Gly-Leu-Glu-Ala-Lys-SEQ. 15*
Beekman, B., et al. 1996. FEBS Lett 390, 221
*-Arg-Pro-Lys-Pro-Val-Glu-Nva-Trp-Arg-Glu-Ala-Lys-SEQ.50*
Beekman, B., et al. 1997. FEBS Lett 418, 305

*-Pro-Leu-Gly-Leu-Trp-Ala-D-Arg-SEQ. 16*
Bickett, D.M., et al. 1993. Anal. Biochem. 212, 58
Knight, C.G., et al. 1992. FEBS Lett. 296, 263
Darlak, K., et al. 1990. J. Biol. Chem. 265, 5199
Stack, M.S., and Gray, R.D. 1989. J. Biol. Chem. 264, 4277

*-Pro-Cha-Gly-Cys(Me)-His-Ala-SEQ. 19*
Bickett, D.M., et al. 1993. Anal. Biochem. 212, 58
*-Pro-Leu-Gly-SCH[$CH_2CH(CH_3)_2$]-CO-Leu-Gly-SEQ. 32*
Weingarten, H., et al. 1985. Anal. Biochem. 147, 437
Weingarten, H., et al. 1985. Biochemistry 24, 6730

*-Arg-Pro-Pro-Gly-Phe-Ser-Ala-Phe-SEQ. 33*
G.D. Johnson and K. Ahn 2000. Anal. Biochem. 286, 112

*-Pro-Cha-Gly-Cys(Me)-His-Ala-SEQ. 20*
Mohan, M.J. et al. 2002. Biochemistry 41, 9462
Hanessian, S. et al. 2001. J. Med. Chem. 44, 3066
Ambrose, W.P. et al. 1998. Anal. Biochem. 263, 150

*-Pro-Leu-Gly-Leu-Dpa-Ala-Arg-SEQ. 21*
Roy, N. et al. 1999. Prot. Expr. Purif. 16, 324
*-Pro-Leu-Gly-[2-mercapto-4-methyl-pentanoyl]-Leu-Gly-SEQ. 22*
Weingarten, H.; Feder, J. 1985. Anal. Biochem. 147, 437
Weingarten, H. et al. 1985. Biochemistry. 24, 6730
Xia, T. et al. 1996. Biochim. Biophys. Acta 1293, 259

*-Pro-Leu-Gly-Cys(Me)-His-Ala-D-Arg-SEQ. 23*
J. Berman et al., 1992. J.Biol.Chem. 267, 1434-1437

Lorsque la première séquence $S_1$ et/ou la seconde séquence $S_2$ sont destinées à être cibles de l'enzyme MMP-10, elles correspondent à l'une au moins des séquences suivantes :

*-Arg-Ala-Ile-His-Ile-Gln-Ala-Glu-SEQ.12*

*-Gly-Pro-His-Leu-Leu-Val-Glu-Ala-SEQ.28*

*-Pro-Leu-Gly-Leu-Dpa-Ala-Arg-SEQ. 21*
Kannan, R. et al. 1999. Prot. Expr. Purif. 16, 76

Lorsque la première séquence $S_1$ et/ou la seconde séquence $S_2$ sont destinées à être cibles de l'enzyme MMP-11, elles correspondent à l'une au moins des séquences suivantes :

*-Pro-Cha-Gly-Cys(Me)-His-Ala-SEQ. 20*
Mohan, M.J. et al. 2002. Biochemistry 41, 9462
Hanessian, S. et al. 2001. J. Med. Chem. 44, 3066
Ambrose, W.P. et al. 1998. Anal. Biochem. 263, 150
*-Pro-Leu-Gly-Leu-Dpa-Ala-Arg-SEQ. 21*
Kannan, R. et al. 1999. Prot. Expr. Purif. 16, 76

Lorsque la première séquence $S_1$ et/ou la seconde séquence $S_2$ sont destinées à être cibles de l'enzyme MMP-12, elles correspondent à l'une au moins des séquences suivantes :

*-Pro-Cha-Gly-Cys(Me)-His-Ala-SEQ. 20*
Mohan, M.J. et al. 2002. Biochemistry 41, 9462
Hanessian, S. et al. 2001. J. Med. Chem. 44, 3066
Ambrose, W.P. et al. 1998. Anal. Biochem. 263, 150
*-Pro-Leu-Gly-Leu-Dpa-Ala-Arg-SEQ. 21*
Park, H.I. et al. 2000. J. Biol. Chem. 275, 20540
*-Pro-Leu-Gly-[2-mercapto-4-methyl-pentanoyl]-Leu-Gly-SEQ. 22*
Weingarten, H.; Feder, J. 1985. Anal. Biochem. 147, 437
Weingarten, H. et al. 1985. Biochemistry. 24, 6730
*-Pro-Leu-Ala-Leu-Trp-Ala-Arg-SEQ. 17*
A. Santala, A. et al. 1999. FEBS Lett. 461, 153-156
Shabani, F. et al.1998. Free Radic.Res. 28, 115-123
Nagase, H. et al. 1994 J.Biol.Chem. 269, 20952-20957
*-Pro-Leu-Gly-Cys(Me)-His-Ala-D-Arg-SEQ. 23*
Berman, J. et al., 1992. J.Biol.Chem. 267, 1434-1437
*-Pro-Tyr-Ala-Tyr-Trp-Met-Arg-SEQ. 51*
Finch-Arietta, M. et al., 1993. Agents Actions 39 SpecNo,C189-C191
Netzel-Arnett, S. et al. 1991 Anal.Biochem.195, 86-92
*-Arg-Pro-Lys-Pro-Leu-Ala-Nva-Trp-SEQ. 52*
Finch-Arietta, M. et al., 1993. Agents Actions 39 SpecNo,C189-C191
Bickett, D.M. et al., 1994. Ann.N.Y.Acad.Sci. 732, 351-355
*-Arg-Pro-Leu-Ala-Leu-Trp-Arg-SEQ. 24*
Kraft, P.J. et al. 2001. Connect.Tissue Res. 42, 149-163
Itoh, M. et al. 1997. J.Pharm.Biomed.Anal. 15, 1417-1426
Welch, A.R. et al. 1995. Arch.Biochem.Biophys. 324, 59-64
*-Pro-Cha-Gly-Nva-His-Ala-SEQ. 25*
Lauer-Fields, J.L. and Fields, G.B. 2002 Biol.Chem. 383, 1095-1105
Lauer-Fields, J.L. et al. 2001. Biochemistry 40, 5795-5803
*-Arg-Pro-Lys-Pro-Tyr-Ala-Nva-Trp-Met-SEQ. 26*
Bremer, C. et al. 2002. Acad.Radiol. 9 Suppl 2, S314-S315
Nagase, H. et al. 1994. J.Biol.Chem. 269, 20952-20957
*-Arg-Pro-Lys-Pro-Val-Glu-Nva-Trp-Arg-SEQ. 50*

Bremer, C. et al.2002. Acad.Radiol. 9 Suppl 2, S314-S315
Nagase, H. et al. 1994. J.Biol.Chem. 269, 20952-20957
*-Arg-Pro-Lys-Pro-Gln-Gln-Phe-Trp-SEQ. 59*
Bickett, D.M. et al. 1994. Ann.N.Y.Acad.Sci. 732, 351-355

Lorsque la première séquence $S_1$ et/ou la seconde séquence $S_2$ sont destinées à être cibles de l'enzyme MMP-13, elles correspondent à l'une au moins des séquences suivantes :

*-Gly-Pro-Leu-Gly-Met-Arg-Gly-Leu-SEQ. 49*
Deng, S.J., et al. 2000. J. Biol. Chem. 275, 31422
*-Arg-Pro-Lys-Pro-Val-Glu-Nva-Trp-Arg-Glu-Ala-Lys-SEQ. 50*
Beekman, B., et al. 1997. FEBS Lett 418, 305
*-Pro-Cha-Gly-Nva-His-Ala-Dpa-SEQ. 25*
Knauper, V., et al. 1996. J. Biol. Chem. 271, 1544
*-Pro-Cha-Gly-Cys(Me)-His-Ala-SEQ. 20*
Mohan, M.J. et al. 2002. Biochemistry 41, 9462
Hanessian, S. et al. 2001. J. Med. Chem. 44, 3066
Ambrose, W.P. et al. 1998. Anal. Biochem. 263, 150
*-Pro-Leu-Gly-Leu-Dpa-Ala-Arg-SEQ. 21*
Knäuper, V. et al. 1996. J. Biol. Chem. 271, 1544
*-Pro-Leu-Gly-[2-mercapto-4-methyl-pentanoyl]-Leu-Gly-SEQ.22*
Weingarten, H.; Feder, J. 1985. Anal. Biochem. 147, 437
Weingarten, H. et al. 1985. Biochemistry. 24, 6730
*-Pro-Leu-Gly-Leu-Trp-Ala-D-Arg-SEQ. 16*
Netzel-Arnett, S. et al. 1991. Anal.Biochem.195, 86-92
Santala, A. et al. 1999. FEBS Lett. 461, 153-156
Bickett, D.M. et al., 1993. Anal.Biochem. 212, 58-64
*-Pro-Leu-Ala-Leu-Trp-Ala-Arg-SEQ. 17*
A. Santala, A. et al. 1999. FEBS Lett. 461, 153-156
Shabani, F. et al.1998. Free Radic.Res. 28, 115-123
Nagase, H. et al. 1994 J.Biol.Chem. 269, 20952-20957
*-Pro-Leu-Gly-Cys(Me)-His-Ala-D-Arg-SEQ. 23*
J. Berman et al., 1992. J.Biol.Chem. 267, 1434-1437
*-Pro-Leu-Gly-Met-Trp-Ser-Arg-SEQ. 31*
Netzel-Arnett, S. et al.1991. Anal.Biochem.195, 86-92
d'Ortho, M.P. et al., 1997. Eur.J.Biochem. 250, 751-757
*-Pro-Tyr-Ala-Tyr-Trp-Met-Arg-SEQ. 51*
Finch-Arietta, M. et al., 1993. Agents Actions 39 SpecNo,C189-C191
Netzel-Arnett, S. et al. 1991 Anal.Biochem.195, 86-92
*-Arg-Pro-Lys-Pro-Leu-Ala-Nva-Trp-SEQ. 52*
Finch-Arietta, M. et al., 1993. Agents Actions 39 SpecNo,C189-C191
Bickett, D.M. et al., 1994. Ann.N.Y.Acad.Sci. 732, 351-355
*-Arg-Pro-Leu-Ala-Leu-Trp-Arg-SEQ. 24*
Kraft, P.J. et al. 2001. Connect.Tissue Res. 42, 149-163
Itoh, M. et al. 1997. J.Pharm.Biomed.Anal. 15, 1417-1426
Welch, A.R. et al. 1995. Arch.Biochem.Biophys. 324, 59-64
*-Pro-Leu-Ala-Tyr-Trp-Ala-Arg-SEQ. 53*
Aschi, M. et al. 2002. J.Comput.Aided Mol.Des 16, 213-225
Netzel-Arnett, S. et al. 1991. Anal.Biochem.195, 86-92
*-Pro-Cha-Gly-Nva-His-Ala-SEQ. 25*
Lauer-Fields, J.L. and Fields, G.B. 2002 Biol.Chem. 383, 1095-1105
Lauer-Fields, J.L. et al. 2001. Biochemistry 40, 5795-5803
*-Arg-Pro-Lys-Pro-Tyr-Ala-Nva-Trp-Met-SEQ. 26*
Bremer, C. et al.2002. Acad.Radiol. 9 Suppl 2, S314-S315
Nagase, H. et al. 1994. J.Biol.Chem. 269, 20952-20957
*-Arg-Pro-Lys-Pro-Gln-Gln-Phe-Trp-SEQ. 59*
Bickett, D.M. et al. 1994. Ann.N.Y.Acad.Sci. 732, 351-355

Lorsque la première séquence $S_1$ et/ou la seconde séquence $S_2$ sont destinées à être cibles de l'enzyme MMP-14, elles correspondent à l'une au moins des séquences suivantes :

-Pro-Leu-Ala-Cys(p-OMeBz)-Trp-Ala-Arg-SEQ. 60
Mucha, A., et al. 1998. J. Biol. Chem. 273, 2763
Holtz, B., et al. 1999. Biochemistry 38, 12174

*-Pro-Cha-Gly-Cys(Me)-His-Ala-SEQ. 20*
Mohan, M.J. et al. 2002. Biochemistry 41, 9462
Hanessian, S. et al. 2001. J. Med. Chem. 44, 3066
Ambrose, W.P. et al. 1998. Anal. Biochem. 263, 150

*-Pro-Leu-Gly-Leu-Dpa-Ala-Arg-SEQ. 21*
Roderfeld, M. et al. 2000 . Prot. Expr. Purif. 19, 369
Kannan, R. et al. 1999. Prot. Expr. Purif. 16, 76

*-Pro-Leu-Gly-[2-mercapto-4-methyl-pentanoyl]-Leu-Gly-SEQ. 22*
Weingarten, H.; Feder, J. 1985. Anal. Biochem. 147, 437
Weingarten, H. et al. 1985. Biochemistry. 24, 6730

Lorsque la première séquence $S_1$ et/ou la seconde séquence $S_2$ sont destinées à être cibles de l'enzyme MMP-15, elles correspondent à l'une au moins des séquences suivantes :

*-Pro-Leu-Gly-Leu-Dpa-Ala-Arg-SEQ. 21*
Xue, C.-B. et al. 2001. J. Med. Chem. 44, 2636

Lorsque la première séquence $S_1$ et/ou la seconde séquence $S_2$ sont destinées à être cibles de l'enzyme MMP-16, elles correspondent à l'une au moins des séquences suivantes :

*-Pro-Leu-Gly-Leu-Dpa-Ala-Arg-SEQ. 21*
Shimada, T. et al. 1999. Eur. J. Biochem. 262, 907
Xue, C.-B. et al. 2001. J. Med. Chem. 44, 2636

Lorsque la première séquence $S_1$ et/ou la seconde séquence $S_2$ sont destinées à être cibles de l'enzyme MMP-17, elles correspondent à l'une au moins des séquences suivantes :

*-Pro-Leu-Gly-Leu-Dpa-Ala-Arg-SEQ. 21*
English, W.R. et al. 2001. FEBS Lett. 491, 137
*-Pro-Leu-Gly-[2-mercapto-4-methyl-pentanoyl]-Leu-Gly-SEQ. 22*

Lorsque la première séquence $S_1$ et/ou la seconde séquence $S_2$ sont destinées à être cibles de l'enzyme MMP-19, elles correspondent à l'une au moins des séquences suivantes :

*-Pro-Leu-Gly-Leu-Dpa-Ala-Arg-SEQ. 21*
*Pendás, A.M. et al. 1997. J. Biol. Chem.* ***272****, 4281*

Lorsque la première séquence $S_1$ et/ou la seconde séquence $S_2$ sont destinées à être cibles de l'enzyme MMP-23, elles correspondent à l'une au moins des séquences suivantes :

*-Pro-Leu-Gly-Leu-Dpa-Ala-Arg-SEQ. 21*
Velasco, G. et al. 1999. J. Biol. Chem. 274, 4570

Lorsque la première séquence $S_1$ et/ou la seconde séquence $S_2$ sont destinées à être cibles de l'enzyme MMP-25, elles correspondent à l'une au moins des séquences suivantes :

*-Pro-Leu-Gly-Leu-Dpa-Ala-Arg-SEQ. 21*
English, W.R. et al. 2001. FEBS Lett. 491, 137

Lorsque la première séquence $S_1$ et/ou la seconde séquence $S_2$ sont destinées à être cibles de l'enzyme MMP-26, elles correspondent à l'une au moins des séquences suivantes :

*-Pro-Leu-Gly-Leu-Trp-Ala-D-Arg-SEQ. 16*
Park, H.I. et al. 2002. J. Biol. Chem. 277, 35168

Comme il ressort de ce qui précède, une MMP donnée est susceptible de reconnaître plusieurs séquences, ces séquences appartenant à l'origine à des protéines distinctes, qui interviennent selon des mécanismes précis dans certaines pathologies.
Comme mentionné ci-dessus, les peptides cycliques de l'invention comprennent au moins une sonde $X_1$ porteuse d'un groupe donneur fluorescent et au moins une sonde $X_2$ porteur d'un groupe accepteur fluorescent ou non fluorescent. Ceux-ci sont généralement choisis de sorte à ce que le spectre d'émission de fluorescence du groupe donneur recouvre, au moins en partie, le spectre d'absorption du groupe accepteur, ce qui se traduit par un phénomène de transfert non radiatif de l'énergie d'excitation du groupe donneur vers le groupe accepteur (ou transfert d'énergie de résonance).
De tels groupes comprennent classiquement un noyau aromatique, tel qu'un noyau benzénique, anthracénique ou coumarine.
On peut citer, par exemple, les couples suivants (le premier membre du couple étant le groupe donneur, tandis que le second membre du couple étant le groupe accepteur) :

*Tryptophane/2,4-dinitrophényle (symbolisé par l'abréviation W/Dnp) ;
*Acide o-aminobenzoïque/2,4-dinitrophényle (symbolisé par l'abréviation Abz/Dnp) ;
*(7-méthoxycoumarin-4-yl)-acétyle/2,4-dinitrophényle (symbolisé par l'abréviation Mca/Dnp) ;
*(7-méthoxycoumarin-4-yl)-acétyle/N-3-(2,4-dinitrophényl)-L-2,3-diaminopropyle (symbolisé par l'abréviation Mca/Dpa) ;
*Tryptophane/Dansyle (symbolisé par l'abréviation W/Dns) ;
*N-méthylanthranoyle/2,4-dinitrophényle (symbolisé par l'abréviation Nma/Dnp) ;
*6,7-diméthoxycoumarin-4-yl-acétyle/acide 6-N-(7-nitrobenz-2-oxa-1,3-diazol-4-yl)aminohexanoïque (symbolisé par l'abréviation DMC/Nbd) ;
*Acide 5-(2'-aminométhyl)naphtalène sulfonique/acide 4-(4'-diméthylaminophénylaza)benzoïque (symbolisé par l'abréviation EDANS/Dabcyl) ;
* acide 7-méthoxycoumarin-3-carboxylique/acide 7-diéthylaminocoumarin-3-carboxylique/ (symbolisé par l'abréviation MC/DAC), lesdits groupes répondant aux formules suivantes :

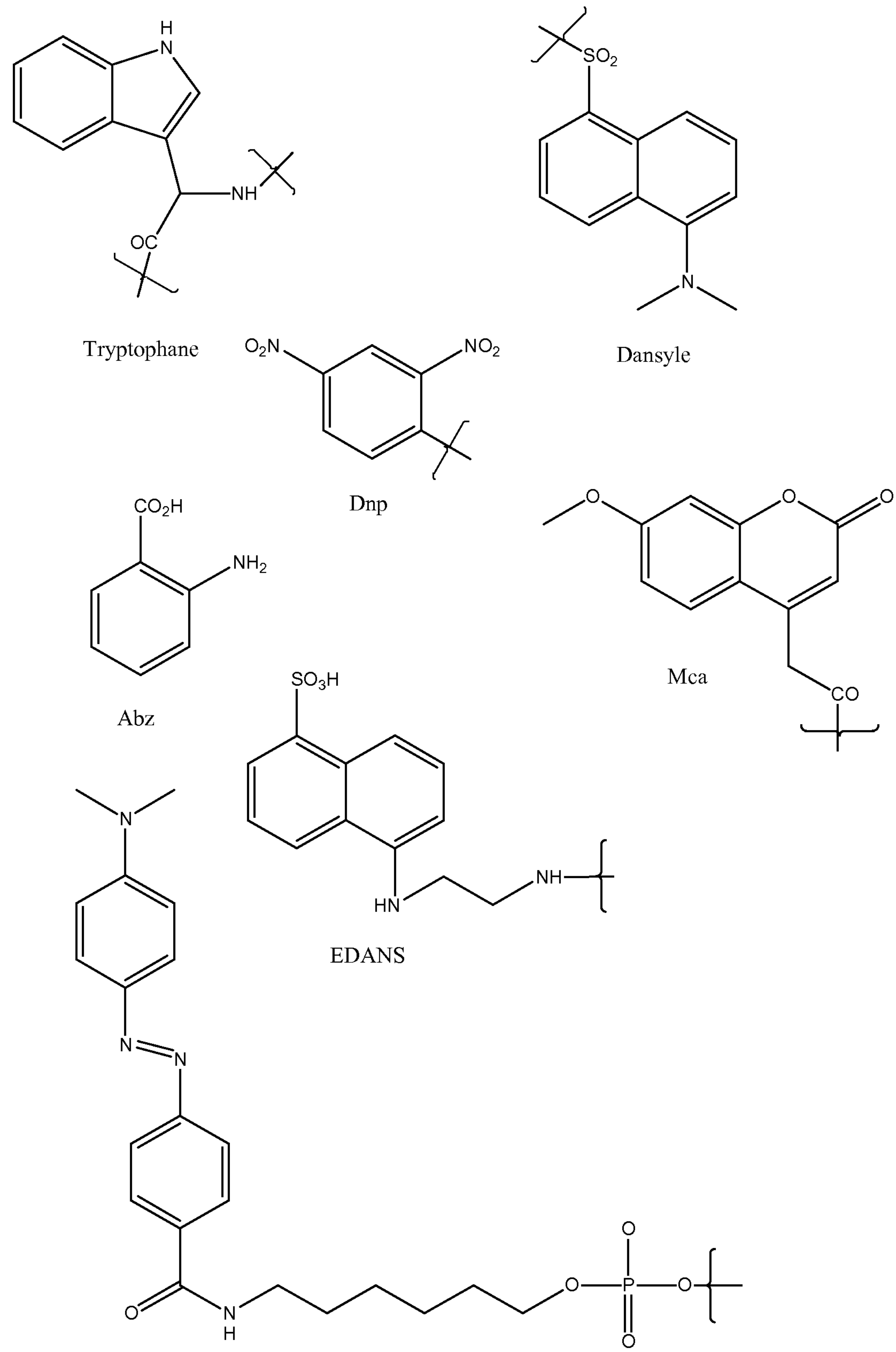

Tryptophane

Dansyle

Dnp

Abz

Mca

EDANS

DABCYL

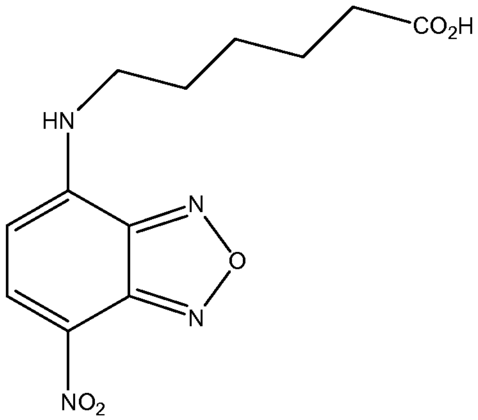

Nbd

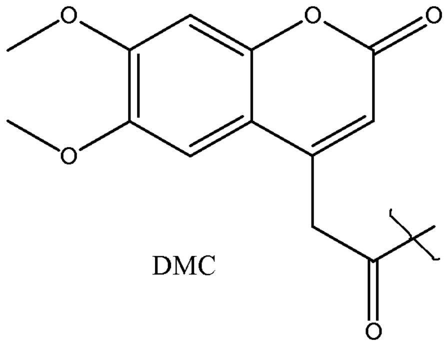

DMC

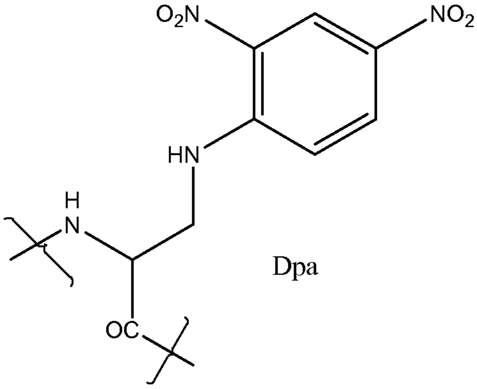

Dpa

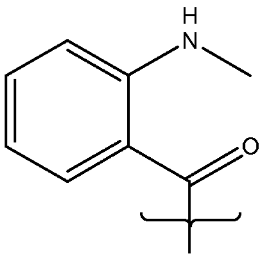

Nma

Avantageusement, le groupe donneur et le groupe accepteur sont des groupes comprenant un noyau coumarine.

Du fait que ces groupes présentent un motif aromatique commun du type coumarine, cela permet la formation d'un complexe de nature hydrophobe ou du type π-stacking, qui participe à la stabilité des peptides cycliques de l'invention.

En particulier, le couple préféré peut être le couple MC/DAC, ayant pour formules respectives, une fois liés à la sonde, les formules suivantes :

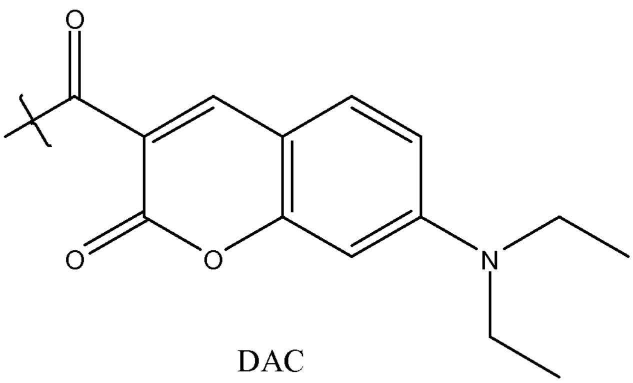

DAC

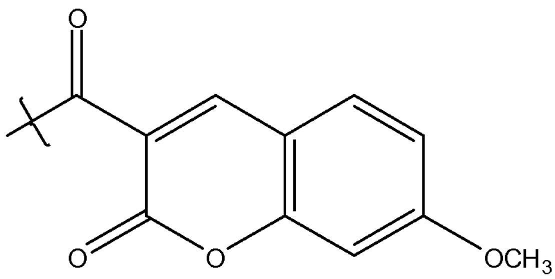

MC

Les sondes $X_1$ et $X_2$ peuvent être des restes d'acide aminé.

Un exemple de sonde $X_1$ peut être ainsi la sonde -Lys(MC)- de formule suivante :

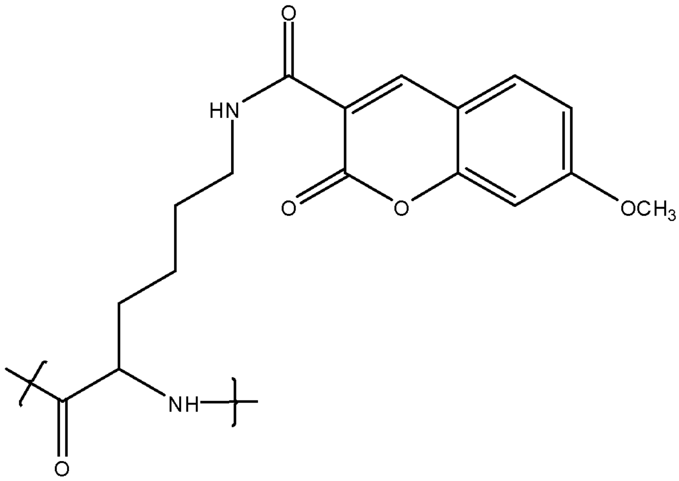

tandis qu'un exemple de sonde $X_2$ peut être la sonde -Lys(DAC)- de formule suivante :

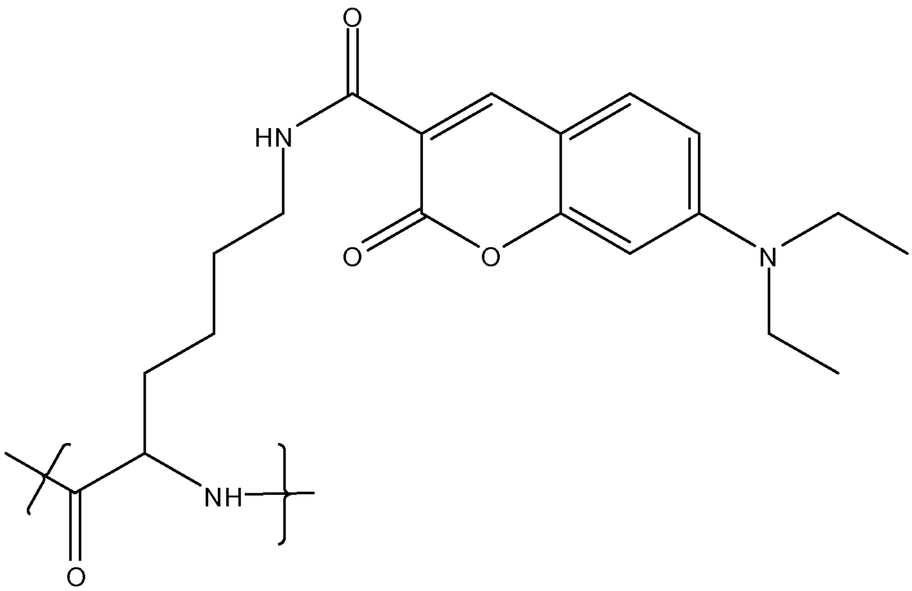

$S_1$ et $S_2$ peuvent correspondre à une séquence cible d'une MMP telle que définie ci-dessus, en particulier à la séquence suivante :

-Gly-Pro-Gln-Gly-Leu-Leu-Gly-Ala-
ou
-Gly-Pro-Gly-Gly-Val-Val-Gly-Pro

ces séquences correspondant respectivement à des séquences reconnues par l'enzyme MMP-1 et l'enzyme MMP-9.

Un peptide cyclique conforme à l'invention peut être un peptide cyclique de formule suivante :

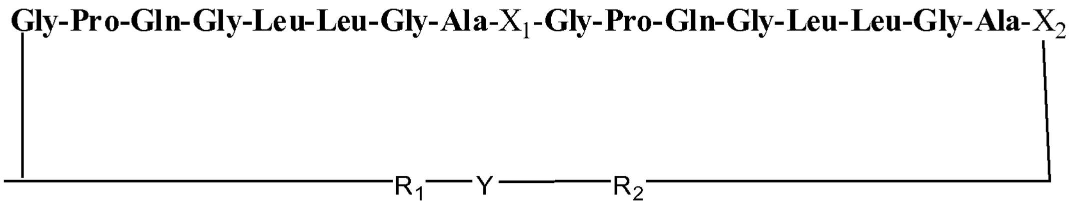

$X_1$, $X_2$, $R_1$, Y et $R_2$ répondant à la même définition que celle donnée ci-dessus.

Un peptide cyclique précis conforme à l'invention répond à la formule suivante :

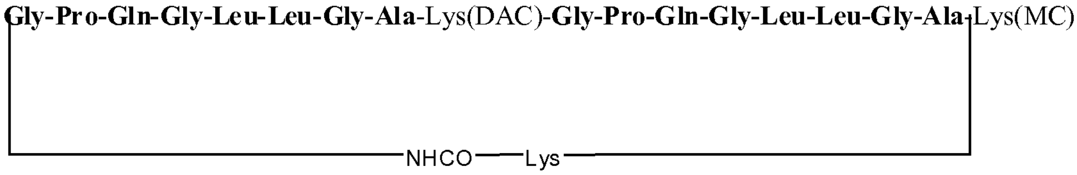

les groupes -Lys(DAC)- et -Lys(MC)- répondant à la même définition que celle donnée ci-dessus.

Les séquences en gras seront clivées en présence de l'enzyme MMP1, contribuant ainsi à éloigner l'une de l'autre les sondes Lys(Dac) et Lys(MC). S'ensuit une variation du transfert de résonance, permettant ainsi de quantifier l'activité de l'enzyme MMP1.

Les peptides cycliques de l'invention peuvent être amenés, par le biais de fonctions pendantes (telles que des fonctions amines issues d'un reste de lysine ou d'acide aspartique), à être fixés sur un support, tel que des particules.

Les peptides cycliques de l'invention peuvent être préparés par des procédés mettant en jeu une étape de synthèse automatique sur une phase solide par un procédé classique, suivie d'un couplage des extrémités du peptide linéaire, soit après avoir libéré le peptide de la phase solide, soit en le libérant ensuite de la phase solide.

La phase solide est classiquement une résine porteuse de groupes aptes à réagir avec une fonction $-NH_2$ ou -COOH de façon à former une liaison covalente, cette liaison étant destinée à être clivée ultérieurement, de façon à libérer le peptide une fois synthétisé.

De telles résines peuvent être des résines trityl, 2-chloro chloro trityle porteuses respectivement de groupes de formules suivantes :

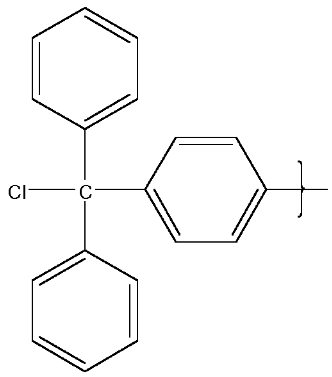

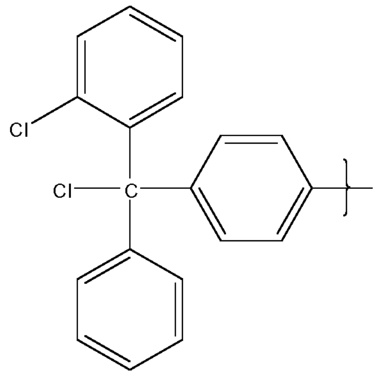

les accolades indiquant l'endroit par lequel les groupes susmentionnés sont fixés à la résine.

Selon un premier mode de réalisation, le procédé de l'invention comprend :

a) une étape de préparation d'un peptide linéaire par synthèse chimique sur une phase solide ;

b) une étape de libération du peptide linéaire de la phase solide ; et

c) une étape de couplage des extrémités du peptide linéaire pour former le peptide cyclique.

Selon un second mode de réalisation, le procédé de l'invention comprend :

a) une étape de préparation du peptide linéaire par synthèse chimique sur une phase solide ;

b) une étape de couplage de l'extrémité libre du peptide linéaire avec une fonction terminale d'un résidu linéaire du peptide linéaire ; et

c) une étape de libération du peptide cyclique de la phase solide, les étapes b) et c) pouvant être réalisées de façon concomitante.

Que ce soit pour le premier mode ou le second mode de réalisation, dans le cadre de la réalisation de l'étape a), la première phase consiste à ancrer un premier acide aminé sur la phase solide.

Cet ancrage peut se réaliser de différentes façons :

- l'ancrage sur la phase solide par une extrémité C-terminale du premier acide aminé ;
- l'ancrage sur la phase solide par la chaîne latérale du premier acide aminé ;
- l'ancrage sur la phase solide par la chaîne principale du premier acide aminé.

Pour le premier type d'ancrage, une fois le peptide synthétisé, la fonction N- terminale déprotégée du peptide réagit, selon un mécanisme d'addition nucléophile, sur la fonction carboxyle libérée ou ancrée encore sur la résine. Dans le cas où la fonction carboxyle est encore ancrée sur la résine, la réaction d'addition nucléophile (étant une addition intramoléculaire) réalise conjointement la cyclisation et la libération du peptide de la résine.

Le deuxième type d'ancrage se faisant par la chaîne latérale d'un acide aminé est généralement dévolu aux acides aminés suivants : asparagine, acide aspartique, glutamine, glutamate, lysine, histidine, serine, thréonine, arginine et tyrosine, qui ont la particularité de présenter une chaîne latérale porteuse d'une fonction, telle que $-CO_2H$, OH, $NH_2$ ou $NH_2CO$, apte à réagir avec un groupe réactif porté par la résine.

Pour la troisième approche fondée sur l'ancrage de la chaîne principale sur la résine, le peptide synthétisé est lié à la résine par un des atomes d'azote d'une liaison amide de la chaîne principale.

Il est entendu, que l'homme du métier utilisera, pour synthétiser la séquence voulue, usera de groupements protecteurs appropriés.

Comme mentionné ci-dessus, les peptides cycliques de l'invention sont destinés à être utilisés pour suivre l'activité d'une enzyme protéase E.

Plus précisément, l'utilisation des peptides cycliques de l'invention peut être envisagée de plusieurs façons :

- ils peuvent permettre d'établir des corrélations entre l'activité d'une enzyme et une pathologie détectée ;
- ils peuvent être utilisés pour effectuer un diagnostic d'une pathologie, en effectuant un dosage d'une enzyme présent dans un échantillon biologique, si la corrélation entre l'activité de l'enzyme et la pathologie à détecter est connue.

Ils pourront être utilisés notamment pour effectuer l'analyse *in vitro* de fluides comprenant l'enzyme dont on veut estimer l'activité.

Ils pourront être utilisés également *in vivo*, notamment lorsque le couple $X_1/X_2$ émet dans le rouge.

Ainsi, l'invention a trait à un réactif prêt à l'emploi comprenant au moins un peptide cyclique conforme à l'invention ainsi qu'à un kit comprenant :

- un premier compartiment comprenant au moins un peptide cyclique conforme à l'invention ; et
- un second compartiment comprenant une solution tampon.

Ce kit est destiné à la préparation de solutions mères, lesquelles seront mises en contact avec l'enzyme protéase, dont on veut mesurer l'activité. L'enzyme protéase peut être contenue dans un fluide biologique.

Comme son nom l'indique, la solution tampon est destinée à fixer le pH d'une solution, de façon à ce que ce pH ne soit ni acide, ni basique, ce qui influerait sur les mesures d'activité enzymatique ultérieures.

Enfin, l'invention a trait à l'utilisation d'au moins un peptide cyclique conforme à l'invention pour déterminer *in vitro* l'activité d'au moins une enzyme protéase.

D'autres caractéristiques et avantages de l'invention apparaîtront à la lecture des exemples qui suivent, donnés à titre illustratif et non limitatif.

## BRÈVE DESCRIPTION DES DESSINS

La figure 1 est un graphique représentant la variation d'intensité de fluorescence $\Delta_{IF}$ donneur (en a.u) du peptide cyclique préparé conformément à l'exemple, en fonction du temps t (en min), ce peptide cyclique étant mis en présence, à raison d'une concentration de 90 $\mu$M avec une solution de MMP-1 (1nM).
La figure 2 est un graphique en double inverse présentant l'inverse des vitesses initiales ($1/v_i$) (en $a.u^{-1}.s$) d'hydrolyse du peptide cyclique préparé en fonction de l'inverse de sa concentration (1/[S]) (en $\mu M^{-1}$).

## EXPOSÉ DÉTAILLÉ DE MODES DE RÉALISATION PARTICULIERS

L'exemple suivant illustre la préparation d'un peptide cyclique conforme à l'invention comprenant deux séquences peptidiques $S_1$ et $S_2$ correspondant chacune à une séquence cible de la MMP1, cette séquence étant la suivante : -Gly-Pro-Gln-Gly-Leu-Leu-Gly-Ala-.
La préparation de ce peptide cyclique comprend les étapes suivantes :

a)la synthèse d'un acide aminé de départ destiné à être greffé sur une résine par sa chaîne latérale : le Fmoc-Lys-OAllyl ;
b) le greffage dudit acide aminé de départ sur une résine ;
c) la synthèse des deux séquences peptidiques sur la résine greffée ;
d) la cyclisation.

Les détails de ces étapes sont exposés ci-dessous.
Dans les protocoles ci-dessous, les abréviations suivantes ont été utilisées :

ACN : Acétonitrile ; Boc : t-butyloxycarbonyl ; DAC : acide 7-diéthylaminocoumarin-3-carboxylique ; DCM : dichlorométhane ; DIEA : N,N'-diisopropyldiéthylamine ; DMF : N,N-diméthylformamide ; ESMS : electrospray mass spectrometry ; $Et_2O$ : diisopropyléther ; Fmoc : 9-fluorénylméthoxycarbonyle ; HBTU : N-[1H-benzotriazol-1-yl)diméthylamino)méthylène]-N-méthylméthanaminium hexafluorophosphate N-oxyde ; HOBt : N-hydroxybenzotriazole ; MC : acide 7-méthoxycoumarin-3-carboxylique ; AMPA : 4-aminophénylmercurique acétate ; PyBOP : benzotriazol-1-yl-oxy-tris-pyrrolidonophosphonium ; TA : température ambiante ; TFA : acide trifluoroacétique ; THF : tétrahydrofurane ; TIS : triisopropylsilane ; TRIS : tris(hydroxyméthyl)aminométhane.

Tous les produits chimiques et les solvants sont de qualité analytique et ont été achetés chez Sigma. La résine 2-chloro chlorotrityle, le PyBOP et tous les acides aminés (protégés en N$\alpha$ par Fmoc) ont été achetés chez Advanced Chemtech.
Toutes les réactions chimiques ont été réalisées sous $N_2$ avec des solvants anhydres. Le DMF est desséché sur $CaH_2$ au reflux pendant une nuit et distillé. Le DCM a été distillé avant utilisation.
Les spectres UV/visible ont été enregistrés sur un spectrophotomètre Hitachi modèle U-2010. Les spectres de fluorescence ont été réalisés sur un spectrofluorimètre PTI et un Jobin-Yvon JY3 avec une cuve thermostatée à 37°C. La synthèse peptidique en phase solide a été effectuée sur un synthétiseur Applied Biosystem 433A. Les purifications ont été réalisées sur une HPLC Shimadzu en phase inverse (contrôleur SLC-10 AVP, pompes LC8A, détecteur UV/Visible SPD-10 AVP (longueurs d'onde 214, 267 et 254 nm) et colonne C18 Satisfaction RP18AB 5 $\mu$m 250*4,6 mm). Le système de solvants utilisé pour l'élution est (A) une solution aqueuse de TFA à 0,1% et (B) une solution aqueuse d'ACN à 70%.

a)Synthèse de la Fmoc-Lys-OAllyl

La Fmoc-Lys-OAllyl répond à la formule suivante :

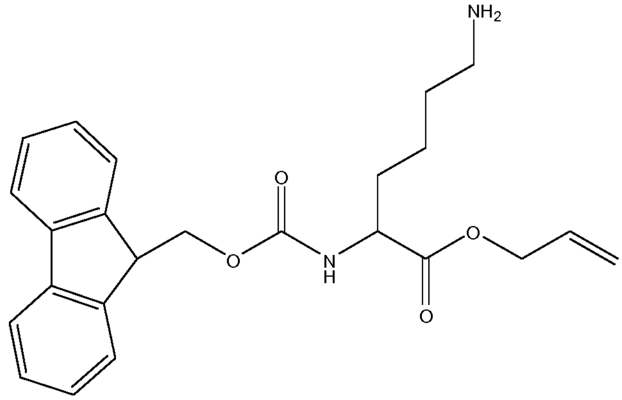

Elle est préparée selon le schéma réactionnel suivant :

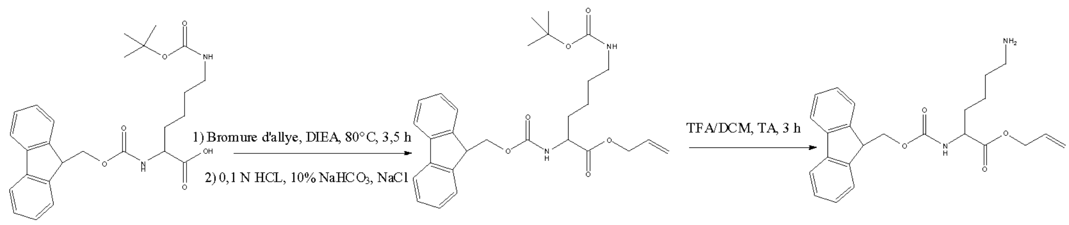


Fmoc-Lys(Boc)-OH Fmoc-Lys(Boc)-OAllyl Fmoc-Lys-OAllyl

A une solution de Fmoc-Lys(Boc)-OH (2g, 4,3 mmol) et de bromure d'allyle (10 mL, 118,2 mmol) est ajouté 1,45 mL de DIEA (8,5 mmol). Après agitation à 80°C pendant 3,5 heures, la solution est diluée avec 200 mL d'acétate d'éthyle puis lavée avec 0,1 N de HCl (3*100 mL), 10% de $NaHCO_3$ (3*100 mL) et une solution saturée en NaCl (3*100 mL) puis séchée sur $MgSO_4$. Après évaporation sous pression réduite, 1,9 g de Fmoc-Lys(Boc)-OAllyl (3,87 mmol, 90%) sont obtenus sous forme d'une poudre blanche.

La Fmoc-Lys(Boc)-OAllyl(1,9 g, 3,87 mmol) est dissoute dans un mélange TFA/DCM (70 mL, 1/1). La solution est agitée à température ambiante pendant 3 heures. Après concentration de la solution, des lavages avec de l'éther et des évaporations successives, 1,45 g de Fmoc-Lys-OAllyl (3,56 mmol, 92%) sont récupérés sous forme d'une poudre blanche.

b) Greffage de la Fmoc-Lys(Boc)-OAllyl sur une résine et d'une sonde Lys(MC)

Le schéma réactionnel est le suivant :

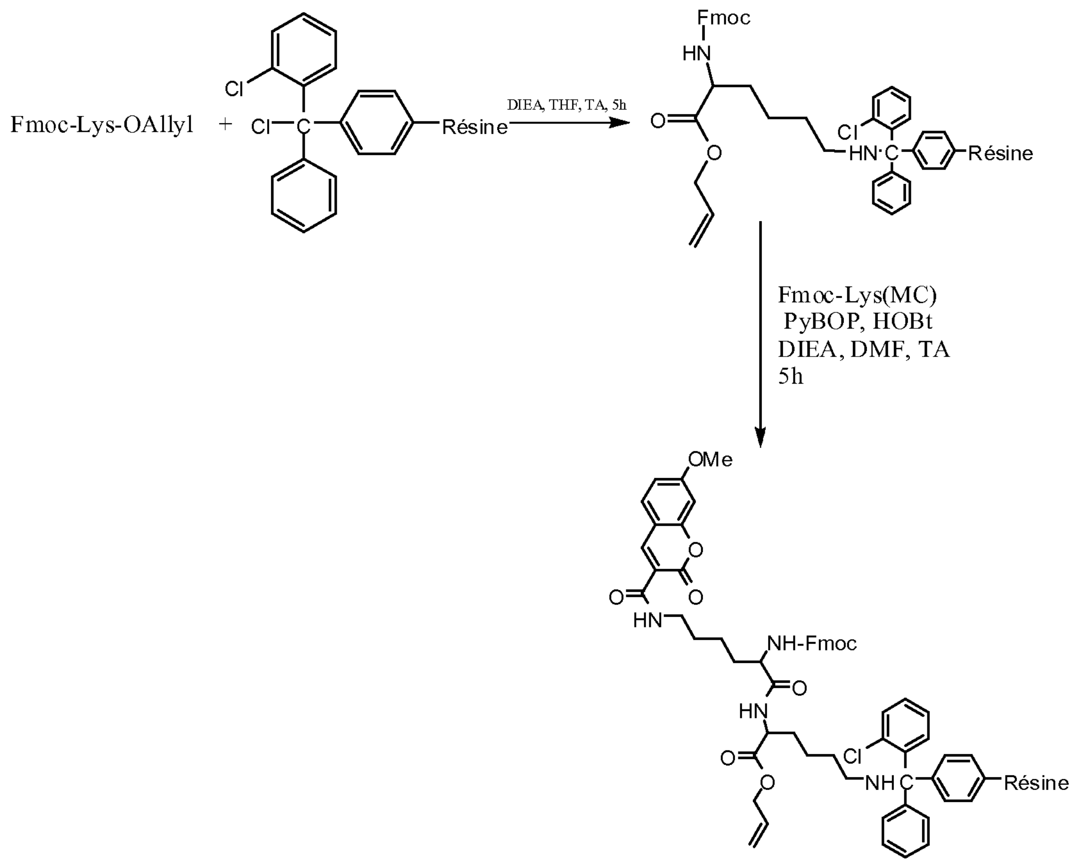


La Fmoc-Lys(Boc)-OAllyl (1,81 g, 4,43 mmol) est dissoute dans du THF anhydre (2 mL) et de la DIEA (734 µL, 4,23 mmol) est ajouté. Après 10 minutes d'agitation à température ambiante, 570 mg de résine 2-chlorotrityle est introduite. Le mélange est agité pendant 5 heures à température ambiante. La résine est, par la suite, filtrée et lavée avec du DMF (20 mL), un mélange DCM/méthanol/DIEA (17/2/1) (2*20 mL), du DMF (10 mL) et du DCM (20 mL). Le taux de substitution de cette nouvelle résine a été déterminé à 0,4 mmol/g par mesure UV du complexe dibenzofulvène après traitement à la pipéridine. Dans un premier temps, la résine Fmoc-Lys-OAllyl-chlorotrityle (300 mg, 0,4 mmol/g) a été déprotégée, par libération du groupe Fmoc, en mettant en contact ladite résine avec un mélange de 20% de pipéridine dans du DMF à température ambiante pendant 3 heures. La présence d'amine primaire libre a été vérifiée par le test de Kaiser. Cette résine est mis à réagir avec de la Fmoc-Lys(MC)-OH (228 mg, 0,4 mmol) en présence de PyBOP (173 mg, 0,392 mmol), de HOBt (54 mg, 0,4 mmol) et de DIEA (100 µL, 0,8 mmol) dans le DMF (5 mL) pendant 6 heures à température ambiante. La résine est ensuite filtrée et lavée avec du DMF, du DCM et du méthanol. 270 mg de résine Fmoc-Lys(MC)-Lys-OAllyl-chlorotrityle sont récupérés. Le taux de substitution de cette nouvelle résine a été déterminé à 0,38 mmol/g par mesure UV du complexe dibenzofulvène après traitement à la pipéridine. Les fonctions amines, qui n'ont pas réagies, ont été acétylées en présence d'ester succimidique d'acétylate (25,12 mg, 0,16 mmol), de pipéridine (12 mg, 0,16 mmol) dans du DMF (5 mL) pendant 40 minutes à température ambiante.

c) Synthèse peptidique

Le schéma réactionnel est le suivant :

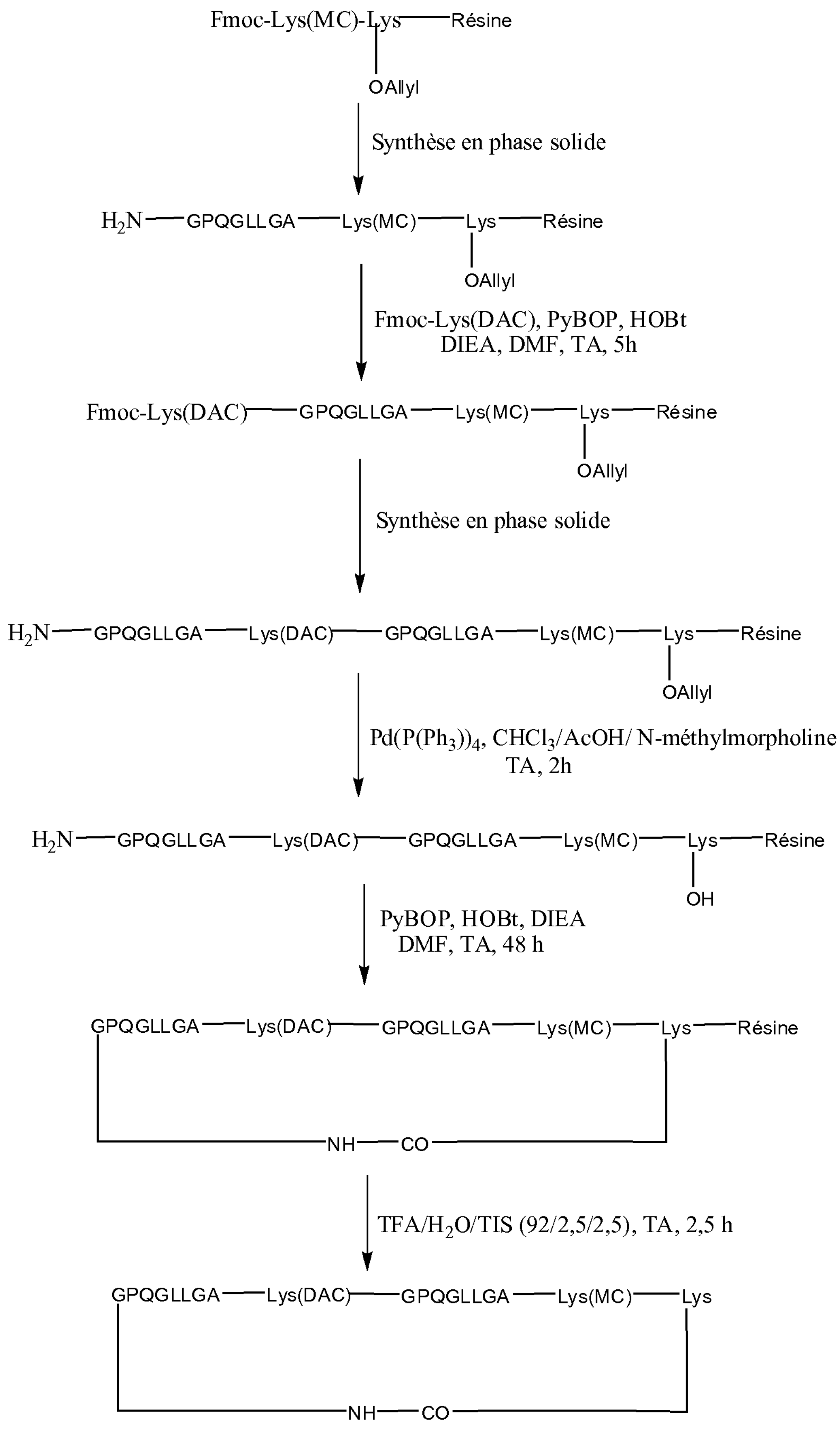


les codes G, P, Q, L et A correspondant respectivement aux acides aminés glycine, proline, glutamine, leucine, alanine.

La résine Fmoc-Lys(MC)-Lys-OAllyl-chlorotrityle est introduite dans le réacteur du synthétiseur. Les acides

aminés (Gly, Pro, Gln(Trt), Leu et Ala) protégés en N$\alpha$ par un groupement Fmoc sont introduits en excès (10 fois). Les étapes de couplage sont réalisées en présence de HBTU, HOBt et DIEA, à l'issue desquelles l'on obtient le peptide suivant GPQGLLGA-, A étant lié à Lys (MC) . Après 18 heures, 370 mg de peptide lié à la résine sont récupérés.

La Fmoc-Lys(DAC)-OH (190 mg, 0,3 mmol) est couplée, en dehors du réacteur du synthétiseur, au peptide sur résine en présence de PyBOP (153 mg, 0,3 mmol), de HOBt (40,53 mg, 0,3 mmol) et de DIEA (99,1 $\mu$L, 0,6 mmol) dans du DMF (5 mL) pendant 5 heures à température ambiante. Ensuite, la résine est filtrée et lavée comme précédemment. 401 mg sont récupérés. Le taux de substitution est évalué à 0,25 mmol/g. Les fonctions amines n'ayant pas réagi sont acétylées par la même procédure que celle décrite ci-dessus.

Le peptide sur résine est, de nouveau, introduit dans le réacteur du synthétiseur automatique de peptide et la même séquence d'acides aminés que précédemment est synthétisée à la suite de la première. 530 mg sont récupérés.

d) Cyclisation

La résine est séchée sous un vide poussé pendant 4 heures puis est mise sous argon. Du $Pd(P(Ph_3))_4$ (346,6, mg, 0,3 mmol) est dissous dans un mélange chloroforme/acide acétique/N-méthylmorpholine (37/2/1, 8 mL) sous flux d'argon. Cette solution est alors mise en contact avec la résine et le mélange résultant est agité occasionnellement à température ambiante pendant 2 heures. La résine est alors filtrée, lavée avec une solution de 0,5% de DIEA dans le DMF (2*10 mL) puis avec une solution de diéthyldithiocarbamate de sodium (0,5% m/m) dans du DMF, afin d'éliminer le catalyseur.

Après le clivage du Fmoc, la résine est lavée avec du HOBt (1M) dans le DMF. Afin de réaliser la cyclisation entre les extrémités N et C terminales, 260 mg de PyBOP (0,5 mmol), 69 mg de HOBt (0,51 mmol) et 180 $\mu$L de DIEA (1 mmol) sont introduits dans 10 mL de N-méthylpyrrolidone. La solution est agitée pendant 48 heures à température ambiante puis la résine est lavée avec 30 mL de DMF, de DCM et de méthanol.

La résine est alors mise en suspension dans une solution de TFA/$H_2O$/TIS (95/2,5/2,5). Après 3 heures à température ambiante, la résine est filtrée, lavée avec du TFA (2*1 mL) et du DCM (10 mL). Le filtrat est évaporé partiellement sous pression réduite puis noyé dans de l'éther froid.

Après 12 heures à -20°C, le précipité est filtré et lavé pour obtenir 170 mg de produit brut. Après purification par HPLC semi-préparative, 92,1 mg du peptide sont obtenus. La structure du peptide a été vérifiée par ESMS.

Le peptide cyclique obtenu dans cet exemple répond à la formule suivante :

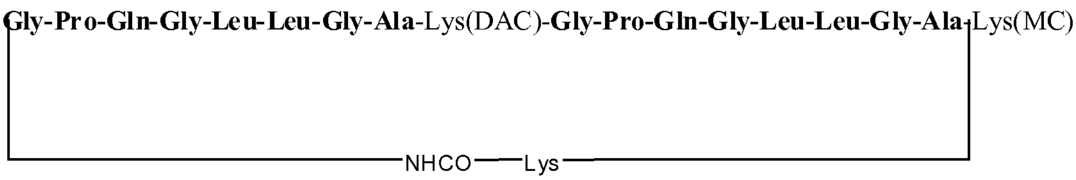

Des essais enzymatiques ont été réalisés dans une solution tampon contenant 0,1 M de TRIS, 0,1 M de NaCl et 10 mM et 10 mM de $CaCl_2$ à un pH de 7,6. 25 $\mu$L de MMP-9 commerciale (90% de forme zymogène) sont activés avec une solution de AMPA (1mM) dans la solution tampon à 37°C pendant 4 heures. La MMP-1 commerciale est déjà sous forme activée. Une solution mère du peptide cyclique préparé conformément au protocole exposé ci-dessus est préparée avec une solution tampon telle que définie ci-dessus à une concentration finale de 90 $\mu$M. La solution de concentration finale de 90 $\mu$M a servi de solution de départ pour réaliser différentes dilutions afin d'obtenir 4 solutions de peptide cyclique/tampon ayant chacune une concentration de 10, 5, 1 et 0,5 $\mu$M.

Tous les essais sont réalisés dans un volume réactionnel final de 2 mL avec 1 nM de MMP-1 active ou 2 nM de MMP-9 activée par de l'AMPA.

Après ajout de l'enzyme, le taux d'hydrolyse initial est déterminé par la variation dans le temps de l'intensité de fluorescence à 403 nm (à une longueur d'excitation de 340 nm). Pour chaque concentration de peptide cyclique testée, une courbe a été tracée représentant la variation d'intensité de fluorescence du groupe donneur $\Delta I_F$ (en a.u) en fonction du temps t (en min), comme cela est représenté sur la figure 1 (pour une concentration de peptide cyclique de 10, 5, 1 et 0,5 $\mu$M.

A partir de l'équation suivante :

$$\mathrm{Fluorescence(t)= fluorescence_{max}(1-exp(-k_{obs}t))}$$

la constante de premier ordre $k_{obs}$ a pu être déterminée. Le calcul de la constante de second ordre (dite constante de

spécificité) $k_{cat}/K_M$ est obtenu grâce à la concentration d'enzyme active utilisée $E_0$ suivant la relation $k_{cat}/K_m=k_{obs}/[E_0]$. Le $k_{cat}/K_m$ du peptide est évalué à 319120 $s^{-1}.M^{-1}$, valeur très élevée par rapport aux meilleurs substrats de la MMP-1 décrits dans la littérature, ce qui atteste de la très grande spécificité du peptide cyclique préparé selon l'invention pour la MMP-1.

En utilisant les vitesses initiales des différents tests, le graphique en double inverse (1/Vi)=f(1/[peptide cyclique]) représenté à la figure 2 a permis de déterminer le $K_m$ du peptide cyclique, qui s'élève à 3,8 μM. Cette valeur est plus faible que celle obtenue pour les meilleurs substrats de la MMP-1 décrits dans la littérature, ce qui atteste d'une excellente affinité du peptide cyclique préparé selon l'invention pour la MMP-1.

Le peptide cyclique de l'invention a également été testé en présence de 2 nM de MMP-9 active. Aucune variation de l'intensité de fluorescence du groupe donneur n'a pu être mesurée au cours du temps. Ce peptide cyclique n'est donc pas substrat de la MMP-9, ce qui montre la sélectivité de ce peptide cyclique. Ce peptide cyclique présente également une très grande sélectivité et une grande affinité pour la MMP-1.

**Revendications**

1. Peptide cyclique répondant à la formule suivante :

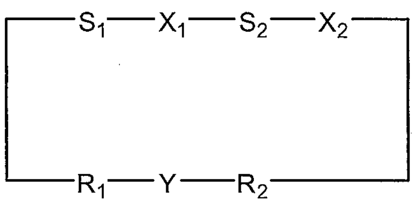

dans laquelle :

- $S_1$ est une première séquence peptidique cible d'une enzyme protéase $E_1$ du type MMP ;
- $S_2$ est une seconde séquence peptidique cible d'une enzyme protéase $E_2$ du type MMP, $S_1$ et $S_2$ pouvant être identiques ou différents, ladite première séquence peptidique $S_1$ et ladite seconde séquence peptidique $S_2$ comprenant de 4 à 14 acides aminés, $E_1$ et $E_2$ pouvant correspondre à la même enzyme protéase ou à deux enzymes protéases distinctes ;
- $X_1$ est une sonde porteuse d'un groupe donneur fluorescent ;
- $X_2$ est une sonde porteuse d'un groupe accepteur fluorescent ou non fluorescent ;
- $R_1$ et $R_2$ correspondent indépendamment à une liaison simple, un reste d'acide aminé ou une séquence peptidique ;
- Y correspond à un groupe -CONH- ou -NHCO- ;

l'enzyme protéase MMP étant choisie parmi la MMP-1, MMP-2, MMP-3, MMP-5, MMP-7, MMP-8, MMP-9, MMP-10, MMP-11, MMP-12, MMP-13, MMP-14, MMP-15, MMP-16, MMP-17, MMP-19, MMP-23, MMP-25 et MMP-26 et les mélanges de celles-ci.

**2.** Peptide cyclique selon la revendication 1, dans lequel :

- lorsqu'elles sont cibles de l'enzyme MMP-1, la première séquence $S_1$ et/ou la seconde séquence $S_2$, répondent à l'une au moins des séquences suivantes :

-Gly-Pro-Gln-Gly-Leu-Leu-Gly-Ala-SEQ. 1
-Ala-Pro-Gln-Gly-Ile-Ala-Gly-Gln-SEQ. 2
-Gly-Pro-Gln-Gly-Leu-Ala-Gly-Gln-SEQ.3
-Gly-Pro-Leu-Gly-Ile-Ala-Gly-Ile-SEQ.4
-Gly-Pro-Glu-Gly-Leu-Arg-Val-Gly-SEQ.5
-Tyr-Glu-Ala-Gly-Leu-Gly-Val-Val-SEQ.6
-Ala-Gly-Leu-Gly-Val-Val-Glu-Arg-SEQ.7
-Ala-Gly-Leu-Gly-Ile-Ser-Ser-Thr-SEQ.8
-Gly-Ala-Met-Phe-Leu-Glu-Ala-Ile-SEQ.9
-Ile-Pro-Glu-Asn-Phe-Phe-Gly-Val-SEQ.10
-Thr-Glu-Gly-Glu-Ala-Arg-Gly-Ser-SEQ.11
*-Arg-Ala-Ile-His-Ile-Gln-Ala-Glu-SEQ.12*
*-Leu-Arg-Ala-Tyr-Leu-Leu-Pro-Ala-SEQ.13*
*-Gly-Pro-Leu-Gly-Met-Arg-Gly-Leu-SEQ.14*
*-Pro-Gln-Gly-Leu-Glu-Ala-Lys-SEQ.15*
*-Pro-Leu-Gly-Leu-Trp-Ala-D-Arg-SEQ. 16*
*-Pro-Leu-Ala-Leu-Trp-Ala-Arg-SEQ. 17*
-Pro-Cha-Abu-Cys(Me)-His-Ala-SEQ.18
-Pro-Cha-Gly-Cys(Me)-His-Ala-SEQ. 19
*-Pro-Cha-Gly-Cys(Me)-His-Ala-SEQ. 20*
*-Pro-Leu-Gly-Leu-Dpa-Ala-Arg-SEQ.21*
*-Pro-Leu-Gly-[2-mercapto-4-methyl-pentanoyl]-Leu-Gl SEQ. 22*
*-Pro-Leu-Ala-Leu-Trp-Ala-Arg-SEQ. 17*
*-Pro-Leu-Gly-Cys(Me)-His-Ala-D-Arg-SEQ. 23*
*-Arg-Pro-Leu-Ala-Leu-Trp-Arg-SEQ. 24*
*-Pro-Cha-Gly-Nva-His-Ala-SEQ. 25*
*-Arg-Pro-Lys-Pro-Tyr-Ala-Nva-Trp-Met-SEQ. 26*

ou
- lorsqu'elles sont cibles de l'enzyme MMP-2, la première séquence $S_1$ et/ou la seconde séquence $S_2$, répondent à l'une au moins des séquences suivantes :

*-Ile-Pro-Glu-Asn-Phe-Phe-Gly-Val-SEQ.10*
-Pro-Pro-Gly-Ala-Tyr-His-Gly-Ala-SEQ.27
*-Arg-Ala-Ile-His-Ile-Gln-Ala-Glu-SEQ.12*
*-Gly-Pro-His-Leu-Leu-Val-Glu-Ala-SEQ.28*

*-Leu-Arg-Ala-Tyr-Leu-Leu-Pro-Ala-SEQ.13*
-Pro-Gln-Gly-Leu-Glu-Ala-Lys-SEQ.29
*-Pro-Leu-Gly-Leu-Trp-Ala-D-Arg-SEQ. 16*
-Pro-Leu-Ala-Nva-Dpa-Ala-Arg-SEQ.30
*-Pro-Leu-Gly-Leu-Dpa-Ala-Arg-SEQ.21*
*-Pro-Leu-Gly-Met-Trp-Ser-Arg-SEQ. 31*
*-Pro-Leu-Gly-SCH[$CH_2CH(CH_3)_2$]-CO-Leu-Gly-SEQ. 32*
*-Arg-Pro-Pro-Gly-Phe-Ser-Ala-Phe-SEQ. 33*
*-Pro-Leu-Gly-Leu-Dpa-Ala-Arg-SEQ.21*
*-Pro-Leu-Gly-[2-mercapto-4-methyl-pentanoyl]-Leu-Gly-SEQ.22*
*-Pro-Leu-Gly-Cys(Me)-His-Ala-D-Arg-SEQ.23*
*-Arg-Pro-Leu-Ala-Leu-Trp-Arg-SEQ.24*
*-Pro-Cha-Gly-Nva-His-Ala-SEQ. 25*
*-Arg-Pro-Lys-Pro-Tyr-Ala-Nva-Trp-Met-SEQ. 26*

ou

- lorsqu'elles sont cibles de l'enzyme MMP-3, la première séquence $S_1$ et/ou la seconde séquence $S_2$, répondent à l'une au moins des séquences suivantes :

*-Gly-Pro-Glu-Gly-Leu-Arg-Val-Gly-SEQ.5*
-Arg-Val-Gly-Phe-Tyr-Glu-Ser-Asp-SEQ.34
-Leu-Leu-Ser-Ala-Leu-Val-Glu-Thr-SEQ.35
-Glu-Ala-Ile-Pro-Met-Ser-Ile-Pro-SEQ.36
-Ile-Ala-Gly-Arg-Ser-Leu-Asn-Pro-SEQ.37
*-Ile-Pro-Glu-Asn-Phe-Phe-Gly-Val-SEQ.10*
-Lys-Pro-Gln-Gln-Phe-Phe-Gly-Leu-SEQ.38
-Asp-Val-Ala-Gln-Phe-Val-Leu-Thr-SEQ.39
-Asp-Thr-Leu-Glu-Val-Met-Arg-Lys-SEQ.40
-Asp-Val-Gly-His-Phe-Arg-Thr-Phe-SEQ.41
-Asp-Ser-Gly-Gly-Phe-Met-Leu-Thr-SEQ.42
-Arg-Val-Ala-Glu-Met-Arg-Gly-Glu-SEQ.43
-Asp-Leu-Gly-Arg-Phe-Gln-Thr-Phe-SEQ.44
-Pro-Phe-Ser-Pro-Leu-Val-Ala-Thr-SEQ.45
*-Leu-Arg-Ala-Tyr-Leu-Leu-Pro-Ala-SEQ.13*
-Ala-Pro-Gly-Asn-Ala-Ser-Glu-Ser-SEQ.46
-Phe-Ser-Ser-Glu-Ser-Lys-Arg-Glu-SEQ.47
*-Arg-Ala-Ile-His-Ile-Gln-Ala-Glu-SEQ.12*
*-Gly-Pro-His-Leu-Leu-Val-Glu-Ala-SEQ.28*
-Pro-Pro-Glu-Glu-Leu-Lys-Phe-Gln-SEQ.48
*-Gly-Pro-Leu-Gly-Met-Arg-Gly-Leu-SEQ. 49*
*-Pro-Gln-Gly-Leu-Glu-Ala-Lys-SEQ. 15*
-Arg-Pro-Lys-Pro-Val-Glu-Nva-Trp-Arg-Glu-Ala-Lys-SEQ. 50
*-Pro-Leu-Gly-Leu-Trp-Ala-D-Arg-SEQ. 16*
*-Pro-Tyr-Ala-Tyr-Trp-Met-Arg-SEQ. 51*
*-Arg-Pro-Lys-Pro-Leu-Ala-Nva-Trp-SEQ. 52*
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Arg-Pro-Lys-Pro-Tyr-Ala-Nva-Trp-Met-SEQ. 26*
*-Arg-Pro-Lys-Pro-Val-Glu-Nva-Trp-Arg-SEQ. 50*

ou

- lorsqu'elles sont cibles de l'enzyme MMP-5, la première séquence $S_1$ et/ou la seconde séquence $S_2$, répondent à l'une au moins des séquences suivantes :

*-Arg-Pro-Lys-Pro-Val-Glu-Nva-Trp-Arg-Glu-Ala-Lys-SEQ. 50*

ou

-lorsqu'elles sont cibles de l'enzyme MMP-7, la première séquence $S_1$ et/ou la seconde séquence $S_2$, répondent à l'une au moins des séquences suivantes :

*-Pro-Leu-Gly-Leu-Trp-Ala-D-Arg-SEQ. 16*
*-Pro-Leu-Gly-Leu-Dpa-Ala-Arg-SEQ. 21*
*-Arg-Pro-Leu-Ala-Leu-Trp-Arg-Ser-SEQ. 24*
*-Pro-Cha-Gly-Cys(Me)-His-Ala-SEQ. 20*
*-Pro-Leu-Gly-[2-mercapto-4-methyl-pentanoyl]-Leu-Gly-SEQ. 22*
*-Pro-Leu-Ala-Leu-Trp-Ala-Arg-SEQ. 17*
*-Pro-Tyr-Ala-Tyr-Trp-Met-Arg-SEQ. 51*
*-Arg-Pro-Lys-Pro-Leu-Ala-Nva-Trp-SEQ. 52*

ou
-lorsqu'elles sont cibles de l'enzyme MMP-8, la première séquence $S_1$ et/ou la seconde séquence $S_2$, répondent à l'une au moins des séquences suivantes :

*-Pro-Leu-Ala-Tyr-Trp-Ala-Arg-SEQ. 53*
*-Pro-Leu-Gly-Leu-Trp-Ala-D-Arg-SEQ. 16*
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Pro-Leu-Ala-Leu-Trp-Ala-Arg-SEQ. 17*
*-Pro-Leu-Gly-Cys(Me)-His-Ala-D-Arg-SEQ. 23*
*-Arg-Pro-Leu-Ala-Leu-Trp-Arg-SEQ. 24*
*-Pro-Cha-Gly-Nva-His-Ala-SEQ . 25*

ou
- lorsqu'elles sont cibles de l'enzyme MMP-9, la première séquence $S_1$ et/ou la seconde séquence $S_2$, répondent à l'une au moins des séquences suivantes :

-Gly-Pro-Pro-Gly-Val-Val-Gly-Pro-SEQ.54
-Gly-Pro-Pro-Gly-Leu-Arg-Gly-Glu-SEQ.55
-Gly-Pro-Gly-Gly-Val-Val-Gly-Pro-SEQ.56
-Ile-Pro-Gln-Asn-Phe-Phe-Gly-Val-SEQ.57
-Pro-Pro-Gly-Ala-Tyr-His-Gly-Ala-SEQ.58
*-Arg-Ala-Ile-His-Ile-Gln-Ala-Glu-SEQ.12*
*-Gly-Pro-Leu-Gly-Met-Arg-Gly-Leu-SEQ. 14*
*-Pro-Gln-Gly-Leu-Glu-Ala-Lys-SEQ. 15*
*-Arg-Pro-Lys-Pro-Val-Glu-Nva-Trp-Arg-Glu-Ala-Lys-SEQ.50*
*-Pro-Leu-Gly-Leu-Trp-Ala-D-Arg-SEQ. 16*
*-Pro-Cha-Gly-Cys(Me)-His-Ala-SEQ. 19*
*-Pro-Leu-Gly-SCH[$CH_2CH(CH_3)_2$]-CO-Leu-Gly-SEQ. 32*
*-Arg-Pro-Pro-Gly-Phe-Ser-Ala-Phe-SEQ. 33*
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Pro-Leu-Gly-Cys(Me)-His-Ala-D-Arg-SEQ. 23*

ou
- lorsqu'elles sont cibles de l'enzyme MMP-10, la première séquence $S_1$ et/ou la seconde séquence $S_2$, répondent à l'une au moins des séquences suivantes :

*-Arg-Ala-Ile-His-Ile-Gln-Ala-Glu-SEQ.12*
*-Gly-Pro-His-Leu-Leu-Val-Glu-Ala-SEQ. 28*
*-Pro-Leu-Gly-Leu-Dpa-Ala-Arg-SEQ. 21*

ou
- lorsqu'elles sont cibles de l'enzyme MMP-11, la première séquence $S_1$ et/ou la seconde séquence $S_2$, répon-

dent à l'une au moins des séquences suivantes :

*-Pro-Cha-Gly-Cys(Me)-His-Ala-SEQ. 20*
*-Pro-Leu-Gly-Leu-Dpa-Ala-Arg-SEQ. 21*

ou
- lorsqu'elles sont cibles de l'enzyme MMP-12, la première séquence $S_1$ et/ou la seconde séquence $S_2$, répondent à l'une au moins des séquences suivantes :

*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Pro-Leu-Ala-Leu-Trp-Ala-Arg- SEQ. 17*
*-Pro-Leu-Gly-Cys(Me)-His-Ala-D-Arg-SEQ. 23*
*-Pro-Tyr-Ala-Tyr-Trp-Met-Arg-SEQ. 51*
*-Arg-Pro-Lys-Pro-Leu-Ala-Nva-Trp-SEQ. 52*
*-Arg-Pro-Leu-Ala-Leu-Trp-Arg-SEQ. 24*
*-Pro-Cha-Gly-Nva-His-Ala-SEQ. 25*
*-Arg-Pro-Lys-Pro-Tyr-Ala-Nva-Trp-Met-SEQ . 26*
*-Arg-Pro-Lys-Pro-Val-Glu-Nva-Trp-Arg-SEQ. 50*
*-Arg-Pro-Lys-Pro-Gln-Gln-Phe-Trp-SEQ. 59*

ou
- lorsqu'elles sont cibles de l'enzyme MMP-13, la première séquence $S_1$ et/ou la seconde séquence $S_2$, répondent à l'une au moins des séquences suivantes :

*-Gly-Pro-Leu-Gly-Met-Arg-Gly-Leu-SEQ. 49*
*-Arg-Pro-Lys-Pro-Val-Glu-Nva-Trp-Arg-Glu-Ala-Lys-SEQ. 50*
*-Pro-Cha-Gly-Nva-His-Ala-Dpa-SEQ. 25*
*-Pro-Cha-Gly-Cys(Me)-His-Ala-SEQ . 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Pro-Leu-Gly-Leu-Trp-Ala-D-Arg-SEQ. 16*
*-Pro-Leu-Ala-Leu-Trp-Ala-Arg-SEQ. 17*
*-Pro-Leu-Gly-Cys(Me)-His-Ala-D-Arg-SEQ. 23*
*-Pro-Leu-Gly-Met-Trp-Ser-Arg-SEQ. 31*
*-Pro-Tyr-Ala-Tyr-Trp-Met-Arg-SEQ. 51*
*-Arg-Pro-Lys-Pro-Leu-Ala-Nva-Trp-SEQ. 52*
*-Arg-Pro-Leu-Ala-Leu-Trp-Arg-SEQ. 24*
*-Pro-Leu-Ala-Tyr-Trp-Ala-Arg-SEQ. 53*
*-Pro-Cha-Gly-Nva-His-Ala-SEQ. 25*
*-Arg-Pro-Lys-Pro-Tyr-Ala-Nva-Trp-Met-SEQ. 26*
*-Arg-Pro-Lys-Pro-Gln-Gln-Phe-Trp-SEQ. 59*

ou
- lorsqu'elles sont cibles de l'enzyme MMP-14, la première séquence $S_1$ et/ou la seconde séquence $S_2$, répondent à l'une au moins des séquences suivantes :

-Pro-Leu-Ala-Cys(p-OMeBz)-Trp-Ala-Arg-SEQ. 60
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*

ou
- lorsqu' elles sont cibles de l'enzyme MMP-15, la première séquence $S_1$ et/ou la seconde séquence $S_2$, répondent à l'une au moins des séquences suivantes :

*-Pro-Leu-Gly-Leu-Dpa-Ala-Arg-SEQ. 21*

ou

- lorsqu'elles sont cibles de l'enzyme MMP-16, la première séquence $S_1$ et/ou la seconde séquence $S_2$, répondent à l'une au moins des séquences suivantes :

*-Pro-Leu-Gly-Leu-Dpa-Ala-Arg-SEQ. 21*

ou

- lorsqu'elles sont cibles de l'enzyme MMP-17, la première séquence $S_1$ et/ou la seconde séquence $S_2$, répondent à l'une au moins des séquences suivantes :

*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*

ou

- lorsqu'elles sont cibles de l'enzyme MMP-19, la première séquence $S_1$ et/ou la seconde séquence $S_2$, répondent à l'une au moins des séquences suivantes :

*-Pro-Leu-Gly-Leu-Dpa-Ala-Arg-SEQ. 21*

ou

- lorsqu'elles sont cibles de l'enzyme MMP-23, la première séquence $S_1$ et/ou la seconde séquence $S_2$, répondent à l'une au moins des séquences suivantes :

*-Pro-Leu-Gly-Leu-Dpa-Ala-Arg-SEQ. 21*

ou

- lorsqu'elles sont cibles de l'enzyme MMP-25, la première séquence $S_1$ et/ou la seconde séquence $S_2$, répondent à l'une au moins des séquences suivantes :

*-Pro-Leu-Gly-Leu-Dpa-Ala-Arg-SEQ. 21*

ou

- lorsqu'elles sont cibles de l'enzyme MMP-26, la première séquence $S_1$ et/ou la seconde séquence $S_2$, répondent à l'une au moins des séquences suivantes :

*-Pro-Leu-Gly-Leu-Trp-Ala-D-Arg-SEQ. 16*

**3.** Peptide cyclique selon l'une quelconque des revendications précédentes, dans lequel le groupe donneur porté par la sonde $X_1$ et le groupe accepteur porté par la sonde $X_2$ comprennent un noyau aromatique.

**4.** Peptide cyclique selon la revendication 3, dans lequel le noyau aromatique est un noyau benzénique, anthracénique ou coumarine.

**5.** Peptide cyclique selon l'une quelconque des revendications précédentes, 1 dans lequel le groupe donneur porté par la sonde $X_1$ et le groupe accepteur porté par la sonde $X_2$ sont choisis parmi les couples suivants :

*Tryptophane/2,4-dinitrophényle ;
*Acide o-aminobenzoïque/2,4-dinitrophényle ;
*(7-méthoxycoumarin-4-yl)-acétyle/2,4-dinitrophényle ;
*(7-méthoxycoumarin-4-yl)-acétyle/N-3-(2,4-dinitrophényl)-L-2,3-diaminopropyle ;
*Tryptophane/Dansyle ;
*N-méthylanthranoyle/2,4-dinitrophényle ;
*6,7-diméthoxycoumarin-4-yl-acétyle/acide 6-N-(7-nitrobenz-2-oxa-1,3-diazol-4-yl)aminohexanoïque ;
*Acide 5-(2'-aminométhyl)naphtalène sulfonique/acide 4-(4'-diméthylaminophénylaza)benzoïque
* acide 7-méthoxycoumarin-3-carboxylique/acide 7-diéthylaminocoumarin-3-carboxylique.

**6.** Peptide cyclique selon l'une quelconque des revendications précédentes, dans lequel le groupe donneur porté par la sonde $X_1$ et le groupe accepteur porté par la sonde $X_2$ comprennent un noyau coumarine.

**7.** Peptide cyclique selon l'une quelconque des revendications précédentes, dans lequel le groupe donneur porté par la sonde $X_1$ et le groupe accepteur porté par la sonde $X_2$ répondent respectivement aux formules suivantes :

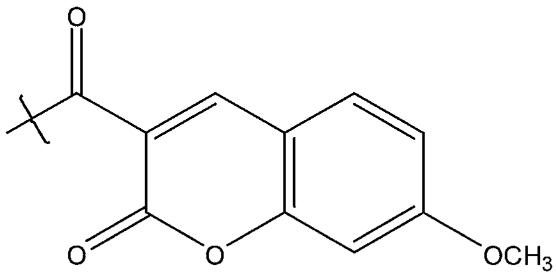

MC

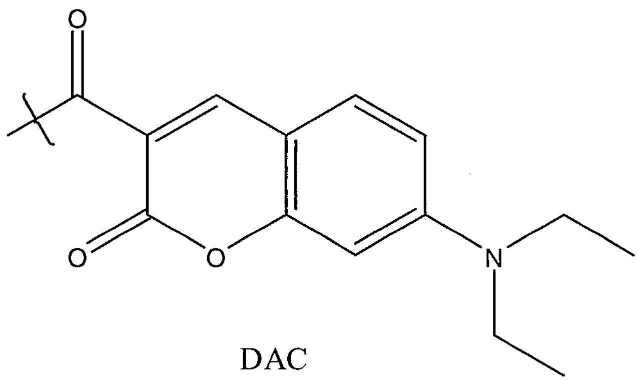

DAC

**8.** Peptide cyclique selon l'une quelconque des revendications précédentes, dans lequel les sondes $X_1$ et $X_2$ sont des restes d'acide aminé.

**9.** Peptide cyclique selon la revendication 8, dans lequel les sondes $X_1$ et $X_2$ répondent respectivement aux formules suivantes :

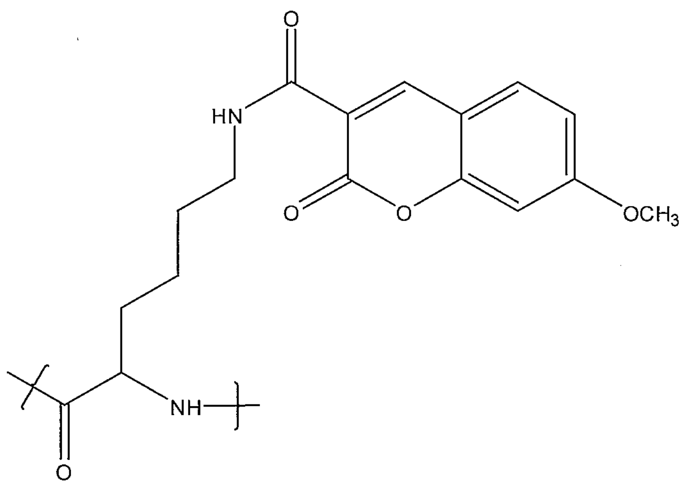

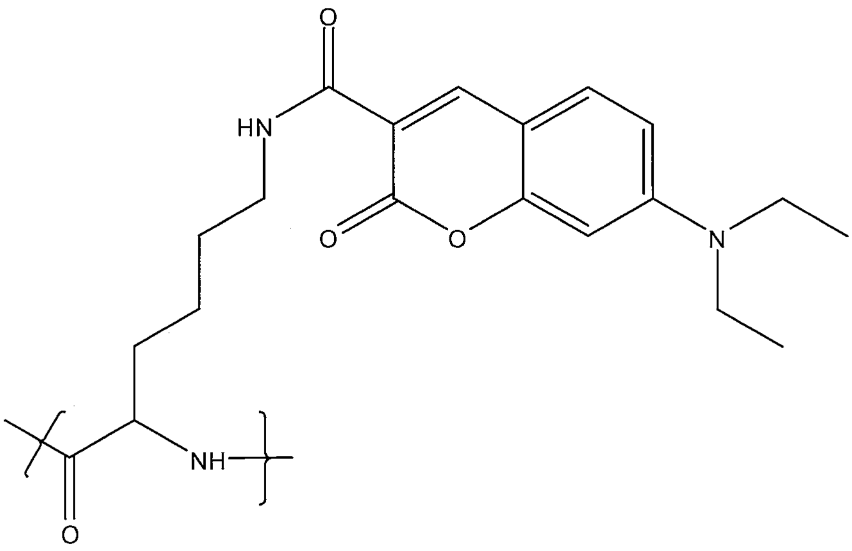

**10.** Peptide cyclique selon la revendication 1, dans lequel les séquences peptidiques $S_1$ et $S_2$ répondent à la formule suivante :

-Gly-Pro-Gln-Gly-Leu-Leu-Gly-Ala-

**11.** Peptide cyclique selon la revendication 10, répondant à la formule suivante :

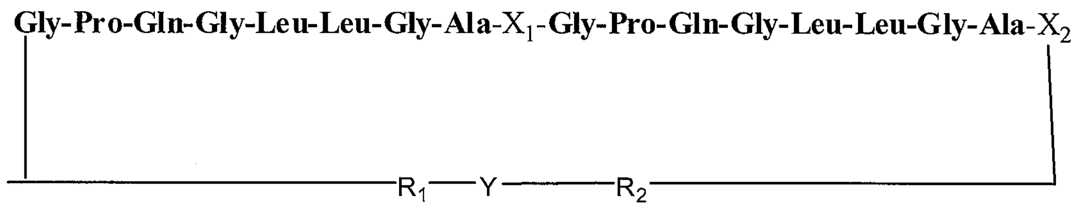

$R_1$, Y et $R_2$ répondant à la même définition que celle donnée à la revendication 1.

**12.** Peptide cyclique selon la revendication 10 ou 11, répondant à la formule suivante :

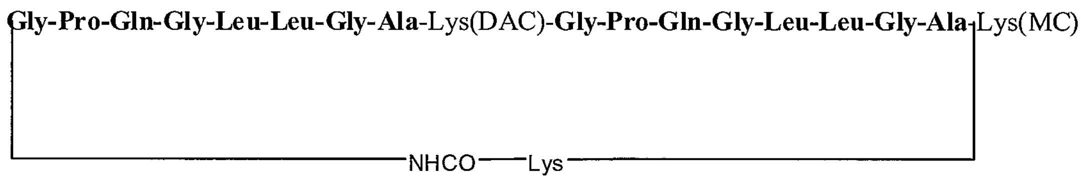

**13.** Procédé de préparation d'un peptide cyclique tel que défini selon la revendication 1, comprenant les étapes suivantes :

a) une étape de préparation d'un peptide linéaire par synthèse chimique sur une phase solide ;
b) une étape de libération du peptide linéaire de la phase solide ; et
c) une étape de couplage des extrémités du peptide linéaire pour former le peptide cyclique.

**14.** Procédé de préparation d'un peptide cyclique tel que défini selon la revendication 1, comprenant les étapes suivantes :

a) une étape de préparation du peptide linéaire par synthèse chimique sur une phase solide ;
b) une étape de couplage de l'extrémité libre du peptide linéaire avec une fonction terminale d'un résidu linéaire du peptide linéaire ; et

c) une étape de libération du peptide cyclique de la phase solide, les étapes b) et c) pouvant être réalisées de façon concomitante.

**15.** Réactif prêt à l'emploi comprenant au moins un peptide cyclique tel que défini selon l'une quelconque des revendications 1 à 12.

**16.** Kit comprenant au moins :

- un premier compartiment comprenant au moins un peptide cyclique tel que défini selon l'une quelconque des revendications 1 à 12 ; et
- un second compartiment comprenant une solution tampon.

**17.** Utilisation d'au moins un peptide cyclique tel que défini selon l'une quelconque des revendications 1 à 12 pour déterminer *in vitro* l'activité d'au moins une enzyme protéase du type MMP.

**Patentansprüche**

**1.** Cyclisches Peptid, das der nachfolgenden Formel entspricht:

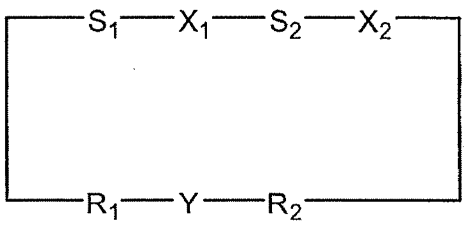

worin:

- $S_1$ eine erste Target-Peptidsequenz eines Proteaseenzyms $E_1$ vom Typ MMP ist;
- $S_2$ eine zweite Target-Peptidsequenz eines Proteaseenzyms $E_2$ vom Typ MMP ist, wobei $S_1$ und $S_2$ identisch oder verschieden sein können, wobei die erste Peptidsequenz $S_1$ und die zweite Peptidsequenz $S_2$ 4 bis 14 Aminosäuren enthalten und $E_1$ und $E_2$ dem gleichen Proteaseenzym oder zwei verschiedenen Proteaseenzymen entsprechen können;
- $X_1$ eine eine fluoreszierende Donorgruppe tragende Sonde ist;
- $X_2$ eine eine fluoreszierende oder nicht fluoreszierende Akzeptorgruppe tragende Sonde ist;
- $R_1$ und $R_2$ unabhängig einer Einfachbindung, einem Aminosäurerest oder einer Peptidsequenz entsprechen;
- Y einer -CONH- oder -NHCO-Gruppe entspricht;

wobei das Proteaseenzym MMP ausgewählt ist aus MMP-1, MMP-2, MMP-3, MMP-5, MMP-7, MMP-8, MMP-9, MMP-10, MMP-11, MMP-12, MMP-13, MMP-14, MMP-15, MMP-16, MMP-17, MMP-19, MMP-23, MMP-25 und MMP-26 und deren Gemische.

**2.** Cyclisches Peptid nach Anspruch 1, wobei.

- dann, wenn sie Target für das Enzym MMP-1 sind, die erste Sequenz $S_1$ und/oder die zweite Sequenz $S_2$ zumindest einer der nachfolgenden Sequenzen entsprechen:

-Gly-Pro-Gln-Gly-Leu-Leu-Gly-Ala-SEQ. 1
-Ala-Pro-Gln-Gly-Ile-Ala-Gly-Gln-SEQ. 2
-Gly-Pro-Gln-Gly-Leu-Ala-Gly-Gln-SEQ.3
-Gly-Pro-Leu-Gly-Ile-Ala-Gly-Ile-SEQ.4
-Gly-Pro-Glu-Gly-Leu-Arg-Val-Gly-SEQ.5
-Tyr-Glu-Ala-Gly-Leu-Gly-Val-Val-SEQ.6
-Ala-Gly-Leu-Gly-Val-Val-Glu-Arg-SEQ.7
-Ala-Gly-Leu-Gly-Ile-Ser-Ser-Thr-SEQ.8

-Gly-Ala-Met-Phe-Leu-Glu-Ala-Ile-SEQ.9
-Ile-Pro-Glu-Asn-Phe-Phe-Gly-Val-SEQ.10
-Thr-Glu-Gly-Glu-Ala-Arg-Gly-Ser-SEQ.11
*-Arg-Ala-Ile-His-Ile-Gln-Ala-Glu-SEQ.12*
*-Leu-Arg-Ala-Tyr-Leu-Leu-Pro-Ala-SEQ.13*
*-Gly-Pro-Leu-Gly-Met-Arg-Gly-Leu-SEQ.14*
*-Pro-Gln-Gly-Leu-Glu-Ala-Lys-SEQ.15*
*-Pro-Leu-Gly-Leu-Trp-Ala-D-Arg-SEQ. 16*
*-Pro-Leu-Ala-Leu-Trp-Ala-Arg-SEQ. 17*
-Pro-Cha-Abu-Cys(Me)-His-Ala-SEQ.18
-Pro-Cha-Gly-Cys(Me)-His-Ala-SEQ. 19
*-Pro-Cha-Gly-Cys(Me)-His-Ala-SEQ . 20*
*-Pro-Leu-Gly-Leu-Dpa-Ala-Arg-SEQ.21*
*-Pro-Leu-Gly-[2-mercapto-4-methyl-pentanoyl]-Leu-Gl SEQ . 22*
*-Pro-Leu-Ala-Leu-Trp-Ala-Arg-SEQ. 17*
*-Pro-Leu-Gly-Cys(Me)-His-Ala-D-Arg-SEQ. 23*
*-Arg-Pro-Leu-Ala-Leu-Trp-Arg-SEQ. 24*
*-Pro-Cha-Gly-Nva-His-Ala-SEQ. 25*
*-Arg-Pro-Lys-Pro-Tyr-Ala-Nva-Trp-Met-SEQ . 26*

oder

- dann, wenn die Target des Enzyms MMP-2 sind, die erste Sequenz $S_1$ und/oder die zweite Sequenz $S_2$ zumindest einer der nachfolgenden Sequenzen entsprechen:

*-Ile-Pro-Glu-Asn-Phe-Phe-Gly-Val-SEQ.10*
-Pro-Pro-Gly-Ala-Tyr-His-Gly-Ala-SEQ.27
*-Arg-Ala-Ile-His-Ile-Gln-Ala-Glu-SEQ.12*
*-Gly-Pro-His-Leu-Leu-Val-Glu-Ala-SEQ. 28*
*-Leu-Arg-Ala-Tyr-Leu-Leu-Pro-Ala-SEQ.13*
-Pro-Gln-Gly-Leu-Glu-Ala-Lys-SEQ.29
*-Pro-Leu-Gly-Leu-Trp-Ala-D-Arg-SEQ . 16*
-Pro-Leu-Ala-Nva-Dpa-Ala-Arg-SEQ.30
*-Pro-Leu-Gly-Leu-Dpa-Ala-Arg-SEQ.21*
*-Pro-Leu-Gly-Met-Trp-Ser-Arg-SEQ. 31*
*-Pro-Leu-Gly-SCH[$CH_2CH(CH_3)_2$]-CO-Leu-Gly-SEQ. 32*
*-Arg-Pro-Pro-Gly-Phe-Ser-Ala-Phe-SEQ. 33*
*-Pro-Leu-Gly-Leu-Dpa-Ala-Arg-SEQ.21*
*-Pro-Leu-Gly-[2-mercapto-4-methyl-pentanoyl]-Leu-Gly-SEQ.22*
*-Pro-Leu-Gly-Cys(Me)-His-Ala-D-Arg-SEQ.23*
*-Arg-Pro-Leu-Ala-Leu-Trp-Arg-SEQ.24*
*-Pro-Cha-Gly-Nva-His-Ala-SEQ. 25*
*-Arg-Pro-Lys-Pro-Tyr-Ala-Nva-Trp-Met-SEQ. 26*

oder

- dann, wenn sie Target des Enzyms MMP-3 sind, die erste Sequenz $S_1$ und/oder die zweite Sequenz $S_2$ zumindest einer der nachfolgenden Sequenzen entsprechen:

*-Gly-Pro-Glu-Gly-Leu-Arg-Val-Gly-SEQ. 5*
-Arg-Val-Gly-Phe-Tyr-Glu-Ser-Asp-SEQ.34
-Leu-Leu-Ser-Ala-Leu-Val-Glu-Thr-SEQ.35
-Glu-Ala-Ile-Pro-Met-Ser-Ile-Pro-SEQ.36
-Ile-Ala-Gly-Arg-Ser-Leu-Asn-Pro-SEQ.37
*-Ile-Pro-Glu-Asn-Phe-Phe-Gly-Val-SEQ. 10*
-Lys-Pro-Gln-Gln-Phe-Phe-Gly-Leu-SEQ.38
-Asp-Val-Ala-Gln-Phe-Val-Leu-Thr-SEQ.39
-Asp-Thr-Leu-Glu-Val-Met-Arg-Lys-SEQ.40
-Asp-Val-Gly-His-Phe-Arg-Thr-Phe-SEQ.41
-Asp-Ser-Gly-Gly-Phe-Met-Leu-Thr-SEQ.42

-Arg-Val-Ala-Glu-Met-Arg-Gly-Glu-SEQ.43
-Asp-Leu-Gly-Arg-Phe-Gln-Thr-Phe-SEQ.44
-Pro-Phe-Ser-Pro-Leu-Val-Ala-Thr-SEQ.45
*-Leu-Arg-Ala-Tyr-Leu-Leu-Pro-Ala-SEQ.13*
-Ala-Pro-Gly-Asn-Ala-Ser-Glu-Ser-SEQ.46
-Phe-Ser-Ser-Glu-Ser-Lys-Arg-Glu-SEQ.47
*-Arg-Ala-Ile-His-Ile-Gln-Ala-Glu-SEQ.12*
*-Gly-Pro-His-Leu-Leu-Val-Glu-Ala-SEQ.28*
-Pro-Pro-Glu-Glu-Leu-Lys-Phe-Gln-SEQ.48
*-Gly-Pro-Leu-Gly-Met-Arg-Gly-Leu-SEQ. 49*
*-Pro-Gln-Gly-Leu-Glu-Ala-Lys-SEQ. 15*
-Arg-Pro-Lys-Pro-Val-Glu-Nva-Trp-Arg-Glu-Ala-Lys-SEQ. 50
*-Pro-Leu-Gly-Leu-Trp-Ala-D-Arg-SEQ. 16*
*-Pro-Tyr-Ala-Tyr-Trp-Met-Arg-SEQ. 51*
*-Arg-Pro-Lys-Pro-Leu-Ala-Nva-Trp-SEQ. 52*
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-f2-mercapto-4-methyl-pentanoyl]-Leu-Gly-SEQ. 22*
*-Arg-Pro-Lys-Pro-Tyr-Ala-Nva-Trp-Met-SEQ. 26*
*-Arg-Pro-Lys-Pro-Val-Glu-Nva-Trp-Arg-SEQ. 50*

oder
- dann, wenn sie Target des Enzyms MMP-5 sind, die erste Sequenz $S_1$ und/oder die zweite Sequenz $S_2$ zumindest einer der nachfolgenden Sequenzen entsprechen:

*-Arg-Pro-Lys-Pro-Val-Glu-Nva-Trp-Arg-Glu-Ala-Lys-SEQ . 50*

oder
- dann, wenn sie Target des Enzyms MMP-7 sind, die erste Sequenz $S_1$ und/oder die zweite Sequenz $S_2$ zumindest einer der nachfolgenden Sequenzen entsprechen:

*-Pro-Leu-Gly-Leu-Trp-Ala-D-Arg-SEQ. 16*
*-Pro-Leu-Gly-Leu-Dpa-Ala-Arg-SEQ. 21*
*-Arg-Pro-Leu-Ala-Leu-Trp-Arg-Ser-SEQ. 24*
*-Pro-Cha-Gly-Cys(Me)-His-Ala-SEQ . 20*
*-Pro-Leu-Gly-[2-mercapto-4-methyl-pentanoyl]-Leu-Gly-SEQ. 22*
*-Pro-Leu-Ala-Leu-Trp-Ala-Arg-SEQ. 17*
*-Pro-Tyr-Ala-Tyr-Trp-Met-Arg-SEQ. 51*
*-Arg-Pro-Lys-Pro-Leu-Ala-Nva-Trp-SEQ. 52*

oder
- dann, wenn sie Target des Enzyms MMP-8 sind, die erste Sequenz $S_1$ und/oder die zweite Sequenz $S_2$ zumindest einer der nachfolgenden Sequenzen entsprechen:

*-Pro-Leu-Ala-Tyr-Trp-Ala-Arg-SEQ. 53*
*-Pro-Leu-Gly-Leu-Trp-Ala-D-Arg-SEQ. 16*
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Pro-Leu-Ala-Leu-Trp-Ala-Arg-SEQ. 17*
*-Pro-Leu-Gly-Cys(Me)-His-Ala-D-Arg-SEQ. 23*
*-Arg-Pro-Leu-Ala-Leu-Trp-Arg-SEQ. 24*
*-Pro-Cha-Gly-Nva-His-Ala-SEQ. 25*

oder
- dann, wenn sie Target des Enzyms MMP-9 sind, die erste Sequenz $S_1$ und/oder die zweite Sequenz $S_2$ zumindest einer der nachfolgenden Sequenzen entsprechen:

-Gly-Pro-Pro-Gly-Val-Val-Gly-Pro-SEQ.54
-Gly-Pro-Pro-Gly-Leu-Arg-Gly-Glu-SEQ.55
-Gly-Pro-Gly-Gly-Val-Val-Gly-Pro-SEQ.56
-Ile-Pro-Gln-Asn-Phe-Phe-Gly-Val-SEQ.57
-Pro-Pro-Gly-Ala-Tyr-His-Gly-Ala-SEQ.58
*-Arg-Ala-Ile-His-Ile-Gln-Ala-Glu-SEQ.12*
*-Gly-Pro-Leu-Gly-Met-Arg-Gly-Leu-SEQ. 14*
*-Pro-Gln-Gly-Leu-Glu-Ala-Lys-SEQ. 15*
*-Arg-Pro-Lys-Pro-Val-Glu-Nva-Trp-Arg-Glu-Ala-Lys-SEQ.50*
*-Pro-Leu-Gly-Leu-Trp-Ala-D-Arg-SEQ. 16*
*-Pro-Cha-Gly-Cys(Me)-His-Ala-SEQ. 19*
*-Pro-Leu-Gly-*$SCH[CH_2CH(CH_3)_2]$*-CO-Leu-Gly-SEQ. 32*
*-Arg-Pro-Pro-Gly-Phe-Ser-Ala-Phe-SEQ. 33*
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Pro-Leu-Gly-Cys(Me)-His-Ala-D-Arg-SEQ. 23*

oder
- dann, wenn sie Target des Enzyms MMP-10 sind, die erste Sequenz $S_1$ und/oder die zweite Sequenz $S_2$ zumindest einer der nachfolgenden Sequenzen entsprechen:

*-Arg-Ala-Ile-His-Ile-Gln-Ala-Glu-SEQ.12*
*-Gly-Pro-His-Leu-Leu-Val-Glu-Ala-SEQ.28*
*-Pro-Leu-Gly-Leu-Dpa-Ala-Arg-SEQ. 21*

oder
- dann, wenn sie Target des Enzyms MMP-11 sind, die erste Sequenz $S_1$ und/oder die zweite Sequenz $S_2$ zumindest einer der nachfolgenden Sequenzen entsprechen:

*-Pro-Cha-Gly-Cys(Me)-His-Ala-SEQ. 20*
*-Pro-Leu-Gly-Leu-Dpa-Ala-Arg-SEQ. 21*

oder
- dann, wenn sie Target des Enzyms MMP-12 sind, die erste Sequenz $S_1$ und/oder die zweite Sequenz $S_2$ zumindest einer der nachfolgenden Sequenzen entsprechen:

*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Pro-Leu-Ala-Leu-Trp-Ala-Arg-SEQ. 17*
*-Pro-Leu-Gly-Cys(Me)-His-Ala-D-Arg-SEQ . 23*
*-Pro-Tyr-Ala-Tyr-Trp-Met-Arg-SEQ. 51*
*-Arg-Pro-Lys-Pro-Leu-Ala-Nva-Trp-SEQ . 52*
*-Arg-Pro-Leu-Ala-Leu-Trp-Arg-SEQ. 24*
*-Pro-Cha-Gly-Nva-His-Ala-SEQ. 25*
*-Arg-Pro-Lys-Pro-Tyr-Ala-Nva-Trp-Met-SEQ . 26*
*-Arg-Pro-Lys-Pro-Val-Glu-Nva-Trp-Arg-SEQ. 50*
*-Arg-Pro-Lys-Pro-Gln-Gln-Phe-Trp-SEQ. 59*

oder
- dann, wenn sie Target des Enzyms MMP-13 sind, die erste Sequenz $S_1$ und/oder die zweite Sequenz $S_2$ zumindest einer der nachfolgenden Sequenzen entsprechen:

*- Gly-Pro-Leu-Gly-Met-Arg-Gly-Leu-SEQ. 49*
*- Arg-Pro-Lys-Pro-Val-Glu-Nva-Trp-Arg-Glu-Ala-Lys-SEQ. 50*
*- Pro-Cha-Gly-Nva-His-A1a-Dpa-SEQ. 25*
*- Pro-Cha-Gly-Cys(Me)-His-Ala-SEQ. 20*

- *Pro-Leu-Gly-Leu-Dpa-Ala-Arg-SEQ. 21*
- *Pro-Leu-Gly-[2-mercapto-4-methyl-pentanoyl]-Leu-Gly-SEQ.22*
- *Pro-Leu-Gly-Leu-Trp-A1a-D-Arg-SEQ. 16*
- *Pro-Leu-Ala-Leu-Trp-Ala-Arg-SEQ. 17*
- *Pro-Leu-Gly-Cys(Me)-His-Ala-D-Arg-SEQ. 23*
- *Pro-Leu-Gly-Met-Trp-Ser-Arg-SEQ. 31*
- *Pro-Tyr-Ala-Tyr-Trp-Met-Arg-SEQ. 51*
- *Arg-Pro-Lys-Pro-Leu-Ala-Nva-Trp-SEQ. 52*
- *Arg-Pro-Leu-Ala-Leu-Trp-Arg-SEQ. 24*
- *Pro-Leu-Ala-Tyr-Trp-Ala-Arg-SEQ. 53*
- *Pro-Cha-Gly-Nva-His-Ala-SEQ. 25 -Arg-Pro-Lys*
- *Pro-Tyr-Ala-Nva-Trp-Met-SEQ. 26*
- *Arg-Pro-Lys-Pro-Gln-Gln-Phe-Trp-SEQ. 59*

oder
- dann, wenn sie Target des Enzyms MMP-14 sind, die erste Sequenz $S_1$ und/oder die zweite Sequenz $S_2$ zumindest einer der nachfolgenden Sequenzen entsprechen:

-Pro-Leu-Ala-Cys(*p*-OMeBz)-Trp-Ala-Arg-SEQ. 60
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*

oder
- dann, wenn sie Target des Enzyms MMP-15 sind, die erste Sequenz $S_1$ und/oder die zweite Sequenz $S_2$ zumindest einer der nachfolgenden Sequenzen entsprechen:

*-Pro-Leu-Gly-Leu-Dpa-Ala-Arg-SEQ. 21*

oder
- dann, wenn sie Target des Enzyms MMP-16 sind, die erste Sequenz $S_1$ und/oder die zweite Sequenz $S_2$ zumindest einer der nachfolgenden Sequenzen entsprechen:

*-Pro-Leu-Gly-Leu-Dpa-Ala-Arg-SEQ. 21*

oder
- dann, wenn sie Target des Enzyms MMP-17 sind, die erste Sequenz $S_1$ und/oder die zweite Sequenz $S_2$ zumindest einer der nachfolgenden Sequenzen entsprechen:

*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*

oder
- dann, wenn sie Target des Enzyms MMP-19 sind, die erste Sequenz $S_1$ und/oder die zweite Sequenz $S_2$ zumindest einer der nachfolgenden Sequenzen entsprechen:

*-Pro-Leu-Gly-Leu-Dpa-Ala-Arg-SEQ. 21*

oder
- dann, wenn sie Target des Enzyms MMP-23 sind, die erste Sequenz $S_1$ und/oder die zweite Sequenz $S_2$ zumindest einer der nachfolgenden Sequenzen entsprechen:

*-Pro-Leu-Gly-Leu-Dpa-Ala-Arg-SEQ. 21*

oder
- dann, wenn sie Target des Enzyms MMP-25 sind, die erste Sequenz $S_1$ und/oder die zweite Sequenz $S_2$ zumindest einer der nachfolgenden Sequenzen entsprechen:

*-Pro-Leu-Gly-Leu-Dpa-Ala-Arg-SEQ. 21*

oder

- dann, wenn sie Target des Enzyms MMP-26 sind, die erste Sequenz $S_1$ und/oder die zweite Sequenz $S_2$ zumindest einer der nachfolgenden Sequenzen entsprechen:

*-Pro-Leu-Gly-Leu-Trp-Ala-D-Arg-SEQ. 16*

**3.** Cyclisches Peptid nach einem der vorangehenden Ansprüche, wobei die von der Sonde $X_1$ getragene Donorgruppe und die von der Sonde $X_2$ getragene Akzeptorgruppe einen aromatischen Ring enthalten.

**4.** Cyclisches Peptid nach Anspruch 3, wobei der aromatische Ring ein Benzol-, ein Anthracen- oder ein Cumarin-Ring ist.

**5.** Cyclisches Peptid nach einem der vorangehenden Ansprüche, wobei die von der Sonde $X_1$ getragene Donorgruppe und die von der Sonde $X_2$ getragene Akzeptorgruppe ausgewählt sind aus folgenden Paaren:

*Tryptophan/2,4-Dinitrophenyl;
* O-Aminobenzoesäure/2,4-Dinitrophenyl;
*(7-Methoxycoumarin-4-yl)-Acetyl/2,4-Dinitrophenyl;
*(7-Methoxycoumarin-4-yl)-Acetyl/N-3-(2,4-Dinitrophenyl)-L-2,3-Diaminopropyl;
*Tryptophan/Dansyl;
*N-Methylanthranoyl/2,4-Dinitrophenyl;
*6, 7-Dimethoxycoumarin-4-yl-Acetyl / 6-N-(7-Nitrobenz-2-Oxa-1, 3-Diazol-4-yl)Amino)hexansäure;
*5-(2'-Aminomethyl)Naphthalinsulfonsäure / 4-(4'-Dimethylamino Phenylazo)Benzoesäure;
* 7-Methoxycoumarin-3-Carboxylsäure / 7-Diethylaminocoumarin-3-Carboxylsäure.

**6.** Cyclisches Peptid nach einem der vorangehenden Ansprüche, wobei die von der Sonde $X_1$ getragene Donorgruppe und die von der Sonde $X_2$ getragene Akzeptorgruppe einen Cumarin-Ring aufweisen.

**7.** Cyclisches Peptid nach einem der vorangehenden Ansprüche, wobei die von der Sonde $X_1$ getragene Donorgruppe und die von der Sonde $X_2$ getragene Akzeptorgruppe den jeweiligen, nachfolgenden Formeln entsprechen:

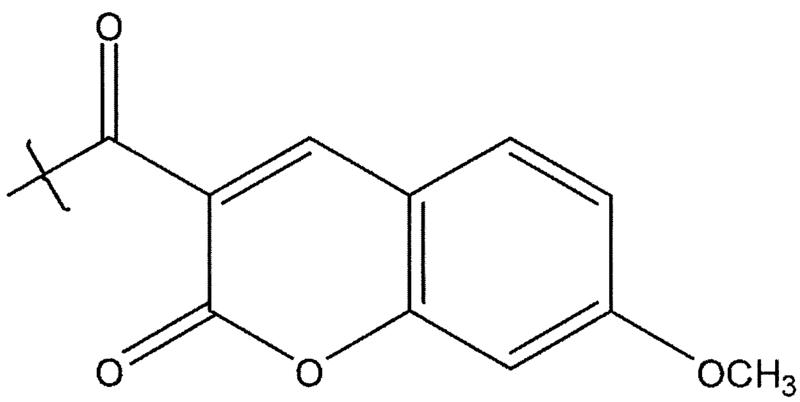

MC

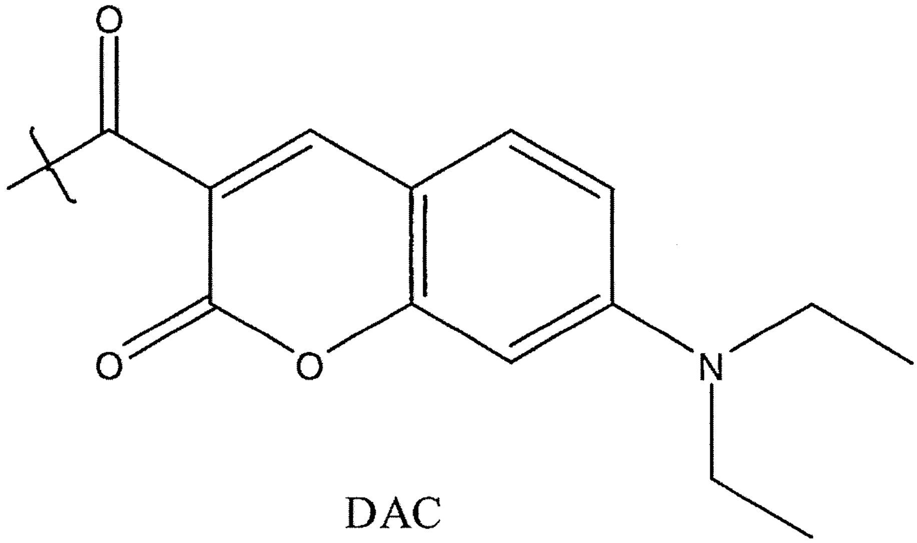

DAC

8. Cyclisches Peptid nach einem der vorangehenden Ansprüche, wobei die von Sonden $X_1$ und $X_2$ Aminosäurereste sind.

9. Cyclisches Peptid nach Anspruch 8, wobei die Sonden $X_1$ und $X_2$ den jeweiligen, nachfolgenden Formeln entsprechen:

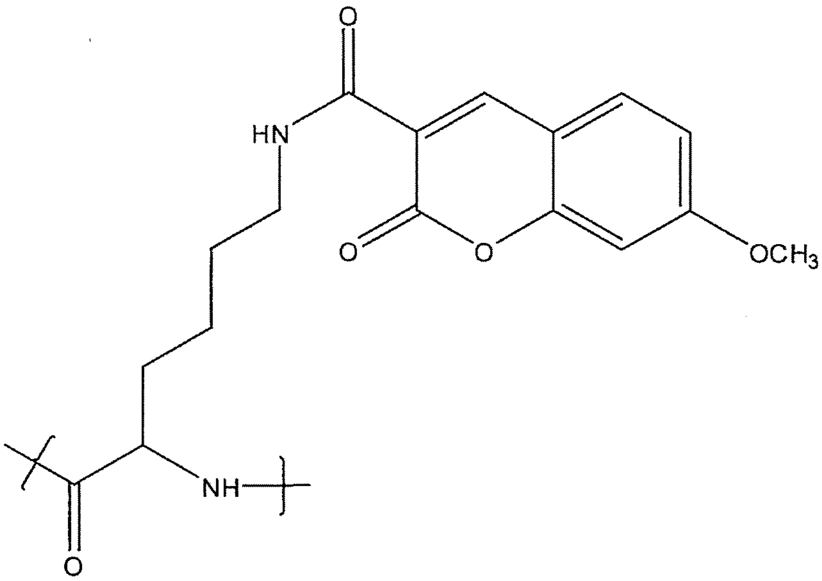

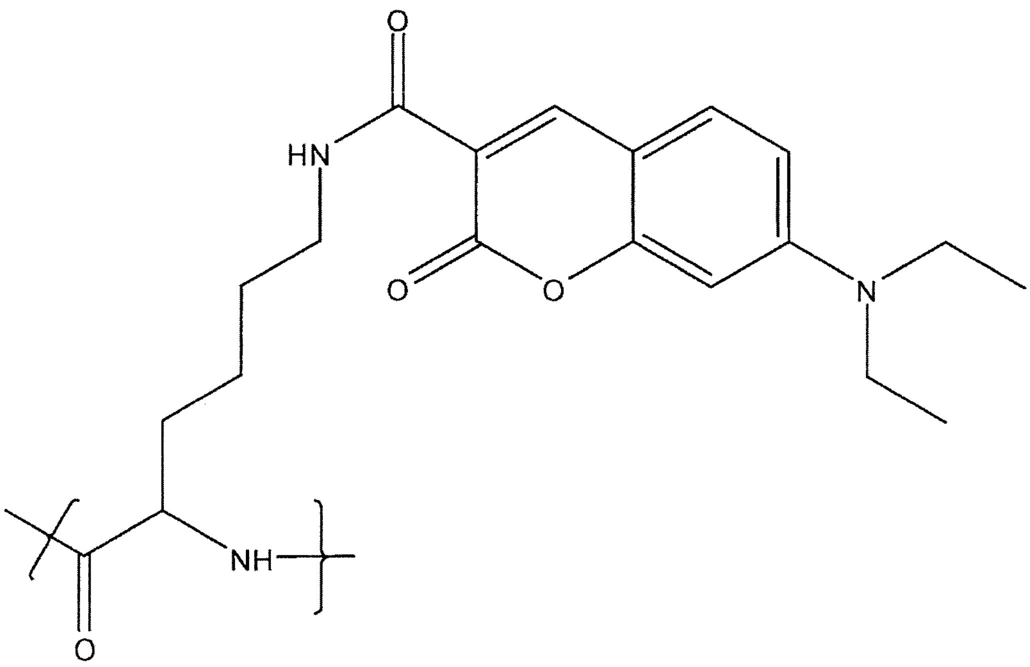

**10.** Cyclisches Peptid nach Anspruch 1, wobei die peptischen Sequenzen $S_1$ und $S_2$ der nachfolgenden Formel entsprechen:

-Gly-Pro-Gln-Gly-Leu-Leu-Gly-Ala-

**11.** Cyclisches Peptid nach Anspruch 10, das der nachfolgenden Formel entspricht:

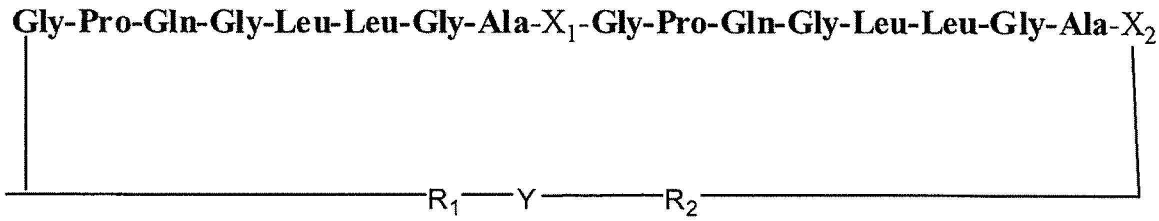

wobei $R_1$, Y und $R_2$ der gleichen Definition wie der in Anspruch 1 angegebenen entsprechen.

**12.** Cyclisches Peptid nach Anspruch 10 oder 11, das der nachfolgenden Formel entspricht:

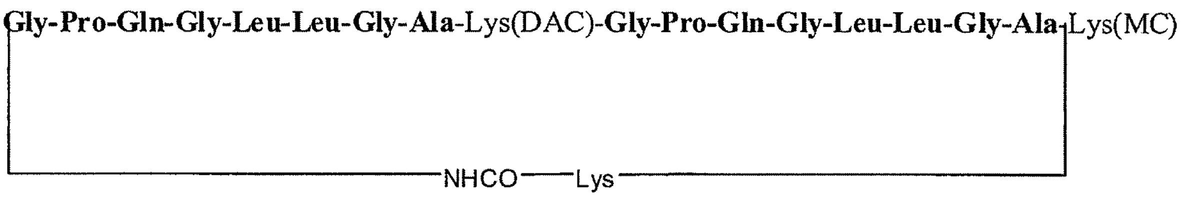

**13.** Verfahren zum Herstellen eines cyclischen Peptids wie in Anspruch 1 definiert, umfassend die nachfolgenden Schritte:

a) einen Schritt des Herstellens eines lineares Peptids durch chemische Festphasensynthese;
b) einen Schritt des Freisetzens des linearen Peptids aus der Festphase; und
c) einen Schritt des Koppelns der Enden des linearen Peptides, um das cyclische Peptid zu bilden.

**14.** Verfahren zum Herstellen eines cyclischen Peptids wie in Anspruch 1 definiert, umfassend die nachfolgenden

Schritte:

a) einen Schritt des Herstellens des linearen Peptids durch chemische Festphasensynthese;
b) einen Schritt des Koppelns des freien Endes des lineares Peptides mit einer Endfunktion eines linearen Rests des lineares Peptids; und
c) einen Schritt des Freisetzens des cyclischen Peptids aus der Festphase, wobei die Schritte b) und c) gleichzeitig erfolgen können.

**15.** Gebrauchsfertiges Reagenz, enthaltend zumindest ein cyclisches Peptid wie in einem der Ansprüche 1 bis 12 definiert ist.

**16.** Kit, enthaltend zumindest:

- ein erstes Fach, das zumindest ein cyclisches Peptid wie nach einem der Ansprüche 1 bis 12 definiert enthält; und
- ein zweites Fach, das eine Pufferlösung enthält.

**17.** Verbindung zumindest eines cyclischen Peptides wie in einem der Ansprüche 1 bis 12 definiert zum Ermitteln in vitro der Aktivität zumindest eines Proteaseenzyms vom Typ MMP.

## Claims

**1.** Cyclic peptide meeting the following formula:

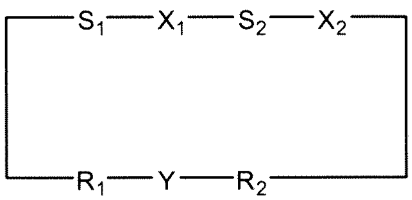

wherein:

- $S_1$ is a first target peptide sequence of a protease enzyme $E_1$ of MMP type;
- $S_2$ is a second target peptide sequence of a protease enzyme $E_2$ of MMP type, $S_1$ and $S_2$ possibly being the same or different, said first peptide sequence $S_1$ and said second peptide sequence $S_2$ comprising 4 to 14 amino acids, $E_1$ and $E_2$ possibly corresponding to the same protease enzyme or to two different protease enzymes;
- $X_1$ is a probe carrying a fluorescent donor group;
- $X_2$ is a probe carrying a fluorescent or non-fluorescent acceptor group;
- $R_1$ and $R_2$ independently correspond to a simple bond, an amino acid residue or a peptide sequence;
- Y corresponds to a -CONH- or -NHCO- group,

the MMP protease enzyme being chosen from among MMP-1, MMP-2, MMP-3, MMP-5, MMP-7, MMP-8, MMP-9, MMP-10, MMP-11, MMP-12, MMP-13, MMP-14, MMP-15, MMP-16, MMP-17, MMP-19, MMP-23, MMP-25 and MMP-26 and mixtures thereof.

**2.** The cyclic peptide according to claim 1, wherein:

- when they are the target of the MMP-1 enzyme, the first sequence $S_1$ and/or the second sequence $S_2$, correspond to at least one of the following sequences:

-Gly-Pro-Gln-Gly-Leu-Leu-Gly-Ala-SEQ. 1
-Ala-Pro-Gln-Gly-Ile-Ala-Gly-Gln-SEQ. 2
-Gly-Pro-Gln-Gly-Leu-Ala-Gly-Gln-SEQ.3
-Gly-Pro-Leu-Gly-Ile-Ala-Gly-Ile-SEQ.4

-Gly-Pro-Glu-Gly-Leu-Arg-Val-Gly-SEQ.5
-Tyr-Glu-Ala-Gly-Leu-Gly-Val-Val-SEQ.6
-Ala-Gly-Leu-Gly-Val-Val-Glu-Arg-SEQ.7
-Ala-Gly-Leu-Gly-Ile-Ser-Ser-Thr-SEQ.8
-Gly-Ala-Met-Phe-Leu-Glu-Ala-Ile-SEQ.9
-Ile-Pro-Glu-Asn-Phe-Phe-Gly-Val-SEQ.10
-Thr-Glu-Gly-Glu-Ala-Arg-Gly-Ser-SEQ.11
*-Arg-Ala-Ile-His-Ile-Gln-Ala-Glu-SEQ.12*
*-Leu-Arg-Ala-Tyr-Leu-Leu-Pro-Ala-SEQ.13*
*-Gly-Pro-Leu-Gly-Met-Arg-Gly-Leu-SEQ.14*
*-Pro-Gln-Gly-Leu-Glu-Ala-Lys-SEQ.15*
*-Pro-Leu-Gly-Leu-Trp-Ala-D-Arg-SEQ. 16*
*-Pro-Leu-Ala-Leu-Trp-Ala-Arg-SEQ. 17*
-Pro-Cha-Abu-Cys(Me)-His-Ala-SEQ.18
-Pro-Cha-Gly-Cys(Me)-His-Ala-SEQ. 19
*-Pro-Cha-Gly-Cys(Me)-His-Ala-SEQ. 20*
*-Pro-Leu-Gly-Leu-Dpa-Ala-Arg-SEQ.21*
*-Pro-Leu-Gly-[2-mercapto-4-methyl-pentanoyl]-Leu-G1 SEQ. 22*
*-Pro-Leu-Ala-Leu-Trp-Ala-Arg-SEQ . 17*
*-Pro-Leu-Gly-Cys(Me)-His-Ala-D-Arg-SEQ. 23*
*-Arg-Pro-Leu-Ala-Leu-Trp-Arg-SEQ. 24*
*-Pro-Cha-Gly-Nva-His-Ala-SEQ. 25*
*-Arg-Pro-Lys-Pro-Tyr-Ala-Nva-Trp-Met-SEQ. 26* or

- when they are the target of the MMP-2 enzyme, the first sequence $S_1$ and/or the second sequence $S_2$ correspond to at least one of the following sequences:

*-Ile-Pro-Glu-Asn-Phe-Phe-Gly-Val-SEQ.10*
-Pro-Pro-Gly-Ala-Tyr-His-Gly-Ala-SEQ.27
*-Arg-Ala-Ile-His-Ile-Gln-Ala-Glu-SEQ.12*
*-Gly-Pro-His-Leu-Leu-Zlal-Glu-Ala-SEQ.28*
*-Leu-Arg-Ala-Tyr-Leu-Leu-Pro-Ala-SEQ.13*
-Pro-Gln-Gly-Leu-Glu-Ala-Lys-SEQ.29
*-Pro-Leu-Gly-Leu-Trp-Ala-D-Arg-SEQ. 16*
-Pro-Leu-Ala-Nva-Dpa-Ala-Arg-SEQ.30
*-Pro-Leu-Gly-Leu-Dpa-Ala-Arg-SEQ.21*
*-Pro-Leu-Gly-Met-Trp-Ser-Arg-SEQ. 31*
*-Pro-Leu-Gly-$SCH[CH_2CH(CH_3)_2]$-CO-Leu-Gly-SEQ. 32*
*-Arg-Pro-Pro-Gly-Phe-Ser-Ala-Phe-SEQ. 33*
*-Pro-Leu-Gly-Leu-Dpa-Ala-Arg-SEQ.21*
*-Pro-Leu-Gly-[2-mercapto-4-methyl-pentanoyl]-Leu-Gly-SEQ.22*
*-Pro-Leu-Gly-Cys(Me)-His-Ala-D-Arg-SEQ.23*
*-Arg-Pro-Leu-Ala-Leu-Trp-Arg-SEQ.24*
*-Pro-Cha-Gly-Nva-His-Ala-SEQ. 25*
*-Arg-Pro-Lys-Pro-Tyr-Ala-Nva-Trp-Met-SEQ. 26*

or
- when they are the target of the MMP-3 enzyme, the first sequence $S_1$ and/or the second sequence $S_2$ correspond to at least one of the following sequences:

*-Gly-Pro-Glu-Gly-Leu-Arg-Val-Gly-SEA. 5*
-Arg-Val-Gly-Phe-Tyr-Glu-Ser-Asp-SEQ.34
-Leu-Leu-Ser-Ala-Leu-Val-Glu-Thr-SEQ.35
-Glu-Ala-Ile-Pro-Met-Ser-Ile-Pro-SEQ.36
-Ile-Ala-Gly-Arg-Ser-Leu-Asn-Pro-SEQ.37
*-Ile-Pro-Glu-Asn-Phe-Phe-Gly-Val-SEQ.10*
-Lys-Pro-Gln-Gln-Phe-Phe-Gly-Leu-SEQ.38
-Asp-Val-Ala-Gln-Phe-Val-Leu-Thr-SEQ.39

-Asp-Thr-Leu-Glu-Val-Met-Arg-Lys-SEQ.40
-Asp-Val-Gly-His-Phe-Arg-Thr-Phe-SEQ.41
-Asp-Ser-Gly-Gly-Phe-Met-Leu-Thr-SEQ.42
-Arg-Val-Ala-Glu-Met-Arg-Gly-Glu-SEQ.43
-Asp-Leu-Gly-Arg-Phe-Gln-Thr-Phe-SEQ.44
-Pro-Phe-Ser-Pro-Leu-Val-Ala-Thr-SEQ.45
*-Leu-Arg-Ala-Tyr-Leu-Leu-Pro-Ala-SEQ.13*
-Ala-Pro-Gly-Asn-Ala-Ser-Glu-Ser-SEQ.46
-Phe-Ser-Ser-Glu-Ser-Lys-Arg-Glu-SEQ.47
*-Arg-Ala-Ile-His-Ile-Gln-Ala-Glu-SEQ.12*
*-Gly-Pro-His-Leu-Leu-Val-Glu-Ala-SEQ.28*
-Pro-Pro-Glu-Glu-Leu-Lys-Phe-Gln-SEQ.48
*-Gly-Pro-Leu-Gly-Met-Arg-Gly-Leu-SEQ. 49*
*-Pro-Gln-Gly-Leu-Glu-Ala-Lys-SEQ. 15*
-Arg-Pro-Lys-Pro-Val-Glu-Nva-Trp-Arg-Glu-Ala-Lys-SEQ. 50
*-Pro-Leu-Gly-Leu-Trp-Ala-D-Arg-SEQ. 16*
*-Pro-Tyr-Ala-Tyr-Trp-Met-Arg-SEQ. 51*
*-Arg-Pro-Lys-Pro-Leu-Ala-Nva-Trp-SEQ . 52*
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Arg-Pro-Lys-Pro-Tyr-Ala-Nva-Trp-Met-SEQ . 26*
*-Arg-Pro-Lys-Pro-Val-Glu-Nva-Trp-Arg-SEQ. 50*

or

- when they are the target of the MMP-5 enzyme, the first sequence $S_1$ and/or the second sequence $S_2$ correspond to at least one of the following sequences:

*-Arg-Pro-Lys-Pro-Val-Glu-Nva-Trp-Arg-Glu-Ala-Lys-SEQ. 50*

or

- when they are the target of the MMP-7 enzyme, the first sequence $S_1$ and/or the second sequence $S_2$ correspond to at least one of the following sequences:

- *Pro-Leu-Gly-Leu-Trp-Ala-D-Arg-SEQ. 16*
- *Pro-Leu-Gly-Leu-Dpa-Ala-Arg-SEQ. 21*
- *Arg-Pro-Leu-Ala-Leu-Trp-Arg-Ser-SEQ. 24*
- *Pro-Cha-Gly-Cys(Me)-His-Ala-SEQ . 20*
- *Pro-Leu-Gly-[2-mercapto-4-methyl-pentanoyl]-Leu-Gly-SEQ. 22*
- *Pro-Leu-Ala-Leu-Trp-Ala-Arg-SEQ. 17*
- *Pro-Tyr-Ala-Tyr-Trp-Met-Arg-SEQ . 51*
- *Arg-Pro-Lys-Pro-Leu-Ala-Nva-Trp-SEQ. 52*

or

- when they are the target of the MMP-8 enzyme, the first sequence $S_1$ and/or the second sequence $S_2$ correspond to at least one of the following sequences:

- *Pro-Leu-Ala-Tyr-Trp-Ala-Arg-SEQ. 53*
- *Pro-Leu-Gly-Leu-Trp-Ala-D-Arg-SEQ. 16*
- *Pro-Cha-Gly-Cys(Me)-His-Ala-SEQ. 20*
- *Pro-Leu-Gly-Leu-Dpa-Ala-Arg-SEQ. 21*
- *Pro-Leu-Gly-[2-mercapto-4-methyl-pentanoyl]-Leu-Gly-SEQ. 22*
- *Pro-Leu-Ala-Leu-Trp-Ala-Arg-SEQ. 17*
- *Pro-Leu-Gly-Cys(Me)-His-Ala-D-Arg-SEQ. 23*
- *Arg-Pro-Leu-Ala-Leu-Trp-Arg-SEQ. 24*
- *Pro-Cha-Gly-Nva-His-Ala-SEQ. 25*

or

- when they are the target of the MMP-9 enzyme, the first sequence $S_1$ and/or the second sequence $S_2$ correspond to at least one of the following sequences:

-Gly-Pro-Pro-Gly-Val-Val-Gly-Pro-SEQ.54
-Gly-Pro-Pro-Gly-Leu-Arg-Gly-Glu-SEQ.55
-Gly-Pro-Gly-Gly-Val-Val-Gly-Pro-SEQ.56
-Ile-Pro-Gln-Asn-Phe-Phe-Gly-Val-SEQ.57
-Pro-Pro-Gly-Ala-Tyr-His-Gly-Ala-SEQ.58
*-Arg-Ala-Ile-His-Ile-Gln-Ala-Glu-SEQ.12*
*-Gly-Pro-Leu-Gly-Met-Arg-Gly-Leu-SEQ. 14*
*-Pro-Gln-Gly-Leu-Glu-Ala-Lys-SEQ. 15*
*-Arg-Pro-Lys-Pro-Val-Glu-Nva-Trp-Arg-Glu-Ala-Lys-SEQ.50*
*-Pro-Leu-Gly-Leu-Trp-Ala-D-Arg-SEQ. 16*
*-Pro-Cha-Gly-Cys(Me)-His-Ala-SEQ. 19*
*-Pro-Leu-Gly-*$SCH[CH_2CH(CH_3)_2]$*-CO-Leu-Gly-SEQ. 32*
*-Arg-Pro-Pro-Gly-Phe-Ser-Ala-Phe-SEQ. 33*
*-Pro-Cha-Gly-Cys (Me) -His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Pro-Leu-Gly-Cys(Me)-His-Ala-D-Arg-SEQ. 23*

or
- when they are the target of the MMP-10 enzyme, the first sequence $S_1$ and/or the second sequence $S_2$ correspond to at least one of the following sequences:

*-Arg-Ala-Ile-His-Ile-Gln-Ala-Glu-SEQ.12*
*-Gly-Pro-His-Leu-Leu-Val-Glu-Ala-SEQ.28*
*-Pro-Leu-Gly-Leu-Dpa-Ala-Arg-SEQ. 21*

or
- when they are the target of the MMP-11 enzyme, the first sequence $S_1$ and/or the second sequence $S_2$ correspond to at least one of the following sequences:

*-Pro-Cha-Gly-Cys(Me)-His-Ala-SEQ. 20*
*-Pro-Leu-Gly-Leu-Dpa-Ala-Arg-SEQ. 21*

or
- when they are the target of the MMP-12 enzyme, the first sequence $S_1$ and/or the second sequence $S_2$ correspond to at least one of the following sequences:

*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*
*-Pro-Leu-Ala-Leu-Trp-Ala-Arg-SEQ. 17*
*-Pro-Leu-Gly-Cys(Me)-His-Ala-D-Arg-SEQ. 23*
*-Pro-Tyr-Ala-Tyr-Trp-Met-Arg-SEQ. 51*
*-Arg-Pro-Lys-Pro-Leu-Ala-Nva-Trp-SEQ. 52*
*-Arg-Pro-Leu-Ala-Leu-Trp-Arg-SEQ. 24*
*-Pro-Cha-Gly-Nva-His-Ala-SEQ . 25*
*-Arg-Pro-Lys-Pro-Tyr-Ala-Nva-Trp-Met-SEQ. 26*
*-Arg-Pro-Lys-Pro-Val-Glu-Nva-Trp-Arg-SEQ. 50*
*-Arg-Pro-Lys-Pro-Gln-Gln-Phe-Trp-SEQ . 59*

or
- when they are the target of the MMP-13 enzyme, the first sequence $S_1$ and/or the second sequence $S_2$ correspond to at least one of the following sequences:

*-Gly-Pro-Leu-Gly-Met-Arg-Gly-Leu-SEQ. 49*

*-Arg-Pro-Lys-Pro-Val-Glu-Nva-Trp-Arg-Glu-Ala-Lys-SEQ. 50*
*-Pro-Cha-Gly-Nva-His-Ala-Dpa-SEQ. 25*
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ.22*
*-Pro-Leu-Gly-Leu-Trp-Ala-D-Arg-SFQ. 16*
*-Pro-Leu-Ala-Leu-Trp-Ala-Arg-SEQ. 17*
*-Pro-Leu-Gly-Cys(Me)-His-Ala-D-Arg-SEQ. 23*
*-Pro-Leu-Gly-Met-Trp-Ser-Arg-SEQ. 31*
*-Pro-Tyr-Ala-Tyr-Trp-Met-Arg-SEQ . 51*
*-Arg-Pro-Lys-Pro-Leu-Ala-Nva-Trp-SEQ. 52*
*-Arg-Pro-Leu-Ala-Leu-Trp-Arg-SEQ. 24*
*-Pro-Leu-Ala-Tyr-Trp-Ala-Arg-SEQ. 53*
*-Pro-Cha-Gly-Nva-His-Ala-SEQ. 25*
*-Arg-Pro-Lys-Pro-Tyr-Ala-Nva-Trp-Met-SEQ. 26*
*-Arg-Pro-Lys-Pro-Gln-Gln-Phe-Trp-SEQ. 59*

or
- when they are the target of the MMP-14 enzyme, the first sequence $S_1$ and/or the second sequence $S_2$ correspond to at least one of the following sequences:

-Pro-Leu-Ala-Cys(*p*-OMeBz)-Trp-Ala-Arg-SEQ. 60
*-Pro-Cha-Gly-Cys(Me)-His-Ala-SEQ. 20*
*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*

or
- when they are the target of the MMP-15 enzyme, the first sequence $S_1$ and/or the second sequence $S_2$ correspond to at least one of the following sequences:

*-Pro-Leu-Gly-Leu-Dpa-Ala-Arg-SEQ. 21*

or
- when they are the target of the MMP-16 enzyme, the first sequence $S_1$ and/or the second sequence $S_2$ correspond to at least one of the following sequences:

*-Pro-Leu-Gly-Leu-Dpa-Ala-Arg-SEQ. 21*

or
- when they are the target of the MMP-17 enzyme, the first sequence $S_1$ and/or the second sequence $S_2$ correspond to at least one of the following sequences:

*-Pro-Leu-Gly-Leu-Dpa-Ala-Arg-SEQ. 21*
*-Pro-Leu-Gly-[2-mercapto-4-methyl-pentanoyl]-Leu-Gly-SEQ. 22*

or
- when they are the target of the MMP-19 enzyme, the first sequence $S_1$ and/or the second sequence $S_2$ correspond to at least one of the following sequences:

*-Pro-Leu-Gly-Leu-Dpa-Ala-Arg-SEQ. 21*

or
- when they are the target of the MMP-23 enzyme, the first sequence $S_1$ and/or the second sequence $S_2$ correspond to at least one of the following sequences:

*-Pro-Leu-Gly-Leu-Dpa-Ala-Arg-SEQ. 21*

or

- when they are the target of the MMP-25 enzyme, the first sequence $S_1$ and/or the second sequence $S_2$ correspond to at least one of the following sequences:

*-Pro-Leu-Gly-Leu-Dpa-Ala-Arg-SEQ. 21*

or

- when they are the target of the MMP-26 enzyme, the first sequence $S_1$ and/or the second sequence $S_2$ correspond to at least one of the following sequences:

*-Pro-Leu-Gly-Leu-Trp-Ala-D-Arg-SEQ. 16*

**3.** The cyclic peptide according to any of the preceding claims, wherein the donor group carried by the $X_1$ probe and the acceptor group carried by the $X_2$ probe comprise an aromatic nucleus.

**4.** The cyclic peptide according to claim 3, wherein the aromatic nucleus is a benzene, anthracene or coumarin nucleus.

**5.** The cyclic peptide according to any of the preceding claims, wherein the donor group caried by the $X_1$ probe and the acceptor group carried by the $X_2$ probe are chosen from among the following pairs:

*Tryptophan/2,4-dinitrophenyl;
*o-aminobenzoic acid/2,4-dinitrophenyl;
*(7-methoxycoumarin-4-yl)-acetyl/2,4-dinitrophenyl;
*(7-methoxycoumarin-4-yl)-acetyl/N-3-(2,4-dinitrophenyl)-L-2,3-diaminopropyl;
*Tryptophan/Dansyl;
*N-methylanthranoyl/2,4-dinitrophenyl;
*6,7-dimethoxycoumarin-4-yl-acetyl/6-N-(7-nitrobenz-2-oxa-1,3-diazol-4-yl)aminohexanoic acid;
*5-(2'-aminomethyl)naphtalene sulphonic acid/4-(4'-dimethylaminophenylaza)benzoic acid;
*7-methoxycoumarin-3-carboxylic acid/7-diethylaminocoumarin-3-carboxylic acid.

**6.** The cyclic peptide according to any of the preceding claims, wherein the donor group carried by the $X_1$ probe and the acceptor group carried by the $X_2$ probe comprise a coumarin nucleus.

**7.** The cyclic peptide according to any of the preceding claims, wherein the donor group carried by the $X_1$ probe and the acceptor group carried by the $X_2$ probe respectively correspond to the following formulas:

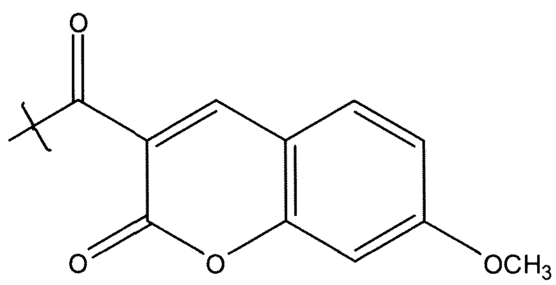

MC

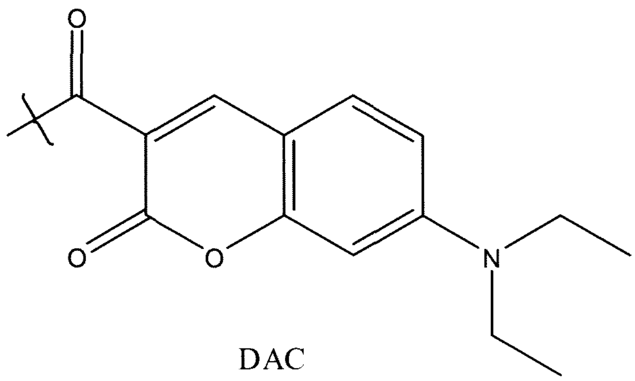

DAC

8. The cyclic peptide according to any of the preceding claims, wherein the probes $X_1$ and $X_2$ are amino acid residues.

9. The cyclic peptide according to claim 8, wherein the probes $X_1$ and $X_2$ respectively meet the following formulas:

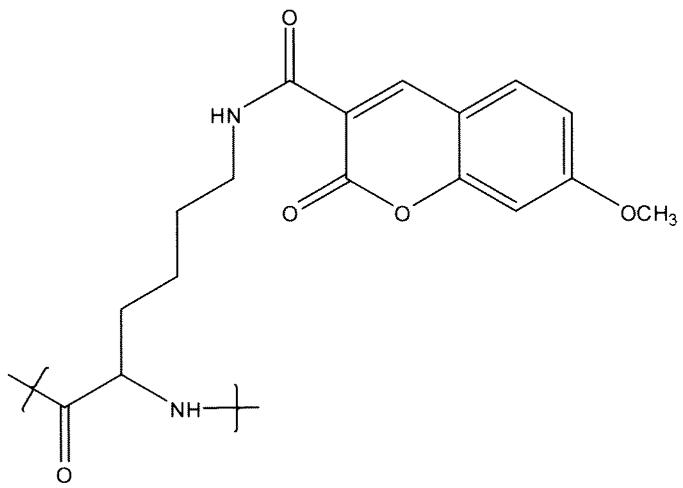

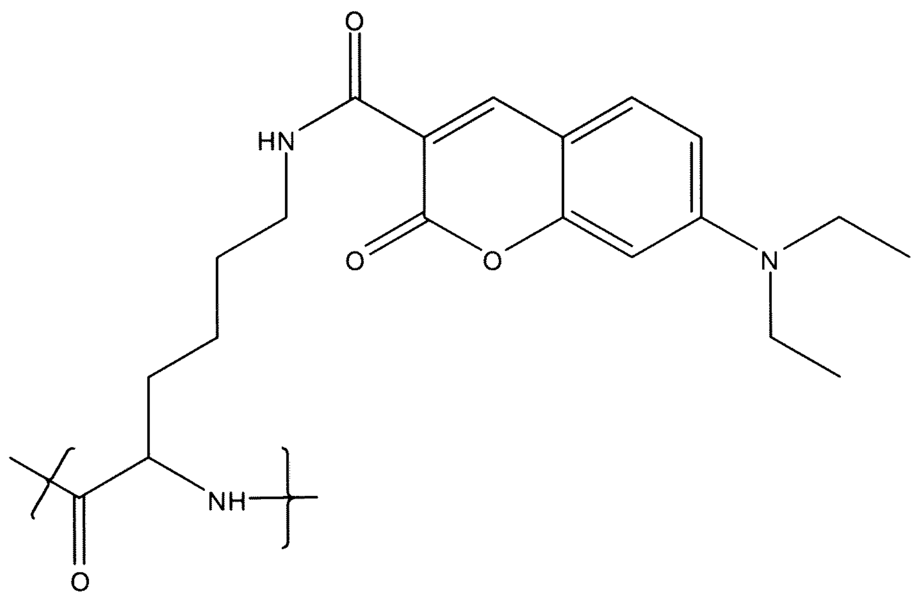

10. The cyclic peptide according to claim 1, wherein the peptide sequences $S_1$ and $S_2$ meet the following formula:

-Gly-Pro-Gln-Gly-Leu-Leu-Gly-Ala-

**11.** The cyclic peptide according to claim 10, meeting the following formula:

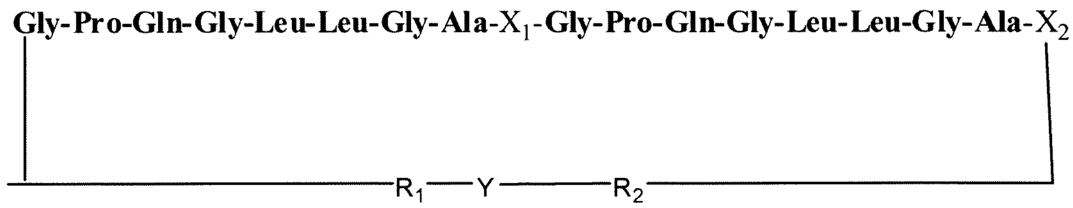

$R_1$, Y and $R_2$ meeting the same definition as the definition given in claim 1.

**12.** The cyclic peptide according to claim 10 or 11, meeting the following formula:

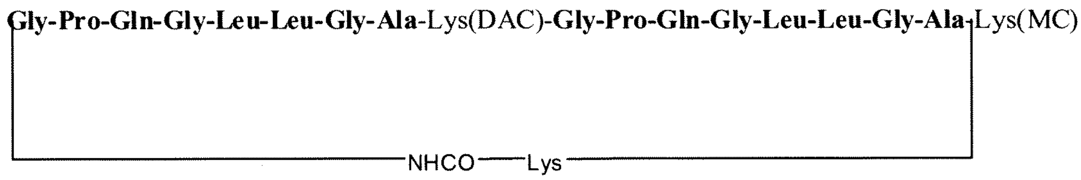

**13.** Method for preparing a cyclic peptide such as defined according to claim 1, comprising the following steps:

a) a preparation step, to prepare a linear peptide by chemical synthesis on a solid phase;
b) a release step, to release the linear peptide from the solid phase; and
c) a coupling step, to couple the ends of the linear peptide to form a cyclic peptide.

**14.** The method for preparing a cyclic peptide such as defined according to claim 1, comprising the following steps:

a) a preparation step to prepare the linear peptide by chemical synthesis on a solid phase;
b) a coupling step, to couple the free end of the linear peptide with a terminal function of a linear residue of the linear peptide; and
c) a release step, to release the cyclic peptide from the solid phase, steps b) and c) possibly being conducted concomitantly.

**15.** Ready-to-use reagent comprising at least one cyclic peptide such as defined according to any of claims 1 to 12.

**16.** Kit at least comprising:

- a first compartment comprising at least one cyclic peptide such as defined according to any of claims 1 to 12; and
- a second compartment comprising a buffer solution.

**17.** Use of at least one cyclic peptide such as defined according to any of claims 1 to 12 for *in vitro* determination of the activity of at least one protease enzyme.

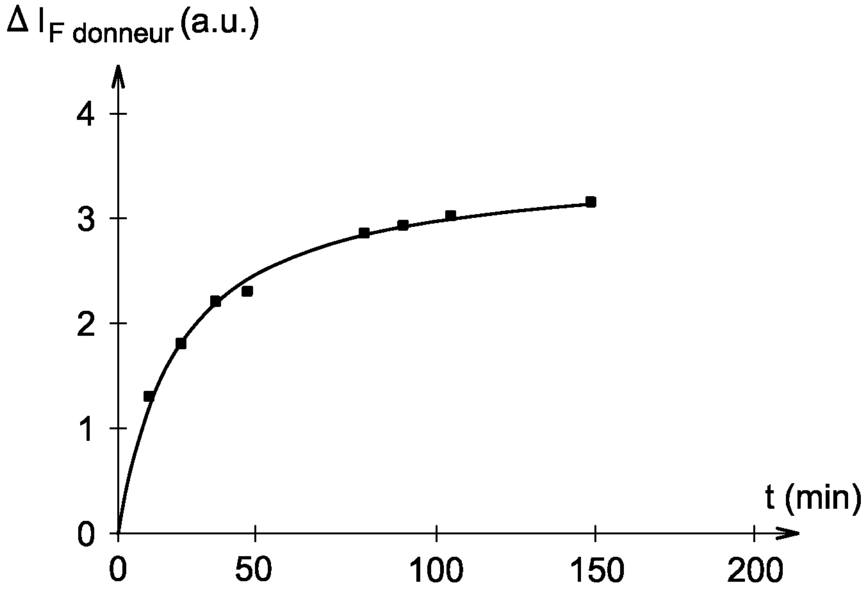
Δ I F donneur (a.u.)
4
3
2
1
0
0
50
100
150
200
t (min)

FIG. 1

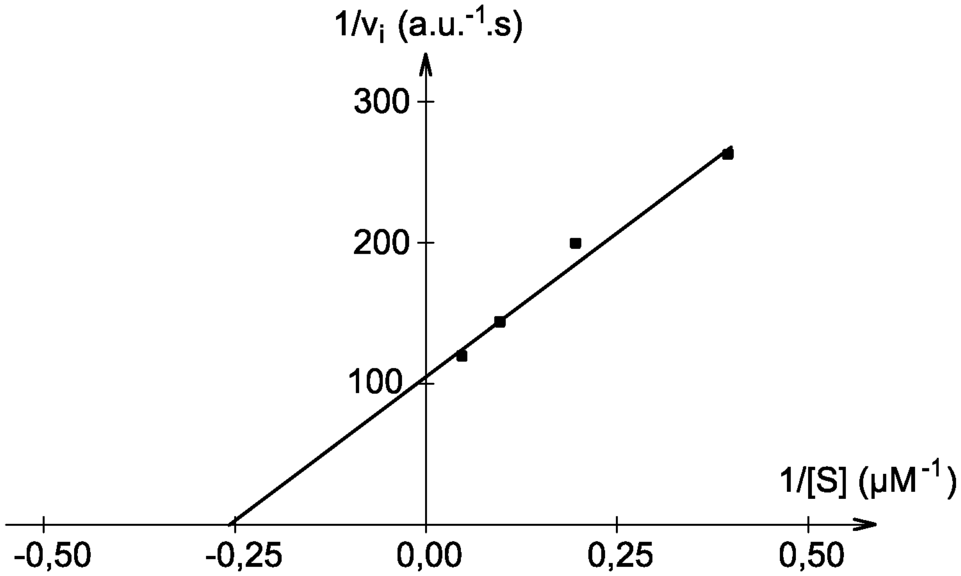
1/vi (a.u.-1.s)
300
200
100
1/[S] (µM-1)
-0,50
-0,25
0,00
0,25
0,50

FIG. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0104623 A
- FR 0853850


### Littérature non-brevet citée dans la description


- **LOMBARD et al.** *Biochimie,* 2005, vol. 87, 265-272
- **GRUENEWALD et al.** *Chemistry Biology,* Août 2005, vol. 12 (8), 873-881
- **DENG, S.J., et al.** *J. Biol. Chem.,* 2000, vol. 275, 31422
- **BEEKMAN, B., et al.** *FEBS Lett,* 1996, vol. 390, 221
- **BICKETT, D.M., et al.** *Anal. Biochem.,* 1993, vol. 212, 58
- **KNIGHT, C.G. et al.** *FEBS Lett.,* 1992, vol. 296, 263
- **DARLAK, K. et al.** *J. Biol. Chem.,* 1990, vol. 265, 5199
- **STACK, M.S., ; GRAY, R.D.** *J. Biol. Chem.,* 1989, vol. 264, 4277
- **NETZEL-ARNETT, S. et al.** *Anal. Biochem.,* 1991, vol. 195, 86
- **MCGEEHAN, G.M. et al.** *J. Biol. Chem.,* 1994, vol. 269, 32814
- **BICKETT, D.M. et al.** *Anal. Biochem.,* 1993, vol. 212, 58
- **MOHAN, M.J. et al.** *Biochemistry,* 2002, vol. 41, 9462
- **HANESSIAN, S. et al.** *J. Med. Chem.,* 2001, vol. 44, 3066
- **AMBROSE, W.P. et al.** *Anal. Biochem.,* 1998, vol. 263, 150
- **KNÄUPER, V. et al.** *J. Biol. Chem.,* 1996, vol. 271, 1544
- **WEINGARTEN, H. ; FEDER, J.** *Anal. Biochem.,* 1985, vol. 147, 437
- **WEINGARTEN, H. et al.** *Biochemistry,* 1985, vol. 24, 6730
- **A. SANTALA, A. et al.** *FEBS Lett.,* 1999, vol. 461, 153-156
- **NAGASE, H. et al.** *J.Biol.Chem.,* 1994, vol. 269, 20952-20957
- **J. BERMAN et al.** *J.Biol.Chem.,* 1992, vol. 267, 1434-1437
- **KRAFT, P.J. et al.** *Connect.Tissue Res.,* 2001, vol. 42, 149-163
- **ITOH, M. et al.** *J.Pharm.Biomed.Anal.,* 1997, vol. 15, 1417-1426
- **WELCH, A.R. et al.** *Arch.Biochem.Biophys,* 1995, vol. 324, 59-64
- **LAUER-FIELDS, J.L. ; FIELDS, G.B.** *Biol.Chem.,* 2002, vol. 383, 1095-1105
- **LAUER-FIELDS, J.L. et al.** *Biochemistry,* 2001, vol. 40, 5795-5803
- **BREMER, C. et al.** *Acad.Radiol.,* 2002, vol. 9 (2), S314-S315
- **BEEKMAN, B. et al.** *FEBS Lett,* 1996, vol. 390, 221
- **DARLAK, K., et al.** *J. Biol. Chem.,* 1990, vol. 265, 5199
- **MURPHY, G. et al.** *J. Biol. Chem.,* 1994, vol. 269, 6632
- **WEINGARTEN, H., et al.** *Anal. Biochem.,* 1985, vol. 147, 437
- **G.D. JOHNSON ; K. AHN.** *Anal. Biochem.,* 2000, vol. 286, 112
- **XIA, T. et al.** *Biochim. Biophys. Acta,* 1996, vol. 1293, 259
- **DENG, S.J. et al.** *J. Biol. Chem.,* 2000, vol. 275, 31422
- **BEEKMAN, B. et al.** *FEBS Lett,* 1997, vol. 418, 305
- **STACK, M.S. ; GRAY, R.D.** *J. Biol. Chem.,* 1989, vol. 264, 4277
- **BICKETT, D.M. et al.** *Ann. N.Y. Acad. Sci.,* 1994, vol. 732, 351
- **WELCH, A.R. et al.** *Biochemistry,* 1996, vol. 35, 10103
- **WELCH, A.R., et al.** *Arch. Biochem. Biophys.,* 1995, vol. 324, 59
- **SHABANI, F. et al.** *Free Radic.Res.,* 1998, vol. 28, 115-123
- **FINCH-ARIETTA, M. et al.** *Agents Actions,* 1993, vol. 39, C189-C191

- **NETZEL-ARNETT, S. et al.** *Anal.Biochem.,* 1991, vol. 195, 86-92
- **BICKETT, D.M. et al.** *Ann.N.Y.Acad.Sci.,* 1994, vol. 732, 351-355
- **GRAMS, F. et al.** *Biol. Chem.,* 2001, vol. 382, 1277
- **WELCH, A.R. et al.** *Arch.Biochem.Biophys.,* 1995, vol. 324, 59-64
- **WEINGARTEN, H. et al.** *Anal. Biochem.,* 1985, vol. 147, 437
- **ROY, N. et al.** *Prot. Expr. Purif.,* 1999, vol. 16, 324
- **KANNAN, R. et al.** *Prot. Expr. Purif.,* 1999, vol. 16, 76
- **PARK, H.I. et al.** *J. Biol. Chem.,* 2000, vol. 275, 20540
- **BERMAN, J. et al.** *J.Biol.Chem.,* 1992, vol. 267, 1434-1437
- **BEEKMAN, B., et al.** *FEBS Lett,* 1997, vol. 418, 305
- **KNAUPER, V., et al.** *J. Biol. Chem.,* 1996, vol. 271, 1544
- **SANTALA, A. et al.** *FEBS Lett.,* 1999, vol. 461, 153-156
- **BICKETT, D.M. et al.** *Anal.Biochem.,* 1993, vol. 212, 58-64
- **D'ORTHO, M.P. et al.** *Eur.J.Biochem.,* 1997, vol. 250, 751-757
- **FINCH-ARIETTA, M. et al.** *Agents Actions,* 1993, vol. 39, C189-C191
- **ASCHI, M. et al.** *J.Comput.Aided Mol.Des,* 2002, vol. 16, 213-225
- **MUCHA, A. et al.** *J. Biol. Chem.,* 1998, vol. 273, 2763
- **HOLTZ, B. et al.** *Biochemistry,* 1999, vol. 38, 12174
- **RODERFELD, M. et al.** *Prot. Expr. Purif.,* 2000, vol. 19, 369
- **XUE, C.-B. et al.** *J. Med. Chem.,* 2001, vol. 44, 2636
- **SHIMADA, T. et al.** *Eur. J. Biochem.,* 1999, vol. 262, 907
- **ENGLISH, W.R. et al.** *FEBS Lett.,* 2001, vol. 491, 137
- **VELASCO, G. et al.** *J. Biol. Chem.,* 1999, vol. 274, 4570
- **PARK, H.I. et al.** *J. Biol. Chem.,* 2002, vol. 277, 35168